# EUROPEAN PATENT APPLICATION

(11) **EP 1 281 775 A2**
(43) Date of publication of application: **05.02.2003**
(21) Application number: 02255231.9
(22) Date of filing: 26.07.2002
(51) Int. Cl.: C12Q 1/68

(54) **Identification and use of molecules implicated in pain**

(30) Priority: 27.07.2001 GB 0118354; 07.02.2002 GB 0202892
(71) Applicant: WARNER-LAMBERT COMPANY, Morris Plains, New Jersey 07950 (US)
(72) Inventor: Brooksbank, Robert Alan, Cambridge, Cambridgeshire CB1 3AW (GB); Dixon, Alistair Kerr, Cambridge, Cambridgeshire CB1 2LL (GB); Lee, Kevin, c/o Pfizer Limited, Sandwich, Kent CT13 9NJ (GB); Pinnock, Robert Denham, Pfizer Global Res. & Dev., Ann Arbor, MI 48105 (US)
(74) Representative: Hayles, James Richard

(57) **Abstract**

The invention relates to the use of:
Use of:
(a) an isolated gene sequence that is up-regulated in the spinal cord of a mammal in response to mechanistically distinct first and second models of neuropathic or central sensitization pain;
(b) an isolated gene sequence comprising a nucleic acid sequence of Table 1;
(c) an isolated gene sequence having at least 80% sequence identity with a nucleic acid sequence of Table 1;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell comprising the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a polypeptide variant thereof with sequential amino acid deletions from the C terminus and/or the N-terminus;
(i) an isolated polypeptide encoded by a nucleotide sequence according to any one of (a) to (d); or
(j) an isolated antibody that binds specifically to a polypeptide according to (h) or (i);
in the screening of compounds for the treatment of pain, or for the diagnosis of pain.

The invention also relates to the use of naturally occurring compounds such as peptide ligands of the expression products of the above gene sequences and their associated signal transduction pathways for use in the treatment of pain.

SEQUENCE LISTING

<110> Warner-Lambert Company
      Lee, Kevin
      Dixon, Alistair
      Brooksbank, Robert
      Pinnock, Robert

<120> Identification and Use of Molecules Implicated in Pain

<130> WL-A-018198

<150> GB 0118354.0
<151> 2001-07-27

<160> 34

<170> PatentIn version 3.0

<210> 1
<211> 604
<212> PRT
<213> Rattus norvegicus

<220>
<223> A-raf protein (AA 1-604)

<400> 1

Met Glu Pro Pro Arg Gly Pro Pro Ala Ser Gly Ala Glu Pro Ser Arg
1               5                   10                  15

Ala Val Gly Thr Val Lys Val Tyr Leu Pro Asn Lys Gln Arg Thr Val
            20                  25                  30

Val Thr Val Arg Asp Gly Met Ser Val Tyr Asp Ser Leu Asp Lys Ala
        35                  40                  45

Leu Lys Val Arg Gly Leu Asn Gln Asp Cys Cys Val Val Tyr Arg Leu
    50                  55                  60

Ile Lys Gly Arg Lys Thr Val Thr Ala Trp Asp Thr Ala Ile Ala Pro
65                  70                  75                  80

Leu Asp Gly Glu Glu Leu Ile Val Glu Val Leu Glu Asp Val Pro Leu
                85                  90                  95

Thr Met His Asn Phe Val Arg Lys Thr Phe Phe Ser Leu Ala Phe Cys
            100                 105                 110

Asp Phe Cys Leu Lys Phe Leu Phe His Gly Phe Arg Cys Gln Thr Cys
        115                 120                 125

Gly Tyr Lys Phe His Gln His Cys Ser Ser Lys Val Pro Thr Val Cys
    130                 135                 140

Val Asp Met Ser Thr Asn Arg Arg Gln Phe Tyr His Ser Ile Gln Asp
145                 150                 155                 160

Leu Ser Gly Gly Ser Arg Gln Gln Glu Val Pro Ser Asn Leu Ser Val
                165                 170                 175

Asn Glu Leu Leu Thr Pro Gln Gly Pro Ser Pro Phe Thr Gln Gln Arg
            180                 185                 190

Asp Gln Glu His Phe Ser Phe Pro Ala Pro Ala Asn Pro Pro Leu Gln
        195                 200                 205

Arg Ile Arg Ser Thr Ser Thr Pro Asn Val His Met Val Ser Thr Thr
    210                 215                 220

Ala Pro Met Asp Ser Ser Leu Met Gln Phe Thr Ala Gln Ser Phe Ser
225                 230                 235                 240

Thr Asp Ala Ala Gly Arg Gly Gly Asp Gly Ala Pro Arg Gly Ser Pro
                245                 250                 255

Ser Pro Ala Ser Val Ser Ser Gly Arg Lys Ser Pro His Ser Lys Leu
            260                 265                 270

Pro Ala Glu Gln Arg Glu Arg Lys Ser Leu Ala Asp Glu Lys Lys Lys
        275                 280                 285

Val Lys Asn Leu Gly Tyr Arg Asp Ser Gly Tyr Tyr Trp Glu Val Pro
    290                 295                 300

Pro Ser Glu Val Gln Leu Leu Lys Arg Ile Gly Thr Gly Ser Phe Gly
305                 310                 315                 320

Thr Val Phe Arg Gly Arg Trp His Gly Asp Val Ala Val Lys Val Leu
                325                 330                 335

Lys Val Ala Gln Pro Thr Ala Glu Gln Ala Gln Ala Phe Lys Asn Glu
            340                 345                 350

Met Gln Val Leu Arg Lys Thr Arg His Val Asn Ile Leu Leu Phe Met
        355                 360                 365

Gly Phe Met Thr Arg Pro Gly Phe Ala Ile Ile Thr Gln Trp Cys Glu
    370                 375                 380

Gly Ser Ser Leu Tyr His His Leu His Val Ala Asp Thr Arg Phe Asp
385                 390                 395                 400

Met Val Gln Leu Ile Asp Val Ala Arg Gln Thr Ala Gln Gly Met Asp
                405                 410                 415

Tyr Leu His Ala Lys Asn Ile Ile His Arg Asp Leu Lys Ser Asn Asn
            420                 425                 430

Ile Phe Leu His Glu Gly Leu Thr Val Lys Ile Gly Asp Phe Gly Leu
        435                 440                 445

Ala Thr Val Lys Thr Arg Trp Ser Gly Ala Gln Pro Leu Glu Gln Pro
    450                 455                 460

Ser Gly Ser Val Leu Trp Met Ala Ala Glu Val Ile Arg Met Gln Asp
465                 470                 475                 480

Pro Asn Pro Tyr Ser Phe Gln Ser Asp Val Tyr Ala Tyr Gly Val Val
                485                 490                 495

Leu Tyr Glu Leu Met Thr Gly Ser Leu Pro Tyr Ser His Ile Gly Ser
            500                 505                 510

Arg Asp Gln Ile Ile Phe Met Val Gly Arg Gly Tyr Leu Ser Pro Asp
        515                 520                 525

Leu Ser Lys Ile Phe Ser Asn Cys Pro Lys Ala Met Arg Arg Leu Leu
    530                 535                 540

Thr Asp Cys Leu Lys Phe Gln Arg Glu Glu Arg Pro Leu Phe Pro Gln
545                 550                 555                 560

Ile Leu Ala Thr Ile Glu Leu Leu Gln Arg Ser Leu Pro Lys Ile Glu
                565                 570                 575

Arg Ser Ala Ser Glu Pro Ser Leu His Arg Thr Gln Ala Asp Glu Leu
            580                 585                 590

Pro Ala Cys Leu Leu Ser Ala Ala Arg Leu Val Pro
        595                 600

<210>  2

<211>  2288

<212>  DNA

<213>  Rattus norvegicus

<220>

<223>  cDNA coding region for A-raf protein

<400> 2
   gcggtagcgt gtgacaggag gcctatggca cctgcccagt cctacctcag cccatcttga 60
   aaaaatcctt agacaacatg gaaccaccac gaggccctcc tgctagtggg gctgagccat 120
   ctcgggcagt tggcactgtc aaagtgtacc tgcctaacaa gcaacgcaca gtggtgactg 180
   tccgggatgg catgagtgtc tatgactctt tggacaaggc cctcaaggtg cggggtctca 240
   atcaggactg ctgtgtggtc tacagactca tcaaaggaag aaagacagtc actgcctggg 300
   acacagccat tgcacctctg gatggcgagg agctcattgt ggaggtcctg gaagatgtgc 360
   cactgaccat gcacaatttt gtacggaaga cattcttcag tttggccttc tgtgacttct 420
   gccttaagtt tctgttccac ggctttcgct gccaaacctg tggctacaag ttccaccagc 480
   attgttcctc caaggtcccc acggtctgcg ttgacatgag taccaaccgc cgacagttct 540
   accacagcat ccaggatttg tctggaggct ccaggcagca ggaggttccc tcaaatctct 600
   ctgtgaatga gctgctaacc ccccagggtc ccagtccctt tacccagcaa cgtgaccagg 660
   agcacttctc cttccctgcc cctgccaatc ccccactgca gcgcatccgc tccacatcta 720
   ctcctaacgt ccacatggtc agcaccacag ctcccatgga ctccagcctc atgcagttta 780
   ctgctcagag cttcagcacc gatgctgctg gtagaggtgg tgatggagct cctcggggta 840
   gccctagccc agctagtgtg tcttcaggga ggaagtcccc acattccaag ttacctgcag 900
   aacagcggga acggaagtcc ttggcagatg aaaagaaaaa agtgaagaac ctggggtacc 960
   gggactcagg ctattactgg gaggtgccac ccagtgaggt acagctgttg aagaggatcg 1020
   ggacaggctc ttttggcact gtgtttcggg ggcgttggca tggcgatgta gctgtgaaag 1080
   tgctcaaggt ggcccaacct accgctgagc aggcccaggc cttcaagaat gagatgcagg 1140
   tgctcaggaa gacacgacat gtcaacattt tgctgtttat gggtttcatg actcggccag 1200
   ggtttgccat catcacacag tggtgtgagg gttccagcct ctaccaccac ctacatgtgg 1260
   ctgacacgcg ctttgacatg gtccagctca ttgatgtggc ccggcagact gcccagggca 1320
   tggactacct ccacgccaag aacatcattc accgagacct aaagtccaac aatatcttcc 1380
   tacatgaggg gctcacagtc aagattggtg acttcggcct ggccacagtg aagacacgat 1440
   ggagtggggc ccagccctta gagcagccct cagggtctgt gctgtggatg gcagctgagg 1500
   tgatccgaat gcaggacccg aacccctaca gcttccagtc ggatgtctat gcctatggtg 1560
   ttgtgctcta tgagcttatg accggctcac tgccctacag ccacattggc agccgtgacc 1620
   agatcatctt tatggtgggt cgtggctatc tgtctccgga cctcagcaaa atcttcagta 1680
   attgccccaa agccatgagg cgcttgctga ctgactgcct caagttccag cgggaggagc 1740
   ggcctctatt tccccagatt ctggccacga tcgagctgct gcagcggtca ctccccaaga 1800
   ttgagcggag tgcctccgaa ccctccttgc accgtaccca ggctgatgag ttgcctgcct 1860
   gccttctcag cgcagcccgc cttgtgcctt agactccact cccagcccac tagggagcca 1920
   ttttcagctt accatgccaa ggcaccccct tcctaccagc caatcattgt tctgtctgtg 1980
   ccctgatact gcctcaggat tccctatccc acaccctggg aaatttgggg gactccaaaa 2040
   actgaggtcc cctgcttcct ccataatttg gtctcctctt ggctttgggg atagttctaa 2100
   tttggagagc tgttttacct ccaatggctg ggattcagtg caaagattcc actcggaacc 2160
   tctttataaa gttttcgcct gacatgtctt cactgaatta tggggttccc agcaccccat 2220
   gcggatttgg gagtttccct ttgtctcccc ccactattca aggactctcc tctttaccaa 2280
   gaagcaca 2288

<210> 3

<211> 327

<212> PRT

<213> Rattus norvegicus

<220>
<223> Protein phosphatase 1, catalytic subunit

<400> 3

Met Ala Asp Gly Glu Leu Asn Val Asp Ser Leu Ile Thr Arg Leu Leu
1               5                   10                  15

Glu Val Arg Gly Cys Arg Pro Gly Lys Ile Val Gln Met Thr Glu Ala
            20                  25                  30

Glu Val Arg Gly Leu Cys Ile Lys Ser Arg Glu Ile Phe Leu Ser Gln
        35                  40                  45

Pro Ile Leu Leu Glu Leu Glu Ala Pro Leu Lys Ile Cys Gly Asp Ile
    50                  55                  60

His Gly Gln Tyr Thr Asp Leu Leu Arg Leu Phe Glu Tyr Gly Gly Phe
65                  70                  75                  80

Pro Pro Glu Ala Asn Tyr Leu Phe Leu Gly Asp Tyr Val Asp Arg Gly
                85                  90                  95

Lys Gln Ser Leu Glu Thr Ile Cys Leu Leu Leu Ala Tyr Lys Ile Lys
            100                 105                 110

Tyr Pro Glu Asn Phe Phe Leu Leu Arg Gly Asn His Glu Cys Ala Ser
        115                 120                 125

Ile Asn Arg Ile Tyr Gly Phe Tyr Asp Glu Cys Lys Arg Arg Phe Asn
    130                 135                 140

Ile Lys Leu Trp Lys Thr Phe Thr Asp Cys Phe Asn Cys Leu Pro Ile
145                 150                 155                 160

Ala Ala Ile Val Asp Glu Lys Ile Phe Cys Cys His Gly Gly Leu Ser
                165                 170                 175

Pro Asp Leu Gln Ser Met Glu Gln Ile Arg Arg Ile Met Arg Pro Thr
            180                 185                 190

Asp Val Pro Asp Thr Gly Leu Leu Cys Asp Leu Leu Trp Ser Asp Pro
        195                 200                 205

Asp Lys Asp Val Gln Gly Trp Gly Glu Asn Asp Arg Gly Val Ser Phe
    210                 215                 220

Thr Phe Gly Ala Asp Val Val Ser Lys Phe Leu Asn Arg His Asp Leu
225                 230                 235                 240

Asp Leu Ile Cys Arg Ala His Gln Val Val Glu Asp Gly Tyr Glu Phe
                245                 250                 255

Phe Ala Lys Arg Gln Leu Val Thr Leu Phe Ser Ala Pro Asn Tyr Cys
            260                 265                 270

Gly Glu Phe Asp Asn Ala Gly Gly Met Met Ser Val Asp Glu Thr Leu
        275                 280                 285

Met Cys Ser Phe Gln Ile Leu Lys Pro Ser Glu Lys Lys Ala Lys Tyr
    290                 295                 300

Gln Tyr Gly Gly Leu Asn Ser Gly Arg Pro Val Thr Pro Pro Arg Thr
305                 310                 315                 320

Ala Asn Pro Pro Lys Lys Arg
                325

<210>  4

<211>  2706

<212>  DNA

<213>  Rattus norvegicus

<220>
<223>  cDNA phosphatase 1, catalytic subunit

<400>  4
cgcccttgtt cccgctgcgg ggaggagagt ctggtgccta caagatggcg gacggggagc      60

tgaacgtgga cagcctcatc acccgcctgc tggaggtacg aggatgtcgt ccgggaaaaa     120

ttgtgcagat gactgaagca gaagtccgag gactgtgtat caagtctcgt gaaatctttc     180

ttagccagcc tattcttttg gaattggaag cgccactgaa gatttgtgga gatattcatg     240

gacagtatac agacttactg agattatttg aatatggagg ttttccacca gaagccaact     300

atcttttctt aggagattat gtggacagag gaaagcagtc tttggaaacc atctgtttgc     360

tattggctta caaaatcaaa tacccagaga acttctttct tctacgagga aaccatgagt     420

gtgctagcat caaccgcatt tatggattct atgatgagtg caaacgaaga tttaatatta     480

aattgtggaa gacattcact gattgtttta attgtctgcc tatagctgct attgttgatg     540

agaaaatctt ctgctgtcat ggaggactgt caccagacct acagtctatg gaacagattc     600

ggagaattat gagacccact gacgtacctg atacaggttt gctttgtgat ttactgtggt     660

ccgacccaga taaggatgta caaggctggg gagaaaatga tcgtggtgtt tcttttactt     720

ttggagctga tgtagtcagt aaatttctga atcgtcatga tttggacttg atttgtcgag 780
cccatcaggt ggtagaagat ggatatgaat tttttgctaa acgacaattg gtaactttat 840
tttctgcccc aaattactgc ggcgagtttg acaatgctgg tggtatgatg agtgtggatg 900
aaactttgat gtgttcattc cagatattga aaccatctga aaagaaagct aagtaccagt 960
atggtgggct gaattctgga cgtcctgtca ctccgcctcg aacagctaat ccaccgaaga 1020
aaaggtgaag acaggaattc cggaaagaga aaccatcaga tttgttaagg acatacttca 1080
taatatataa gtgtgcactg taaaaccatc cagccattcg acacccttta tgatgtcaca 1140
cctttaactt aaggagacgg gtaaaggatc ttaaattttt ttctaataga aagatgtgct 1200
acactgtatt gtaataagta tactctgtta taatattcaa caaagttaaa tccaaattca 1260
aaagtatcca ttaaagttct atcttctcat atcacagttt ttaaagttga aagcatccca 1320
gttaaactag ctgcgttagt tacccagatg agagcatgaa gatccatctg tgtaatgtgg 1380
ctttagtgtt gcttggttgt ttctttattt tgggcttgtt ttgttttgtt tgtttttgct 1440
agaataatgg catctacttt tcctattttt ccctaaccat tttaaaaagt gaaaatggga 1500
agagctttaa agacattcac caactattct tttccttcac ttatctactt aaggaactgt 1560
tggatcttac taagaaaact tacgcctcat aataaaaagg aactttagag gccgataggt 1620
tttaaaaata tacaaactat ttgatccaat gattttaatc aaacagtttg actgggcaaa 1680
ctttgcagct gataatgact atttcgcttt ttacaaattg ccactgattt ggatttgtgc 1740
actctaacct ttaatttatt gatgctctat tgtgcagtag catttcattt aagataaggc 1800
tcatatagta ctatccaaaa ttagttggta atgtgattat gtggtacctt ggctttaggt 1860
tttaattcgc acgaaacacc ttttggcatg cttaactttc tggtattatc ctcacctgca 1920
ttggttttgt tttttggggt ttttgttgtt gtttgtttgt ttgtttttag atccacagaa 1980
catgagaatc ctttttgaca agccttggaa agctggctct tctttccctc tctatgtgaa 2040
ggatgtattt aaatgaacac tggtcagtgg gacattgtca gctctgagta ttgggtgctt 2100
cactgtctaa taattgccat gtgaatgttg tttttgactg taaggctatg tcactaaaga 2160
tttttactct gcgttttcat aatcaaaggt catgatgtgt atagacatgc tttgtagtga 2220
agtatagtag caataatttc tgcacatgat caagagttta ttgcagcatt tctttccctg 2280
ttctctcttt tttaagggtt agcattaaca aatgtcaagg aatagcaaag tcaacaaaga 2340
ctttaggagg tggaattaag aacacacaga tttgtgatct ttggatgtga cacttattgg 2400
atgttattct aaagtcttat tgaacattgt caaatttgta agcttcatgg ggatggacat 2460
aatgtttata taatgccctt cttatgtgtt accatagatg tgaaacctta tattgtcttt 2520
gaaaatgtta aattgagaac tctgttaaca ttttatggat tggcacatta tattactgca 2580
agaaacattt gattttcagc acagtgcaaa agttctttaa aatgcatatg tctttttttc 2640
taattcaatt ttgtttaaag cacattttaa atgtagtttt ctcatttagt aaaaagttgt 2700

ctaatt                                                                2706

<210>  5

<211>  776

<212>  PRT

<213>  Rattus norvegicus

<220>
<223>  Protein: phosphofructokinase muscle isozyme

<400>  5

Met Thr His Glu Glu His His Glu Ala Lys Thr Leu Gly Ile Gly Lys
1               5                   10                  15

Ala Ile Ala Val Leu Thr Ser Gly Gly Asp Ala Gln Gly Met Asn Ala
            20                  25                  30

Thr Val Arg Ala Val Val Arg Val Gly Ile Phe Thr Gly Leu Arg Val
        35                  40                  45

Phe Phe Val His Glu Gly Tyr Gln Gly Leu Val Asp Gly Gly Glu His
    50                  55                  60

Ile Arg Glu Ala Thr Trp Glu Ser Val Ser Met Met Leu Gln Leu Gly
65                  70                  75                  80

Gly Thr Val Ile Gly Ser Ala Arg Cys Lys Asp Phe Arg Glu Arg Glu
                85                  90                  95

Gly Arg Leu Arg Ala Ala His Asn Leu Val Lys Arg Gly Ile Thr Asn
            100                 105                 110

Leu Cys Val Ile Gly Gly Asp Gly Ser Leu Thr Gly Ala Asp Thr Phe
        115                 120                 125

Arg Ser Glu Trp Ser Asp Leu Leu Asn Asp Leu Gln Lys Asp Gly Lys
    130                 135                 140

Ile Thr Ala Glu Glu Arg Thr Lys Ser Ser Tyr Leu Asn Ile Val Phe
145                 150                 155                 160

Leu Val Gly Ser Ile Asp Asn Asp Phe Cys Gly Thr Asp Met Thr Ile
                165                 170                 175

Gly Thr Asp Ser Ala Leu His Arg Ile Val Glu Ile Val Asp Ala Ile
            180                 185                 190

Thr Thr Thr Ala Gln Ser His Gln Arg Thr Phe Val Leu Glu Val Met
        195                 200                 205

Gly Arg His Cys Gly Tyr Leu Ala Leu Val Thr Ser Leu Ser Cys Gly
    210                 215                 220

Ala Asp Trp Val Phe Ile Pro Glu Cys Pro Pro Asp Asp Asp Trp Glu
225                 230                 235                 240

Glu His Leu Cys Arg Arg Leu Ser Glu Thr Arg Thr Arg Gly Ser Arg
                245                 250                 255

Leu Asn Ile Ile Ile Val Ala Glu Gly Ala Ile Asp Lys Asn Gly Lys
            260                 265                 270

Pro Ile Thr Ser Glu Asp Ile Lys Asn Leu Val Val Lys Arg Leu Gly
        275                 280                 285

Tyr Asp Thr Arg Val Thr Val Leu Gly His Val Gln Arg Gly Gly Thr
    290                 295                 300

Pro Ser Ala Phe Asp Arg Ile Leu Gly Ser Arg Met Gly Val Glu Ala
305                 310                 315                 320

Val Met Ala Leu Leu Glu Gly Thr Pro Asp Thr Pro Ala Cys Val Val
                325                 330                 335

Ser Leu Ser Gly Asn Thr Ala Val Arg Leu Pro Leu Met Glu Cys Val
            340                 345                 350

Gln Val Thr Lys Asp Val Thr Lys Ala Met Asp Glu Lys Arg Phe Asp
        355                 360                 365

Glu Ala Ile Lys Leu Arg Gly Arg Ser Phe Met Asn Asn Trp Glu Val
    370                 375                 380

Tyr Lys Leu Leu Ala His Val Arg Pro Pro Val Ser Lys Gly Gly Leu
385                 390                 395                 400

His Thr Val Ala Val Met Asn Val Gly Ala Pro Ala Ala Gly Met Asn
                405                 410                 415

Ala Ala Val Arg Ser Thr Val Arg Ile Gly Leu Ile Gln Gly Asn Arg
            420                 425                 430

Val Leu Val Val His Asp Gly Phe Glu Gly Leu Ala Lys Gly Gln Ile
        435                 440                 445

Glu Glu Ala Gly Trp Ser Tyr Val Gly Gly Trp Thr Gly Gln Gly Gly
    450                 455                 460

Ser Lys Leu Gly Thr Lys Arg Thr Leu Pro Lys Lys Asn Leu Glu Gln
465                 470                 475                 480

Ile Ser Ala Asn Ile Thr Lys Tyr Asn Ile Gln Gly Leu Val Ile Ile
                485                 490                 495

Gly Gly Phe Glu Ala Tyr Thr Gly Gly Leu Glu Leu Met Glu Gly Arg
            500                 505                 510

Lys Gln Phe Asp Glu Leu Cys Ile Pro Phe Val Val Ile Pro Ala Thr
        515                 520                 525

Val Ser Asn Asn Val Pro Gly Ser Asp Phe Ser Ile Gly Ala Asp Thr
    530                 535                 540

Ala Leu Asn Thr Ile Cys Thr Thr Cys Asp Arg Ile Lys Gln Ser Ala
545                 550                 555                 560

Ala Gly Thr Lys Arg Arg Val Phe Ile Ile Glu Thr Met Gly Gly Tyr
                565                 570                 575

Cys Gly Tyr Leu Ala Thr Met Ala Gly Leu Ala Ala Gly Ala Asp Ala
            580                 585                 590

Ala Tyr Ile Phe Glu Glu Pro Phe Thr Ile Arg Asp Leu Gln Val Asn
        595                 600                 605

Val Glu His Leu Val Gln Lys Met Lys Thr Thr Val Lys Arg Gly Leu
    610                 615                 620

Val Leu Arg Asn Glu Lys Cys Asn Glu Asn Tyr Thr Thr Asp Phe Ile
625                 630                 635                 640

Phe Asn Leu Tyr Ser Glu Glu Gly Lys Gly Ile Phe Asp Ser Arg Lys
                645                 650                 655

Asn Val Leu Gly His Met Gln Gln Gly Gly Asn Pro Thr Pro Phe Asp
            660                 665                 670

Arg Asn Phe Ala Thr Lys Met Gly Ala Lys Ala Thr Asn Trp Met Ser
        675                 680                 685

Gly Lys Ile Lys Glu Ser Tyr Arg Asn Gly Arg Ile Phe Ala Asn Thr
    690                 695                 700

Pro Asp Ser Gly Cys Val Leu Gly Met Arg Lys Arg Ala Leu Val Phe
705                 710                 715                 720

Gln Pro Val Thr Glu Leu Lys Asp Gln Thr Asp Phe Glu His Arg Ile
                725                 730                 735

Pro Lys Glu Gln Trp Trp Leu Lys Leu Arg Pro Ile Leu Lys Ile Leu
            740                 745                 750

Ala Lys Tyr Glu Ile Asp Leu Asp Thr Ser Asp His Ala His Leu Glu
        755                 760                 765

His Ile Ser Arg Lys Arg Ser Gly
    770                 775

<210>  6

<211>  2757

<212>  DNA

<213>  Rattus norvegicus


<220>
<223>  cDNA: phosphofructokinase muscle isozyme

<400>  6
gccagcgagg agagctaaaa ctacaagagt ggatcatgac ccatgaagag catcatgaag      60

ccaaaaccct ggggatcggc aaggccatcg ccgtgttgac ctctggtgga gatgcccaag     120

gtatgaatgc tacggtcagg gctgtggtac gagttggcat cttcaccggg ctccgcgtct     180

tctttgtcca tgagggttac caaggcctgg tggatggtgg agagcacatc agggaggcca     240

cctgggagag cgtgtccatg atgctccagc tgggaggcac ggtgattgga agtgcccgat     300

gcaaggactt ccgggagcga gaaggacgac tccgagctgc ccacaacctg gtgaagcggg     360

ggatcaccaa tctgtgtgtc atcggaggcg atggcagcct cactggggct gacacttttc     420

gttcagagtg gagtgactta ttgaatgacc tccagaaaga tgggaagatc acagccgagg     480

agcgtacaaa gtccagctac ctgaacatcg ttttcctggt tggctcaatc gacaatgact     540

tctgtggcac tgatatgacc attggtactg actctgccct gcaccgcatt gtagagatcg     600

tggacgccat caccaccacc gctcagagcc accagaggac atttgtgtta gaagtgatgg 660
gccgccactg tggatacctg gcccttgtca cctctctgtc gtgtggggcc gactgggttt 720
tcattcccga gtgtccgcca gatgacgact gggaagaaca cctttgtcgc cgtctcagtg 780
agacaagaac ccgtggttct cgtctcaaca tcatcattgt tgctgagggt gcaatcgaca 840
aaaacgggaa gccaatcacc tcagaagaca tcaagaacct ggtggtaaag cgtcttggat 900
atgataccag ggtcactgtt ctgggacatg tacagagggg tgggacacca tcagcctttg 960
accggatcct gggcagcagg atgggtgtgg aagcagtgat ggcacttttg gaggggaccc 1020
cagacacccc agcctgtgtg gtgagcctct ctggtaatac ggctgtgcgc ctgcccctca 1080
tggagtgtgt ccaggtgacc aaagacgtga ccaaggctat ggatgagaag agatttgatg 1140
aagccattaa gctgagaggc cggagcttca tgaacaactg ggaggtatac aagcttctag 1200
ctcatgtcag acccccagtc tctaagggcg ggttgcacac ggtggctgtg atgaacgtgg 1260
gggccccagc tgctggaatg aatgctgcag ttcgctctac tgtgaggatt ggccttatcc 1320
aaggcaaccg agtgctggtc gtgcatgatg gctttgaggg tctggccaaa ggtcagattg 1380
aggaagctgg ctggagctat gttggaggct ggactggtca aggtggttcc aaacttggta 1440
ctaaaaggac tctacccaag aagaacctgg aacagatcag tgccaacata accaagtata 1500
acatccaggg cctggttatc attgggggct ttgaggctta cacagggggc ttggagctga 1560
tggagggcag gaagcagttt gatgagctct gcatcccatt tgtggtcatt cccgccacgg 1620
tttccaataa tgtcccaggg tcagacttca gcatcggggc tgacacagca ctgaacacca 1680
tctgcacgac ctgtgaccga atcaagcagt ctgcagcagg caccaagcgg cgagtgttta 1740
tcatcgagac gatgggtggt tactgtggct atctggccac catggcagga ctggcggctg 1800
gggctgatgc tgcctacatt tttgaggagc ccttcaccat ccgagatctc caggttaatg 1860
ttgaacatct ggtgcagaag atgaaaacaa ctgtgaagag aggcctggtg ctgaggaatg 1920
agaagtgcaa cgagaactac actactgatt tcattttcaa cctgtactct gaggagggga 1980
agggcatctt cgacagcagg aagaacgtgc ttggccacat gcagcagggt ggaaacccaa 2040
ctccctttga caggaatttt gccaccaaga tgggtgctaa ggctacgaat tggatgtctg 2100
ggaaaatcaa agagagttac cgtaatggac ggatctttgc caacactccc gactcaggtt 2160
gtgttctggg gatgcgtaag agggccctgg tctttcagcc agtgactgag ctgaaggacc 2220
agacagattt tgagcaccga atccccaaag aacagtggtg gctgaagctg aggccaatcc 2280
tcaaaatcct ggccaagtac gagattgatc tggacacctc tgaccacgcc cacctggagc 2340
acatttccag gaagcggtct ggagaagctg ctgtctagac cactttggag tgaggggaaa 2400
atcacctgat catggtcagc tcacaccctg gtagacctca gcccatggct tctcagtgtt 2460
gtagcccagc ccctaccctt ctaggtttcc ctgtactctg tacctgcaac caggatcact 2520
gtggccaggt gttgggggag gggtggtgag tgcctctcct aggtagctgt ccttccttgc 2580

accctggctt catctgtcac acaggctgga tgtctctagt gctactgcta gatttcagct 2640

tctcaagagt aaaagtgagc tttatttatt tctttgtgat aacaaagagt cctaggtcct 2700

ttccttgtac cacagtgaag tgtaactaca ctaataaaag ccagctggcc actgtga 2757

<210> 7

<211> 885

<212> PRT

<213> Rattus norvegicus

<220>
<223> Protein: Phosphodiesterase 1

<400> 7

Met Ala Arg Gln Gly Cys Leu Gly Ser Phe Gln Val Ile Ser Leu Phe
1               5                   10                  15

Thr Phe Ala Ile Ser Val Asn Ile Cys Leu Gly Phe Thr Ala Ser Arg
            20                  25                  30

Ile Lys Arg Ala Glu Trp Asp Glu Gly Pro Pro Thr Val Leu Ser Asp
        35                  40                  45

Ser Pro Trp Thr Asn Thr Ser Gly Ser Cys Lys Gly Arg Cys Phe Glu
    50                  55                  60

Leu Gln Glu Val Gly Pro Pro Asp Cys Arg Cys Asp Asn Leu Cys Lys
65                  70                  75                  80

Ser Tyr Ser Ser Cys Cys His Asp Phe Asp Glu Leu Cys Leu Lys Thr
                85                  90                  95

Val Arg Gly Trp Glu Cys Thr Lys Asp Arg Ser Gly Glu Val Arg Asn
            100                 105                 110

Glu Glu Asn Ala Cys His Cys Pro Glu Asp Cys Leu Ser Arg Gly Asp
        115                 120                 125

Cys Cys Thr Asn Tyr Gln Val Val Cys Lys Gly Glu Ser His Trp Val
    130                 135                 140

Asp Asp Ala Ala Arg Asn Gln Ser Ser Glu Cys Leu Gln Val Cys Pro
145                 150                 155                 160

Pro Pro Leu Ile Ile Phe Ser Val Asp Gly Phe Arg Ala Ser Tyr Met
                165                 170                 175

Lys Lys Gly Ser Lys Val Met Pro Asn Ile Glu Lys Leu Arg Ser Cys
            180                 185                 190

Gly Thr His Val Pro Tyr Thr Arg Pro Val Tyr Pro Thr Lys Thr Phe
        195                 200                 205

Pro Asn Leu Tyr Thr Leu Ala Thr Gly Leu Tyr Pro Glu Ser His Gly
    210                 215                 220

Ile Val Gly Asn Ser Met Tyr Asp Pro Val Phe Asp Ala Ser Phe His
225                 230                 235                 240

Leu Arg Gly Arg Glu Lys Phe Asn His Arg Trp Trp Gly Gly Gln Pro
                245                 250                 255

Leu Trp Ile Thr Ala Thr Lys Gln Gly Val Arg Ala Gly Thr Phe Phe
            260                 265                 270

Trp Ser Val Ser Ile Pro His Glu Arg Arg Ile Leu Thr Ile Leu Gln
        275                 280                 285

Trp Leu Ser Leu Pro Asp Asn Glu Arg Pro Ser Val Tyr Ala Phe Tyr
    290                 295                 300

Ser Glu Gln Pro Asp Phe Ser Gly His Lys Tyr Gly Pro Phe Gly Pro
305                 310                 315                 320

Glu Met Thr Asn Pro Leu Arg Glu Ile Asp Lys Thr Val Gly Gln Leu
                325                 330                 335

Met Asp Gly Leu Lys Gln Leu Arg Leu His Arg Cys Val Asn Val Ile
            340                 345                 350

Phe Val Gly Asp His Gly Met Glu Asp Val Thr Cys Asp Arg Thr Glu
        355                 360                 365

Phe Leu Ser Asn Tyr Leu Thr Asn Val Asp Asp Ile Thr Leu Val Pro
    370                 375                 380

Gly Thr Leu Gly Arg Ile Arg Ala Lys Ser Ile Asn Asn Ser Lys Tyr
385                 390                 395                 400

Asp Pro Lys Thr Ile Ile Ala Asn Leu Thr Cys Lys Lys Pro Asp Gln
                405                 410                 415

His Phe Lys Pro Tyr Met Lys Gln His Leu Pro Lys Arg Leu His Tyr
            420                 425                 430

Ala Asn Asn Arg Arg Ile Glu Asp Ile His Leu Leu Val Asp Arg Arg
        435                 440                 445

Trp His Val Ala Arg Lys Pro Leu Asp Val Tyr Lys Lys Pro Ser Gly
    450                 455                 460

Lys Cys Phe Phe Gln Gly Asp His Gly Phe Asp Asn Lys Val Asn Ser
465                 470                 475                 480

Met Gln Thr Val Phe Val Gly Tyr Gly Pro Thr Phe Lys Tyr Arg Thr
                485                 490                 495

Lys Val Pro Pro Phe Glu Asn Ile Glu Leu Tyr Asn Val Met Cys Asp
            500                 505                 510

Leu Leu Gly Leu Lys Pro Ala Pro Asn Asn Gly Thr His Gly Ser Leu
        515                 520                 525

Asn His Leu Leu Arg Thr Asn Thr Phe Arg Pro Thr Met Pro Asp Glu
    530                 535                 540

Val Ser Arg Pro Asn Tyr Pro Gly Ile Met Tyr Leu Gln Ser Glu Phe
545                 550                 555                 560

Asp Leu Gly Cys Thr Cys Asp Asp Lys Val Glu Pro Lys Asn Lys Leu
                565                 570                 575

Glu Glu Leu Asn Lys Arg Leu His Thr Lys Gly Ser Thr Glu Ala Glu
            580                 585                 590

Thr Gly Lys Phe Arg Gly Ser Lys His Glu Asn Lys Lys Asn Leu Asn
        595                 600                 605

Gly Ser Val Glu Pro Arg Lys Glu Arg His Leu Leu Tyr Gly Arg Pro
    610                 615                 620

Ala Val Leu Tyr Arg Thr Ser Tyr Asp Ile Leu Tyr His Thr Asp Phe
625                 630                 635                 640

Glu Ser Gly Tyr Ser Glu Ile Phe Leu Met Pro Leu Trp Thr Ser Tyr
                645                 650                 655

Thr Ile Ser Lys Gln Ala Glu Val Ser Ser Ile Pro Glu His Leu Thr
            660                 665                 670

Asn Cys Val Arg Pro Asp Val Arg Val Ser Pro Gly Phe Ser Gln Asn
        675                 680                 685

Cys Leu Ala Tyr Lys Asn Asp Lys Gln Met Ser Tyr Gly Phe Leu Phe
    690                 695                 700

Pro Pro Tyr Leu Ser Ser Ser Pro Glu Ala Lys Tyr Asp Ala Phe Leu
705                 710                 715                 720

Val Thr Asn Met Val Pro Met Tyr Pro Ala Phe Lys Arg Val Trp Ala
                725                 730                 735

Tyr Phe Gln Arg Val Leu Val Lys Lys Tyr Ala Ser Glu Arg Asn Gly
            740                 745                 750

Val Asn Val Ile Ser Gly Pro Ile Phe Asp Tyr Asn Tyr Asp Gly Leu
        755                 760                 765

Arg Asp Thr Glu Asp Glu Ile Lys Gln Tyr Val Glu Gly Ser Ser Ile
    770                 775                 780

Pro Val Pro Thr His Tyr Tyr Ser Ile Ile Thr Ser Cys Leu Asp Phe
785                 790                 795                 800

Thr Gln Pro Ala Asp Lys Cys Asp Gly Pro Leu Ser Val Ser Ser Phe
                805                 810                 815

Ile Leu Pro His Arg Pro Asp Asn Asp Glu Ser Cys Asn Ser Ser Glu
            820                 825                 830

Asp Glu Ser Lys Trp Val Glu Glu Leu Met Lys Met His Thr Ala Arg
        835                 840                 845

Val Arg Asp Ile Glu His Leu Thr Gly Leu Asp Phe Tyr Arg Lys Thr
    850                 855                 860

Ser Arg Ser Tyr Ser Glu Ile Leu Thr Leu Lys Thr Tyr Leu His Thr
865                 870                 875                 880

Tyr Glu Ser Glu Ile
                885

<210> 8

<211> 3216

<212> DNA

<213> Rattus norvegicus

<220>
<223> cDNA Phosphodiesterase 1

<400> 8
ggtacccaac agcctgaact cagagccccg agagcagagc attcagggca agcagaaaca 60

ccctgcagag gctttccaag aatccctcgg catggcaaga caaggctgtc tcgggtcatt 120

ccaggtaata tccttgttca cttttgccat cagtgtcaat atctgcttag gattcacagc 180

aagtcgaatt aagagggcag aatgggatga aggacctccc acagtgctgt ctgactctcc 240

atggaccaac acctctggat cctgcaaagg tagatgcttt gagcttcaag aggttggccc 300

tccagactgt cggtgtgaca acctgtgtaa gagctacagc agctgctgcc acgatttcga 360

tgagctctgt ttgaaaacag tccgaggctg ggagtgcacc aaagacagaa gtggggaagt 420

acgaaacgag gaaaatgcct gtcactgccc agaagactgc ttgtccaggg gagactgctg 480

taccaactac caagtggtct gcaaaggaga atcacactgg gtagatgatg ctgcgagaaa 540

tcaaagttcc gaatgcctgc aggtttgtcc gcctccgtta atcatcttct ctgtggatgg 600

tttccgtgca tcatacatga agaaaggcag caaggttatg cccaacattg agaaactgcg 660

gtcctgtggc acccatgtcc cctacacgag gcctgtgtac cccacaaaaa ccttccctaa 720

tctatatacg ctggccactg gtttatatcc ggaatcccat ggaattgtcg gtaattcaat 780

gtatgatcct gtctttgatg cttcgttcca tctacgaggg cgagagaagt ttaatcatag 840

gtggtgggga ggccaaccgc tatggattac agccaccaag caaggggtga gagctggaac 900

attcttttgg tctgtgagca tccctcatga acggaggatc ctaaccattc ttcagtggct 960

ttctctgcca gacaacgaga ggccttcagt ttatgccttc tactcagagc agcctgattt 1020

ttctggacac aagtacggcc cttttggccc tgagatgaca aatcctctga gggagattga 1080

caagaccgtg gggcagttaa tggatggact gaaacaactc aggctgcatc gctgtgtgaa 1140

cgttatcttt gttggagacc atggaatgga agatgtgaca tgtgacagaa ctgagttctt 1200

gagcaactat ctgactaatg tggatgacat tactttagtg cctggaactc tgggaagaat 1260

tcgagccaaa tctatcaata attctaaata tgaccctaaa accattattg ctaacctcac 1320

gtgcaaaaaa ccggatcagc actttaagcc ttacatgaaa cagcaccttc ccaaacggtt 1380

gcactatgcc aacaacagaa gaattgaaga catccattta ttggtcgatc gaagatggca 1440

tgttgcaagg aaacctttgg acgtttataa gaaaccatca ggaaaatgtt ttttccaggg 1500

tgaccacggc tttgataaca aggtcaatag catgcagact gttttcgtag gttatggccc 1560

aacttttaag tacaggacta aagtgcctcc atttgaaaac attgaacttt acaatgttat 1620

gtgcgatctc ctaggcttga agcccgctcc caataatgga actcatggaa gcttgaatca 1680

cctactgcgt acaaatacct ttaggccaac catgccagac gaagtcagcc gacctaacta 1740

cccagggatt atgtaccttc agtccgagtt tgacctgggc tgcacctgtg acgataaggt 1800

agagccaaag aacaaattgg aagaactcaa taaacgtctt cataccaaag gatcaacaga 1860

agctgaaacc gggaaattca gaggcagcaa acatgaaaac aagaaaaacc ttaatggaag 1920

tgttgaacct agaaaagaga gacatctcct gtatggacgg cctgcagtgc tctatcggac 1980

tagctatgat atcttatacc atacggactt tgaaagtggt tatagtgaaa tattcttaat 2040

gcctctctgg acatcgtata ccatttctaa gcaggctgag gtctccagca tcccagaaca 2100

cctgaccaac tgtgttcgtc ctgatgtccg tgtgtctcca ggattcagtc agaactgttt 2160

agcttataaa aatgataaac agatgtcata tggattcctt tttcctccct acctgagctc 2220

ctccccagaa gctaagtatg atgcattcct cgtaaccaac atggttccaa tgtaccccgc 2280

cttcaaacgt gtttgggctt atttccaaag ggttttggtg aagaaatatg cttcagaaag 2340

gaatggagtc aacgtaataa gtggaccgat ttttgactac aattacgatg gcctacgtga 2400

cactgaagat gaaattaaac agtatgtgga aggcagctct atacctgtcc ccacccacta 2460

ctacagcatc atcaccagct gcctggactt cactcagcct gcagacaagt gtgacggtcc 2520

cctctctgtg tcttccttca tccttcctca ccgacccgac aatgatgaga gctgtaatag 2580

ctccgaggat gagtcgaagt gggtagagga actcatgaag atgcacacag ctcgggtgcg 2640

ggacattgag cacctcactg gtctggattt ctaccggaag actagccgta gctattcgga 2700

aattctgacc ctcaagacat acctgcatac atatgagagc gagatttaac tttctgggcc 2760

tgggcagtgt agtcttagca actggtgtat atttttatat tgtgtttgta tttattaatt 2820

tgaaccagga cacaaacaaa caaagaaaca aacaaataaa aaaaaaaacc acttagtatt 2880

ttaatcctgt accaaatctg acatattaag ctgaatgact gtgctatttt ttttccttaa 2940

ttcttgattt agacagagtt gtgttctgag cagagtttat agtgaacact gaggctcaca 3000

atccaagtag aagctacgtg gatctacaag gtgctgcagg ttgaaaattt gcattgagga 3060

aatattagtt ttccagggca cagtcaccac gtgtagttct gttctgtttt gaaagactga 3120

ttttgtaaag gtgcattcat ctgctgttaa ctttgacaga catatttatg ccttatagac 3180

caagcttaaa tataataaat cacacattca gatttt 3216

<210> 9

<211> 1020

<212>  PRT

<213>  Rattus norvegicus



<220>
<223>  Protein: Na+, K+ ATPase alpha(+) isoform catalytic subunit

<400>  9

Met Gly Arg Gly Ala Gly Arg Glu Tyr Ser Pro Ala Ala Thr Thr Ala
1               5                   10                  15

Glu Asn Gly Gly Gly Lys Lys Lys Gln Lys Glu Lys Glu Leu Asp Glu
            20                  25                  30

Leu Lys Lys Glu Val Ala Met Asp Asp His Lys Leu Ser Leu Asp Glu
        35                  40                  45

Leu Gly Arg Lys Tyr Gln Val Asp Leu Ser Lys Gly Leu Thr Asn Gln
    50                  55                  60

Arg Ala Gln Asp Ile Leu Ala Arg Asp Gly Pro Asn Ala Leu Thr Pro
65                  70                  75                  80

Pro Pro Thr Thr Pro Glu Trp Val Lys Phe Cys Arg Gln Leu Phe Gly
                85                  90                  95

Gly Phe Ser Ile Leu Leu Trp Ile Gly Ala Leu Leu Cys Phe Leu Ala
            100                 105                 110

Tyr Gly Ile Leu Ala Ala Met Glu Asp Glu Pro Ser Asn Asp Asn Leu
        115                 120                 125

Tyr Leu Gly Ile Val Leu Ala Ala Val Val Ile Val Thr Gly Cys Phe
    130                 135                 140

Ser Tyr Tyr Gln Glu Ala Lys Ser Ser Lys Ile Met Asp Ser Phe Lys
145                 150                 155                 160

Asn Met Val Pro Gln Gln Ala Leu Val Ile Arg Glu Gly Glu Lys Met
                165                 170                 175

Gln Ile Asn Ala Glu Glu Val Val Val Gly Asp Leu Val Glu Val Lys
            180                 185                 190

Gly Gly Asp Arg Val Pro Ala Asp Leu Arg Ile Ile Ser Ser His Gly
        195                 200                 205

Cys Lys Val Asp Asn Ser Ser Leu Thr Gly Glu Ser Glu Pro Gln Thr
    210                 215                 220

Arg Ser Pro Glu Phe Thr His Glu Asn Pro Leu Glu Thr Arg Asn Ile
225                 230                 235                 240

Cys Phe Phe Ser Thr Asn Cys Val Glu Gly Thr Ala Arg Gly Ile Val
                245                 250                 255

Ile Ala Thr Gly Asp Arg Thr Val Met Gly Arg Ile Ala Thr Leu Ala
            260                 265                 270

Ser Gly Leu Glu Val Gly Gln Thr Pro Ile Ala Met Glu Ile Glu His
        275                 280                 285

Phe Ile Gln Leu Ile Thr Gly Val Ala Val Phe Leu Gly Val Ser Phe

290                 295                 300

Phe Val Leu Ser Leu Ile Leu Gly Tyr Ser Trp Leu Glu Ala Val Ile
305                 310                 315                 320

Phe Leu Ile Gly Ile Ile Val Ala Asn Val Pro Glu Gly Leu Leu Ala
                325                 330                 335

Thr Val Thr Val Cys Leu Thr Leu Thr Ala Lys Arg Met Ala Arg Lys
            340                 345                 350

Asn Cys Leu Val Lys Asn Leu Glu Ala Val Glu Thr Leu Gly Ser Thr
        355                 360                 365

Ser Thr Ile Cys Ser Asp Lys Thr Gly Thr Leu Thr Gln Asn Arg Met
    370                 375                 380

Thr Val Ala His Met Trp Phe Asp Asn Gln Ile His Glu Ala Asp Thr
385                 390                 395                 400

Thr Glu Asp Gln Ser Gly Ala Thr Phe Asp Lys Arg Ser Pro Thr Trp
                405                 410                 415

Thr Ala Leu Ser Arg Ile Ala Gly Leu Cys Asn Arg Ala Val Phe Lys
            420                 425                 430

Ala Gly Gln Glu Asn Ile Ser Val Ser Lys Arg Asp Thr Ala Gly Asp
        435                 440                 445

Ala Ser Glu Ser Ala Leu Leu Lys Cys Ile Glu Leu Ser Cys Gly Ser
    450                 455                 460

Val Arg Lys Met Arg Asp Arg Asn Pro Lys Val Ala Glu Ile Pro Phe
465                 470                 475                 480

Asn Ser Thr Asn Lys Tyr Gln Leu Ser Ile His Glu Arg Glu Asp Ser
                485                 490                 495

Pro Gln Ser His Val Leu Val Met Lys Gly Ala Pro Glu Arg Ile Leu
            500                 505                 510

Asp Arg Cys Ser Thr Ile Leu Val Gln Gly Lys Glu Ile Pro Leu Asp
        515                 520                 525

Lys Glu Met Gln Asp Ala Phe Gln Asn Ala Tyr Met Glu Leu Gly Gly
    530                 535                 540

Leu Gly Glu Arg Val Leu Gly Phe Cys Gln Leu Asn Leu Pro Ser Gly
545                 550                 555                 560

Lys Phe Pro Arg Gly Phe Lys Phe Asp Thr Asp Glu Leu Asn Phe Pro
                565                 570                 575

Thr Glu Lys Leu Cys Phe Val Gly Leu Met Ser Met Ile Asp Pro Pro
            580                 585                 590

Arg Ala Ala Val Pro Asp Ala Val Gly Lys Cys Arg Ser Ala Gly Ile
        595                 600                 605

Lys Val Ile Met Val Thr Gly Asp His Pro Ile Thr Ala Lys Ala Ile
    610                 615                 620

Ala Lys Gly Val Gly Ile Ile Ser Glu Gly Asn Glu Thr Val Glu Asp
625                 630                 635                 640

Ile Ala Ala Arg Leu Asn Ile Pro Val Ser Gln Val Asn Pro Arg Glu

                645                 650                 655

Ala Lys Ala Cys Val Val His Gly Ser Asp Leu Lys Asp Met Thr Ser
            660                 665                 670

Glu Gln Leu Asp Glu Ile Leu Arg Asp His Thr Glu Ile Val Phe Ala
        675                 680                 685

Arg Thr Ser Pro Gln Gln Lys Leu Ile Ile Val Glu Gly Cys Gln Arg
    690                 695                 700

Gln Gly Ala Ile Val Ala Val Thr Gly Asp Gly Val Asn Asp Ser Pro
705                 710                 715                 720

Ala Leu Lys Lys Ala Asp Ile Gly Ile Ala Met Gly Ile Ser Gly Ser
                725                 730                 735

Asp Val Ser Lys Gln Ala Ala Asp Met Ile Leu Leu Asp Asp Asn Phe
            740                 745                 750

Ala Ser Ile Val Thr Gly Val Glu Glu Gly Arg Leu Ile Phe Asp Asn
        755                 760                 765

Leu Lys Lys Ser Ile Ala Tyr Thr Leu Thr Ser Asn Ile Pro Glu Ile
    770                 775                 780

Thr Pro Phe Leu Leu Phe Ile Ile Ala Asn Ile Pro Leu Pro Leu Gly
785                 790                 795                 800

Thr Val Thr Ile Leu Cys Ile Asp Leu Gly Thr Asp Met Val Pro Ala
                805                 810                 815

Ile Ser Leu Ala Tyr Glu Ala Ala Glu Ser Asp Ile Met Lys Arg Gln
            820                 825                 830

Pro Arg Asn Ser Gln Thr Asp Lys Leu Val Asn Glu Arg Leu Ile Ser
        835                 840                 845

Met Ala Tyr Gly Gln Ile Gly Met Ile Gln Ala Leu Gly Gly Phe Phe
    850                 855                 860

Thr Tyr Phe Val Ile Leu Ala Glu Asn Gly Phe Leu Pro Ser Arg Leu
865                 870                 875                 880

Leu Gly Ile Arg Leu Asp Trp Asp Asp Arg Thr Thr Asn Asp Leu Glu
                885                 890                 895

Asp Ser Tyr Gly Gln Glu Trp Thr Tyr Glu Gln Arg Lys Val Val Glu
            900                 905                 910

Phe Thr Cys His Thr Ala Phe Phe Ala Ser Ile Val Val Val Gln Trp
        915                 920                 925

Ala Asp Leu Ile Ile Cys Lys Thr Arg Arg Asn Ser Val Phe Gln Gln
    930                 935                 940

Gly Met Lys Asn Lys Ile Leu Ile Phe Gly Leu Leu Glu Glu Thr Ala
945                 950                 955                 960

Leu Ala Ala Phe Leu Ser Tyr Cys Pro Gly Met Gly Val Ala Leu Arg
                965                 970                 975

Met Tyr Pro Leu Lys Val Thr Trp Trp Phe Cys Ala Phe Pro Tyr Ser
            980                 985                 990

Leu Leu Ile Phe Ile Tyr Asp Glu Val Arg Lys Leu Ile Leu Arg Arg

995                 1000                1005

Tyr Pro Gly Gly Trp Val Glu Lys Glu Thr Tyr Tyr
    1010                1015                1020

<210> 10

<211> 5109

<212> DNA

<213> Rattus norvegicus

<220>
<223> cDNA: Na+, K+ ATPase alpha(+) isoform catalytic subunit

<400> 10
tctgccaggg tctccagctg ccccagacag gcggtgtggt cttgggatcc tcctggtgac      60

ctttccagcc taggtcccct cagccactct gccccaagat gggacgtggg gcagggcgtg     120

agtactcgcc tgccgccacc actgcggaga atgggggtgg caagaagaaa cagaaagaga     180

aggagctcga tgagctgaag aaggaggttg ccatggatga ccacaagctg tccttggatg     240

agctgggccg aaaataccaa gtggatctgt ccaagggcct caccaaccag cgagctcagg     300

atattctggc tagagacgga cccaacgccc tcactccacc ccctacaact cctgagtggg     360

tcaagttctg ccgtcagctt tttgggggct tctctatcct gctgtggatt ggggcgcttc     420

tctgcttctt agcctatggt atcctggccg ccatggagga cgaaccatcc aatgacaatt     480

tatatctagg tatcgtgcta gcagctgtag ttatcgtcac tggctgcttc tcctactacc     540

aggaagccaa aagctccaag attatggact ccttcaagaa catggtgcct cagcaagctc     600

tggtcatccg agagggagag aagatgcaga tcaatgcaga ggaggtggtc gtgggagacc     660

tggtggaagt gaagggtgga gaccgtgtcc ccgctgacct ccggatcatc tcctcccacg     720

gttgcaaggt ggataactca tccctgacag gggagtcgga gccccagacc cggtcccctg     780

agttcaccca tgagaatccc ttggagaccc gcaatatctg tttcttctct accaactgtg     840

tggaaggcac tgccaggggc attgtgatcg ccacaggtga ccggacggtg atgggccgca     900

tagccactct tgcctctggc ctagaggtgg gacagacgcc gatagccatg gagatcgagc     960

atttcatcca gctgatcacg ggggtggccg tgttcctggg ggtctccttc tttgttctgt    1020

cgctcatcct gggctacagc tggctggagg ctgtcatctt cctcatcggc atcatcgtag    1080

ccaacgtccc cgaagggctc ttggccactg ttactgtgtg cctgacgctg acagccaagc    1140

gcatggctcg caagaactgc ctggtgaaga acctggaggc ggtggagacg ctgggctcca    1200

cgtccaccat ctgctcggac aagacaggca ccctcaccca gaaccgcatg acggtggctc    1260

acatgtggtt tgacaaccag atccatgagg ctgacaccac tgaagatcag tctggggcca    1320

cttttgacaa gcggtccccg acgtggacag ccctgtctcg gatcgctggt ctctgcaatc    1380

gtgccgtctt caaggctggg caggagaaca tctccgtgtc taagcgggac acagctggtg    1440

acgcctctga gtcagctctg ctcaagtgca tcgagttgtc ctgtggctca gtgaggaaga 1500
tgagggacag gaatcccaag gtggcagaaa ttcccttcaa ctctaccaac aaatatcagc 1560
tttccatcca tgagagggaa gacagccccc agagccatgt gctggtgatg aaaggtgccc 1620
cggagcgcat cctggaccga tgctctacca tcctggtaca gggcaaggag atccctcttg 1680
acaaggagat gcaagatgcc tttcaaaacg cctacatgga gctgggagga ctcggggagc 1740
gagtgctggg cttctgtcag ctgaacctgc cttctggaaa gtttcctcgg ggcttcaaat 1800
ttgacacgga tgagctgaac tttcccacag agaagctctg ctttgtgggg ctcatgtcta 1860
tgattgatcc ccccagagca gctgtgccag atgctgtggg caagtgcaga agtgcaggca 1920
tcaaggtgat catggtgact ggggatcacc ctatcacagc caaggccatt gccaaaggtg 1980
tgggcatcat atcagagggt aacgagactg tggaagacat tgcagccagg ctcaacattc 2040
ctgtgagtca agtcaatccc agagaagcca aggcatgtgt agtgcacggc tcagacctga 2100
aggacatgac ttcagagcag ctggatgaga tcctcaggga ccacacggag atcgtgtttg 2160
cccggacctc ccctcagcag aagctcatca ttgtggaggg ctgtcagagg cagggagcca 2220
tcgtggcagt gactggtgac ggggtgaacg actcccccgc gctgaagaag gctgacattg 2280
gcattgccat gggcatctct ggctctgatg tctctaagca ggcagctgac atgatccttc 2340
tcgacgacaa ctttgcctcc attgtgacgg gcgtggagga ggggcgcctg atctttgaca 2400
acctgaagaa gtccatcgcg tacaccctga ccagcaacat ccctgagatc acccccttcc 2460
tgctgttcat cattgccaac atcccccttc ccctgggcac cgtgaccatc ctgtgcatcg 2520
acctgggcac agacatggtt cctgccatct cattagcata cgaagcggct gagagcgaca 2580
tcatgaagag gcagccacgg aactcccaga cggacaagct ggtgaacgag aggcttatca 2640
gcatggctta cggacagatc ggcatgatcc aggctctggg cggcttcttc acctactttg 2700
taatactggc agagaacggc ttcctgccat cgaggctgct tgggatccgc cttgactggg 2760
atgatcggac taccaacgac ctggaggaca gctatggaca agagtggacc tatgagcagc 2820
ggaaggtggt ggagttcaca tgccacacgg ccttctttgc cagcatcgtg gttgtgcagt 2880
gggctgacct catcatttgc aagacccggc gcaactcggt gttccagcag ggcatgaaga 2940
acaagatcct gatttttggg ctgctagaag agacggctct ggctgccttc ctgtcttact 3000
gcccgggtat gggggtggcc ctccgaatgt acccactcaa ggtcacgtgg tggttctgtg 3060
cctttcccta cagtctcctc atcttcatct atgatgaagt ccgaaagctc atcctgcggc 3120
ggtaccctgg gggctgggtg gagaaggaga cgtactactg agctcaccga caaaaggaag 3180
aacaggggag atggggtgct ccagaggggc tggtgggtgt tgtggtgaag ggaagggctg 3240
gggagacaca aggaagcgat ggtggcgtga actcagtggg taggcttggg taaataaact 3300
tgaggagact gctccaactg ctccataggt cccgctgtga accctaagac agtgcatgtt 3360
ggggtcgcct cctcagatcc ttcccgtccc actctcccac attgtctaca ttttctgaag 3420

aaccgggggt cgccctagcc ctccctgtgt cccagtcctt caccctcacc tgcattattc 3480
cattattcaa acagatcaac acccaaaggt taatcctgtc taaccctgga ggaaagcctg 3540
tcagaccacc agtacccacc actaccacca ccaccaccac caccaccacc accaccacca 3600
ccaccaccac caccccctgt tcactcctct tcccattctt gccttcctca ccttcctgcc 3660
tgagtcttcc cttgctcctc cccttacacc ttgaaaacac aaaattctgc ttctgtgagt 3720
gcaagagcct agggccagaa aaggaagcca gttggagaga tggggcctgt ctcccagcca 3780
agaccagtca ggaaccagag gggagctggg ctggcaagtg gaggttgggg tgcactggct 3840
gagaaagaaa gaaaggaagg aaggaaggaa ggaaggaagg aaggaagaaa ggaaggaagg 3900
aagtgaccac aggtgtgtca tctccagcct tcaggtatat gggacaggct ctggatctgt 3960
gagagactta agagactagc acaccagcag accaaattcc catctcatca gactagcagt 4020
aagtgccacc cagtgcccct ctgacccttg ggtagtggtc ctctctgtcc acaaggctca 4080
gatttcacag aaggttcagc tatctcaacc acatactctt gggaacaccc cccttcttta 4140
gaataattag ttctctgggg cctcgtgctg ttctgagagc cccattagct gccacttctc 4200
ctcgtgctct ctcactgcct tctgcttcct acccacctgc tgaacccacg ttatgtccag 4260
tatcgccttg cttgtcctga aaaaggatct cttggccatt ggcaggaatc agtgtagaaa 4320
tgtttccagg acatccctga ctttcgggaa catgcagaat cagtgtagct catgacacag 4380
tcagaaactt tagacacaag agaaattctt aagagaccta tgcacctttg acctctcaga 4440
ttgagacagg aagctggctt caggttccta tggcatagag ttatctttcc ttactctctt 4500
ctcaacctaa cgtattcgct cttcagacag ctgcctgttc tctaatcctg gcctagaaag 4560
catagcatag atgcacctgg atcaatgagg gaagcaagag agaatcagca aggaaactgg 4620
aaggcttgag gtgggaatat gaaagtcaag acaagcatca agccaggccc aagggcctcc 4680
caaaggctac tgtctcatcg tggtggatgg agttttgcct taccctaaat accttgaaat 4740
ttgtcagtca tgcatagcct tcagtcggaa gtcaaaatgg gacaccgtat ttatttgggc 4800
ctgactaatt tgagatcact gactttgaac aaaagtttac ctttgcacaa tcaataaaat 4860
catctgctag gtaattcaag agcataaacg atactgctag gagcagcata gttagtttca 4920
aagtatgctt tccgagcact ttagcaatct ccctttagaa tcaggaagtg cataggctaa 4980
ttactatcag tcccgatata tttgttaaag gaacacctac aagatcctta ctggtgacct 5040
tctgtgagac actagtttga ggcactacat gtgtacttga aaataataaa gttgcatttc 5100
tttatgaat 5109

<210> 11
<211> 617
<212> PRT

<213>  Mus musculus

<220>
<223>  Protein: vacuolar adenosine triphosphatase subunit A

<400>  11

Met Asp Phe Ser Lys Leu Pro Lys Ile Arg Asp Glu Asp Lys Glu Ser
1               5                   10                  15

Thr Phe Gly Tyr Val His Gly Val Ser Gly Pro Val Val Thr Ala Cys
            20                  25                  30

Asp Met Ala Gly Ala Ala Met Tyr Glu Leu Val Arg Val Gly His Ser
        35                  40                  45

Glu Leu Val Gly Glu Ile Ile Arg Leu Glu Gly Asp Met Ala Thr Ile
    50                  55                  60

Gln Val Tyr Glu Glu Thr Ser Gly Val Ser Val Gly Asp Pro Val Leu
65                  70                  75                  80

Arg Thr Gly Lys Pro Arg Ser Val Glu Leu Gly Pro Gly Ile Met Gly
                85                  90                  95

Ala Ile Phe Asp Gly Ile Gln Arg Pro Leu Ser Asp Ile Ser Ser Gln
            100                 105                 110

Thr Gln Ser Ile Tyr Ile Pro Arg Gly Val Asn Val Ser Ala Leu Ser
        115                 120                 125

Arg Asp Ile Lys Trp Glu Phe Ile Pro Ser Lys Asn Leu Arg Val Gly
    130                 135                 140

Ser His Ile Thr Gly Gly Asp Ile Tyr Gly Ile Val Asn Glu Asn Ser
145                 150                 155                 160

Leu Ile Lys His Lys Ile Met Leu Pro Pro Arg Asn Arg Gly Ser Val
                165                 170                 175

Thr Tyr Ile Ala Pro Pro Gly Asn Tyr Asp Ala Ser Asn Val Val Leu
            180                 185                 190

Glu Leu Glu Phe Glu Gly Val Lys Glu Lys Phe Ser Met Val Gln Val
        195                 200                 205

Trp Pro Val Arg Gln Val Arg Pro Val Thr Glu Lys Leu Pro Ala Asn
    210                 215                 220

His Pro Leu Leu Thr Gly Gln Arg Val Leu Asp Ala Leu Phe Pro Cys
225                 230                 235                 240

Val Gln Gly Gly Thr Thr Ala Ile Pro Gly Ala Phe Gly Cys Gly Lys
                245                 250                 255

Thr Val Ile Ser Gln Ser Leu Ser Lys Tyr Ser Asn Ser Asp Val Ile
            260                 265                 270

Ile Tyr Val Gly Cys Gly Glu Arg Gly Asn Glu Met Ser Glu Val Leu
        275                 280                 285

Arg Asp Phe Pro Glu Leu Thr Met Glu Val Asp Gly Lys Ala Glu Ser
    290                 295                 300

Ile Met Lys Arg Thr Ala Leu Val Ala Asn Thr Ser Asn Met Pro Val
305                 310                 315                 320

Ala Ala Arg Glu Ala Ser Ile Tyr Thr Gly Ile Thr Leu Ser Glu Tyr
                325                 330                 335

Phe Arg Asp Met Gly Tyr His Val Ser Met Met Ala Asp Ser Thr Ser
            340                 345                 350

Arg Trp Ala Glu Ala Leu Arg Glu Ile Ser Gly Arg Leu Ala Glu Met
        355                 360                 365

Pro Ala Asp Ser Gly Tyr Pro Ala Tyr Leu Gly Ala Arg Leu Ala Ser
    370                 375                 380

Phe Tyr Glu Arg Ala Gly Arg Val Lys Cys Leu Gly Asn Pro Glu Arg
385                 390                 395                 400

Glu Gly Ser Val Ser Ile Val Gly Ala Val Ser Pro Pro Gly Gly Asp
                405                 410                 415

Phe Ser Asp Pro Val Thr Ser Ala Thr Leu Gly Ile Val Gln Val Phe
            420                 425                 430

Trp Gly Leu Asp Lys Lys Leu Ala Gln Arg Lys His Phe Pro Ser Val
        435                 440                 445

Asn Trp Leu Ile Ser Tyr Ser Lys Tyr Met Arg Ala Leu Asp Glu Tyr
    450                 455                 460

Tyr Asp Lys His Phe Thr Glu Phe Val Pro Leu Arg Thr Lys Ala Lys
465                 470                 475                 480

Glu Ile Leu Gln Glu Glu Gly Asp Leu Ala Glu Ile Val Gln Leu Val
                485                 490                 495

Gly Lys Ala Ser Leu Ala Glu Thr Asp Lys Ile Thr Leu Glu Val Ala
            500                 505                 510

Lys Leu Ile Lys Asp Asp Phe Leu Gln Gln Asn Gly Tyr Thr Pro Tyr
        515                 520                 525

Asp Arg Phe Cys Pro Phe Tyr Lys Thr Val Gly Met Leu Ser Asn Met
    530                 535                 540

Ile Ser Phe Tyr Asp Met Ala Arg Arg Ala Val Glu Thr Thr Ala Gln
545                 550                 555                 560

Ser Asp Asn Lys Ile Thr Trp Ser Ile Ile Arg Glu His Met Gly Glu
                565                 570                 575

Ile Leu Tyr Lys Leu Ser Ser Met Lys Phe Lys Asp Pro Val Lys Asp
            580                 585                 590

Gly Glu Ala Lys Ile Lys Ala Asp Tyr Ala Gln Leu Leu Glu Asp Met
        595                 600                 605

Gln Asn Ala Phe Arg Ser Leu Glu Asp
    610                 615

<210>  12

<211>  3007

<212>  DNA

<213>  Mus musculus

<220>
<223>  cDNA: vacuolar adenosine triphosphatase subunit A

<400>  12
gcaggtaaat ttaacacaat ggatttctcc aagctaccca aaatccgaga tgaggataaa      60

gaaagtacat ttggttatgt gcatggagtc tcagggcctg tggttacagc ctgtgacatg     120

gcgggcgctg ccatgtacga gctggtgaga gtggggcaca gcgagctggt tggagaaatt     180

attcgattgg aaggtgacat ggccaccatt caggtgtatg aagaaacttc tggtgtgtct     240

gttggagacc ccgtactccg cactggtaaa cctcgctcgg tcgagctggg tcccgggatt     300

atgggagcca tttttgatgg tatacagaga cctctgtcgg atatcagcag tcagacccaa     360

agtatctaca tccccagagg agtcaatgtg tctgctctca gcagagatat caaatgggag     420

tttataccca gcaaaaacct acgggttggt agtcatatca ctggtggaga catttatggg     480

attgtcaatg agaactccct catcaaacac aaaatcatgt tgcccccacg taacagagga     540

agcgtgactt acatcgcgcc gcctgggaat tatgatgcat cgaatgtcgt cctggagctt     600

gagtttgaag gtgtgaagga gaagttcagc atggtccaag tgtggcctgt gcggcaggtg     660

cggcctgtca ctgagaagct gcccgccaat caccccttac ttactggcca gagagtcctc     720

gatgcccttt tcccgtgtgt tcagggagga actactgcta tcccaggcgc ctttggctgt     780

ggaaagactg tgatttccca gtctctatcc aagtactcca acagtgacgt catcatctat     840

gtcggctgcg gtgagagagg caacgagatg tcagaagttc tccgagactt ccctgagctc     900

accatggagg ttgatgggaa agcagagtcc atcatgaaga ggacagcgct ggtagccaac     960

acctccaaca tgcctgtggc tgcgagagag gcctccatct acactggaat tacactatca    1020

gaatatttcc gtgacatggg ctaccacgtc agtatgatgg ccgactctac ctctagatgg    1080

gctgaggccc tcagagaaat ctctggtcgt ttagctgaga tgcctgcaga tagtggatac    1140

cctgcatacc ttggtgcccg gctggcttct ttctatgagc gagcaggcag agtgaaatgt    1200

ctcggaaacc ctgagagaga agggagtgtc agcattgtag gagcagtttc tccacctggt    1260

ggtgattttt ctgatccagt cacttctgca acgctgggta ttgttcaggt gttctggggc    1320

ttggataaga agttagctca gcgcaagcac ttcccctctg tcaactggct catcagctac    1380

agcaagtaca tgcgtgccct ggacgagtac tatgacaagc acttcacgga gtttgttcct    1440

ctgaggacca aagctaagga gatcctgcag gaagaagggg atctggcaga aatcgtgcag    1500

ctcgtgggca aggcctcttt agcagagacg gataaaatca ctctggaggt agcaaaactt    1560

attaaagatg acttcctaca gcaaaatggg tacactcctt atgacaggtt ctgtccattc    1620

tacaagacag tggggatgct gtccaacatg atttcattct atgacatggc ccgccgagct    1680

gtggagacca ctgcccagag tgacaataag atcacatggt ccattatccg ggagcacatg    1740

ggggagattc tctataaact ctcctccatg aaattcaagg atccagtgaa agatggcgag 1800

gcaaagatca aggccgacta tgcacagctt cttgaagata tgcagaatgc attccgtagc 1860

cttgaagact agaactgtga tttcttccct cttccacggc aagctcatac gtgtatattt 1920

tcctgaattt ctcatctcca acccttagct tccatattgt gcagctttga gactagtgcc 1980

tatgtgtgtt cttgttcatt ttgctgtttc tttggtaggt cttataaaac aaacattcct 2040

ttgttccagt gtttgaagga gctagctccc ttacctttat ctgaagtggt gaatgtagtg 2100

catatgatat acattgtaag atacacattg taacatgatc catactgtaa acttgtatgt 2160

aaggtgacta ccccttccct catctccagt aaactgtaaa caggactact gcatgtactc 2220

tgttgggaat ggaaggccag aactccatac cgtggatgga tgggtactta ggaaacaact 2280

cagcatttgt agtcagacca cttgtaactt agtggtttgt tgagtaacca ttttgcagga 2340

aatacttcca tttaaaaaca taaaagatta atgttccaat tatttgtatc aatcaggacc 2400

atttttgtgg ggcacttggg aactatttgt ttttcaaaca gacatttgca agactgaaca 2460

taatagataa atcagttacc tctgaaaatg tggaaagaaa gggaaaaaaa gaaccaggtg 2520

gtcaaactta aattgacatc atcttgttaa agcatatttt atttcactaa gagaaattta 2580

atatcagaga cttttatata ctcaattact aggaaacctt tttttaagta caatttaaaa 2640

atcattgaaa atgtgatcca catcatagcc attcttttcc tcagacttag tcagacaagc 2700

ttctcagagt ggtgggatgg ggattagaat accacagaca ctctgcagtg cctgcaggca 2760

gtcggcccca ggacaaccac tgctgtagga gtttggggcc agggtggcat ggttttcaca 2820

aggtacatgt gtcacgtgtt tgtttgcctg ttgacattct gaaaacagca agtttaccaa 2880

ttgcagaaaa tactttctgt tttctctttc acatgctcag aaagcttctc aaaggtatct 2940

ggtcacagca gcttccttcc tgttatagag gtaaaaggtg ttcttatatt taactggtaa 3000

caaaaga 3007

<210> 13

<211> 823

<212> PRT

<213> Rattus norvegicus

<220>
<223> Protein: hypoxia-inducible factor-1 alpha

<400> 13

Met Glu Gly Ala Gly Gly Glu Asn Glu Lys Lys Asn Arg Met Ser Ser
1               5                   10                  15

Glu Arg Arg Lys Glu Lys Ser Arg Asp Ala Ala Arg Ser Arg Arg Ser

                    20                  25                  30

Lys Glu Ser Glu Val Phe Tyr Glu Leu Ala His Gln Leu Pro Leu Pro
        35                  40                  45

His Asn Val Ser Ser His Leu Asp Lys Ala Ser Val Met Arg Leu Thr
    50                  55                  60

Ile Ser Tyr Leu Arg Val Arg Lys Leu Leu Gly Ala Gly Asp Leu Asp
65                  70                  75                  80

Ile Glu Asp Glu Met Lys Ala Gln Met Asn Cys Phe Tyr Leu Lys Ala
                85                  90                  95

Leu Asp Gly Phe Val Met Val Leu Thr Asp Asp Gly Asp Met Ile Tyr
            100                 105                 110

Ile Ser Asp Asn Val Asn Lys Tyr Met Gly Leu Thr Gln Phe Glu Leu
        115                 120                 125

Thr Gly His Ser Val Phe Asp Phe Thr His Pro Cys Asp His Glu Glu
    130                 135                 140

Met Arg Glu Met Leu Thr His Arg Asn Gly Pro Val Arg Lys Gly Lys
145                 150                 155                 160

Glu Gln Asn Thr Gln Arg Ser Phe Phe Leu Arg Met Lys Cys Thr Leu
                165                 170                 175

Thr Ser Arg Gly Arg Thr Met Asn Ile Lys Ser Ala Thr Trp Lys Val
            180                 185                 190

Leu His Cys Thr Gly His Ile His Val Tyr Asp Thr Ser Ser Asn Gln
        195                 200                 205

Pro Gln Cys Gly Tyr Lys Lys Pro Pro Met Thr Cys Leu Val Leu Ile
    210                 215                 220

Cys Glu Pro Ile Pro His Pro Ser Asn Ile Glu Ile Pro Leu Asp Ser
225                 230                 235                 240

Lys Thr Phe Leu Ser Arg His Ser Leu Asp Met Lys Phe Ser Tyr Cys
                245                 250                 255

Asp Glu Arg Ile Thr Glu Leu Met Gly Tyr Glu Pro Glu Glu Leu Leu
            260                 265                 270

Gly Arg Ser Ile Tyr Glu Tyr Tyr His Ala Leu Asp Ser Asp His Leu
        275                 280                 285

Thr Lys Thr His His Asp Met Phe Thr Lys Gly Gln Val Thr Thr Gly
    290                 295                 300

Gln Tyr Arg Met Leu Ala Lys Arg Gly Gly Tyr Val Trp Val Glu Thr
305                 310                 315                 320

Gln Ala Thr Val Ile Tyr Asn Thr Lys Asn Ser Gln Pro Gln Cys Ile
                325                 330                 335

Val Cys Val Asn Tyr Val Val Ser Gly Ile Ile Gln His Asp Leu Ile
            340                 345                 350

Phe Ser Leu Gln Gln Thr Glu Ser Val Leu Lys Pro Val Glu Ser Ser
        355                 360                 365

Asp Met Lys Met Thr Gln Leu Phe Thr Lys Val Glu Ser Glu Asp Thr

370                 375                 380

Ser Cys Leu Phe Asp Lys Leu Lys Lys Glu Pro Asp Ala Leu Thr Leu
385                 390                 395                 400

Leu Ala Pro Ala Ala Gly Asp Thr Ile Ile Ser Leu Asp Phe Gly Ser
                405                 410                 415

Asp Asp Thr Glu Thr Glu Asp Gln Gln Leu Glu Asp Val Pro Leu Tyr
            420                 425                 430

Asn Asp Val Met Phe Pro Ser Ser Asn Glu Lys Leu Asn Ile Asn Leu
        435                 440                 445

Ala Met Ser Pro Leu Pro Ala Ser Glu Thr Pro Lys Pro Leu Arg Ser
    450                 455                 460

Ser Ala Asp Pro Ala Leu Asn Gln Glu Val Ala Leu Lys Leu Glu Ser
465                 470                 475                 480

Ser Pro Glu Ser Leu Gly Leu Ser Phe Thr Met Pro Gln Ile Gln Asp
                485                 490                 495

Gln Pro Ala Ser Pro Ser Asp Gly Ser Thr Arg Gln Ser Ser Pro Glu
            500                 505                 510

Pro Asn Ser Pro Ser Glu Tyr Cys Phe Asp Val Asp Ser Asp Met Val
        515                 520                 525

Asn Val Phe Lys Leu Glu Leu Val Glu Lys Leu Phe Ala Glu Asp Thr
    530                 535                 540

Glu Ala Lys Asn Pro Phe Ser Ala Gln Asp Thr Asp Leu Asp Leu Glu
545                 550                 555                 560

Met Leu Ala Pro Tyr Ile Pro Met Asp Asp Asp Phe Gln Leu Arg Ser
                565                 570                 575

Phe Asp Gln Leu Ser Pro Leu Glu Ser Asn Ser Pro Ser Pro Pro Ser
            580                 585                 590

Val Ser Thr Val Thr Gly Phe Gln Gln Thr Gln Leu Gln Lys Pro Thr
        595                 600                 605

Ile Thr Val Thr Ala Thr Ala Thr Ala Thr Thr Asp Glu Ser Lys Ala
    610                 615                 620

Val Thr Lys Asp Asn Ile Glu Asp Ile Lys Ile Leu Ile Ala Ser Pro
625                 630                 635                 640

Pro Ser Thr Gln Val Pro Gln Glu Met Thr Thr Ala Lys Ala Ser Ala
                645                 650                 655

Tyr Ser Gly Thr His Ser Arg Thr Ala Ser Pro Asp Arg Ala Gly Lys
            660                 665                 670

Arg Val Ile Glu Lys Thr Asp Lys Ala His Pro Arg Ser Leu Asn Leu
        675                 680                 685

Ser Val Thr Leu Asn Gln Arg Asn Thr Val Pro Glu Glu Glu Leu Asn
    690                 695                 700

Pro Lys Thr Ile Ala Leu Gln Asn Ala Gln Arg Lys Arg Lys Met Glu
705                 710                 715                 720

His Asp Gly Ser Leu Phe Gln Ala Ala Gly Ile Gly Thr Leu Leu Gln

                725                 730                 735

Gln Pro Gly Asp Arg Ala Pro Thr Met Ser Leu Ser Trp Lys Arg Val
            740                 745                 750

Lys Gly Tyr Ile Ser Ser Glu Gln Asp Gly Met Glu Gln Lys Thr Ile
        755                 760                 765

Phe Leu Ile Pro Ser Asp Leu Ala Cys Arg Leu Leu Gly Gln Ser Met
    770                 775                 780

Asp Glu Ser Gly Leu Pro Gln Leu Thr Ser Tyr Asp Cys Glu Val Asn
785                 790                 795                 800

Ala Pro Ile Gln Gly Ser Arg Asn Leu Leu Gln Gly Glu Glu Leu Leu
                805                 810                 815

Arg Ala Leu Asp Gln Val Asn
            820

<210> 14

<211> 3718

<212> DNA

<213> Rattus norvegicus

<220>
<223> cDNA: hypoxia-inducible factor-1 alpha

<400> 14
   gacaccgcgg gcaccgattc gccatggagg gcgccggcgg cgagaacgag aagaaaaata 60
   ggatgagttc cgaacgtcga aaagaaaagt ctagggatgc agcacgatct cggcgaagca 120
   aagagtctga agttttttat gagcttgctc atcagttgcc acttccccac aacgtgagct 180
   cccatcttga taaagcttct gttatgaggc tcaccatcag ttacttacgt gtgaggaaac 240
   ttctaggtgc tggtgatctt gacattgaag atgaaatgaa agcacagatg aactgctttt 300
   atctgaaagc cctggatggc tttgttatgg tgctaacaga tgatggtgac atgatttaca 360
   tttctgataa cgtgaacaaa tacatggggt tgactcagtt tgaactaact ggacacagtg 420
   tgtttgattt tacccatcca tgtgaccatg aggaaatgag agaaatgctt acacacagaa 480
   atggcccagt gagaaagggg aaagaacaaa acacgcagcg aagctttttt ctcagaatga 540
   aatgtaccct aacaagccgg gggaggacga tgaacatcaa gtcagcaacg tggaaggtgc 600
   tgcactgcac aggccacatt catgtgtatg ataccagcag taaccagccg cagtgtggct 660
   acaagaaacc gcctatgacg tgcttggtgc tgatttgtga acccattcct catccatcaa 720
   acattgaaat tcctttagac agcaagacat ttctcagtcg acacagcctc gatatgaaat 780
   tttcttactg tgatgaaagg attactgagt tgatgggtta tgagccagaa gaacttttgg 840
   gccgttcaat ttatgaatat tatcatgctt tggactctga tcatctgacc aaaactcatc 900
   atgacatgtt tactaaagga caagtcacca caggacagta caggatgctt gcaaaaagag 960
   gtggatatgt ctgggttgag actcaagcaa ctgttatata taatacgaag aactctcagc 1020
   cacagtgcat tgtgtgtgtg aattatgttg taagtggtat tattcagcac gacttgattt 1080
   tctcccttca acaaacagaa tctgtcctca aaccagttga atcttcagat atgaaaatga 1140
   cccagctgtt cactaaagtg gaatctgagg acacgagctg cctcttcgac aagcttaaga 1200
   aagagcccga tgccctgact ctgctagctc cagcggctgg ggacacgatc atatcactgg 1260
   acttcggcag cgatgacacg gaaactgaag accaacaact tgaagatgtc ccgttgtaca 1320
   atgatgtaat gttcccctct tctaatgaga aattaaatat aaatctggca atgtctccat 1380
   tacctgcctc tgaaactcca aagccacttc gaagtagtgc tgatcctgca ctgaatcaag 1440
   aggttgcatt gaagttagag tcaagcccag agtcactggg actttctttt accatgcccc 1500
   agattcaaga tcagccagca agtccttctg atggaagcac tagacaaagc tcacctgagc 1560
   ctaacagtcc cagtgagtac tgctttgatg tggacagcga tatggtcaat gtattcaagt 1620
   tggaactggt ggaaaaactg tttgctgaag acacagaagc gaagaatcca ttttcagctc 1680
   aggacactga tttagacttg gaaatgctgg ctccctatat cccaatggat gatgatttcc 1740
   agttacgttc ctttgatcag ttgtcaccat tagagagcaa ttctccaagc cctccgagtg 1800
   tgagcacagt tacaggattc cagcagaccc agttacagaa acctaccatc actgtcactg 1860
   ccaccgcaac tgccaccact gatgaatcaa aagcagtgac gaaggacaat atagaagaca 1920
   ttaaaatact gattgcatct ccaccttcta cccaagtacc tcaagaaatg accactgcta 1980
   aggcatcagc atacagtggt actcacagtc ggacagcctc accagacaga gcaggaaaga 2040
   gagtcataga aaaaacagac aaagctcatc caaggagcct taacctatct gtcactttga 2100
   atcaaagaaa tactgttcct gaagaagaat taaacccaaa gacaatagct ttgcagaatg 2160
   ctcagaggaa gcgaaaaatg gaacatgatg gctccctttt tcaagcagca ggaattggaa 2220
   cgttactgca gcaaccaggt gaccgtgccc ctactatgtc gctttcttgg aaacgagtga 2280
   aaggatacat atctagtgaa caggatggaa tggagcagaa gacaattttt ttaataccct 2340
   ctgatttagc atgtagactg ctggggcagt caatggatga gagtggatta ccacagctga 2400
   ccagttacga ttgtgaagtt aatgctccca tacaaggcag cagaaaccta ctgcagggtg 2460
   aagaattact cagagctttg gatcaagtta actgagcttt tcctaatctc attcctttga 2520
   ttgttaattt ttgtgttcag ttgttgttgt tgtctgtggg gtttcgtttc tgttggttgt 2580
   tttggacact ggtggctcag cagtctattt atattttcta tatctcattt agaggcctgg 2640
   ctacagtact gcaccaactc agatagttta gtttgggccc cttcctcctt cattttcact 2700
   gatgctcttt ttaccatgtc cttcgaatgc cagatcacag cacattcaca gctccccagc 2760
   atttcaccaa tgcattgctg tagtgtcgtt taaaatgcac ctttttattt atttattttt 2820
   ggtgagggag tttgtccctt attgaattat ttttaatgaa atgccaatat aattttttaa 2880
   gaaggcagta aatcttcatc atgatgatag gcagttgaaa attttttact catttttttc 2940
   atgttttaca tgaaaataat gctttgccag cagtacatgg tagccacaat tgcacaatat 3000
   attttcttaa aaataccagc agttactcat gcatatattc tgcatttata aaactagttt 3060
   ttaagaagaa actttttttg gcctatggaa ttgttaagcc tggatcatga tgctgttgat 3120
   cttataatga ttcttaaact gtatggtttc tttatatggg taaagccatt tacatgatat 3180
   agagagatat gcttatatct ggaaggtata tggcatttat ttggataaaa ttctcaattg 3240
   agaagttatc tggtgtttct ttactttacc ggctcaaaag aaaacagtcc ctatgtagtt 3300
   gtggaagctt atgctaatat tgtgtaattg atattaaaca ttaaatgttc tgcctatcct 3360
   gttggtataa agacattttg agcatactgt aaacaaaaaa atcatgcatt gttagtaaaa 3420
   ttgcctagta tgttaatttg ttgaaaatac gatgtttggt tttatgcact ttgtcgctat 3480
   taacatcctt tttttcatat agatttcaat aattgagtaa ttttagaagc attattttag 3540
   aaatatagag ttgtcatagt aaacatcttg tttttttttc tttttttcta tgtacattgt 3600
   ataaattttt cattcccttg ctctttgtag ttgggtctaa cactaactgt actgttttgt 3660
   tatatcaaat aaacatcttc tgtggaccag gaaaaaaaaa aaaaaaaaaa aaaaaaaa 3718

<210> 15

<211> 227

<212> PRT

<213> Rattus norvegicus

<220>
<223> Protein: cytochrome c oxidase subunit II

<400> 15

Met Ala Tyr Pro Phe Gln Leu Gly Leu Gln Asp Ala Thr Ser Pro Ile
1               5                   10                  15

Met Glu Glu Leu Thr Asn Phe His Asp His Thr Leu Met Ile Val Phe
            20                  25                  30

Leu Ile Ser Ser Leu Val Leu Tyr Ile Ile Ser Leu Met Leu Thr Thr
        35                  40                  45

Lys Leu Thr His Thr Ser Thr Met Asp Ala His Glu Val Glu Thr Ile
    50                  55                  60

Trp Thr Ile Leu Pro Ala Val Ile Leu Ile Leu Ile Ala Leu Pro Ser
65                  70                  75                  80

Leu Arg Ile Leu Tyr Met Met Asp Glu Ile Asn Asn Pro Val Leu Thr
                85                  90                  95

Val Lys Thr Met Gly His Gln Trp Tyr Trp Ser Tyr Glu Tyr Thr Asp
            100                 105                 110

Tyr Glu Asp Leu Cys Phe Asp Ser Tyr Met Ile Pro Thr Asn Asp Leu
        115                 120                 125

Lys Leu Gly Glu Leu Arg Leu Leu Glu Val Asp Asn Arg Val Val Leu

130                 135                 140

Pro Met Glu Leu Pro Ile Arg Met Leu Ile Ser Ser Glu Asp Val Leu
145                 150                 155                 160

His Ser Trp Pro Ile Pro Ser Leu Gly Leu Lys Thr Asp Ala Ile Pro
                165                 170                 175

Gly Arg Pro Asn Gln Ala Thr Val Thr Ser Asn Arg Leu Gly Leu Phe
            180                 185                 190

Tyr Gly Gln Cys Ser Glu Ile Cys Gly Ser Asn His Ser Phe Met Leu
        195                 200                 205

Ile Val Leu Glu Met Val Pro Leu Lys Tyr Phe Glu Asn Trp Ser Ala
    210                 215                 220

Ser Met Ile
225

<210>  16

<211>  686

<212>  DNA

<213>  Rattus norvegicus


<220>
<223> cDNA: cytochrome c oxidase subunit II

<400>  16
acatggctta cccatttcaa cttggcttac aagacgccac atcaccaatc atagaagaac      60

ttacaaactt tcatgaccac accctaataa ttgtattcct catcagctcc ctagtacttt     120

atattatttc actaatacta acaacaaaac taacacacac aagcacaata gacgcccatg     180

aagtagaaac aatttgaaca attctcccag ctgtcattct tattctaatc gcccttccct     240

ccctacgaat tctatacata atagacgaga ttaataaccc agttctaaca gtaaaaacta     300

taggacacca atgatactga agctatgaat atactgacta tgaagaccta tgctttgact     360

cctacataat cccaaccaat gacctaaaac taggtgaact tcgcttatta gaagttgata     420

atcgggtagt cttaccaata gaacttccaa ttcgtatact aatctcatcc gaagacgtcc     480

tgcactcatg acccatccct tcactagggt taaaaaccga cgcaatcccc ggccgcccga     540

accaagctac agtcacatca aaccgactag gtctattcta tggccaatgc tctgaaattt     600

gcggctcaaa tcacagcttc atactcattg tactagaaat agtgcctcta aaatatttcg     660

aaaactgatc agcttctata atttaa                                          686

<210>  17

<211>  260

<212>  PRT

<213>  Rattus norvegicus

<220>
<223> Protein: HYDROXYACYLGLUTATHIONE HYDROLASE

<400>  17

Met Lys Ile Glu Leu Leu Pro Ala Leu Thr Asp Asn Tyr Met Tyr Leu
1               5                   10                  15

Ile Ile Asp Glu Asp Thr Gln Glu Ala Ala Val Val Asp Pro Val Gln
            20                  25                  30

Pro Gln Lys Val Ile Glu Thr Val Lys Lys His Arg Val Lys Leu Thr
        35                  40                  45

Thr Val Leu Thr Thr His His His Trp Asp His Ala Gly Gly Asn Glu
    50                  55                  60

Lys Leu Val Lys Leu Glu Pro Gly Leu Lys Val Tyr Gly Gly Asp Asp
65                  70                  75                  80

Arg Ile Gly Ala Leu Thr His Lys Val Thr His Leu Ser Thr Leu Glu
                85                  90                  95

Val Gly Ser Leu Ser Val Lys Cys Leu Ser Thr Pro Cys His Thr Ser
            100                 105                 110

Gly His Ile Cys Tyr Phe Val Ser Lys Pro Gly Ser Ser Glu Pro Ser
        115                 120                 125

Ala Val Phe Thr Gly Asp Thr Leu Phe Val Ala Gly Cys Gly Lys Phe
    130                 135                 140

Tyr Glu Gly Thr Ala Asp Glu Met Tyr Lys Ala Leu Leu Glu Val Leu
145                 150                 155                 160

Gly Arg Leu Pro Pro Asp Thr Lys Val Ile Cys Gly His Glu Tyr Thr
                165                 170                 175

Val Asn Asn Leu Lys Phe Ala Arg His Val Glu Pro Gly Asn Thr Ala
            180                 185                 190

Val Gln Glu Lys Leu Ala Trp Ala Lys Glu Lys Asn Ala Ile Gly Glu
        195                 200                 205

Pro Thr Val Pro Ser Thr Leu Ala Glu Glu Phe Thr Tyr Asn Pro Phe
    210                 215                 220

Met Arg Val Lys Glu Lys Thr Val Gln Gln His Ala Gly Glu Thr Asp
225                 230                 235                 240

Pro Val Thr Thr Met Arg Ala Ile Arg Arg Glu Lys Asp Gln Phe Lys
                245                 250                 255

Val Pro Arg Asp
            260

<210>  18

<211>  504

<212>  PRT

<213>  Homo sapiens

<220>
<223> Protein: 26S PROTEASOME SUBUNIT S5B

<400>  18

Met Ala Ala Gln Ala Leu Ala Leu Leu Arg Glu Val Ala Arg Leu Glu
1               5                   10                  15

Ala Pro Leu Glu Glu Leu Arg Ala Leu His Ser Val Leu Gln Ala Val
            20                  25                  30

Pro Leu Asn Glu Leu Arg Gln Gln Ala Ala Glu Leu Arg Leu Gly Pro
        35                  40                  45

Leu Phe Ser Leu Leu Asn Glu Asn His Arg Glu Lys Thr Thr Leu Cys
    50                  55                  60

Val Ser Ile Leu Glu Arg Leu Leu Gln Ala Met Glu Pro Val His Val
65                  70                  75                  80

Ala Arg Asn Leu Arg Val Asp Leu Gln Arg Gly Leu Ile His Pro Asp
                85                  90                  95

Asp Ser Val Lys Ile Leu Thr Leu Ser Gln Ile Gly Arg Ile Val Glu
            100                 105                 110

Asn Ser Asp Ala Val Thr Glu Ile Leu Asn Asn Ala Glu Leu Leu Lys
        115                 120                 125

Gln Ile Val Tyr Cys Ile Gly Gly Glu Asn Leu Ser Val Ala Lys Ala
    130                 135                 140

Ala Ile Lys Ser Leu Ser Arg Ile Ser Leu Thr Gln Ala Gly Leu Glu
145                 150                 155                 160

Ala Leu Phe Glu Ser Asn Leu Leu Asp Asp Leu Lys Ser Val Met Lys
                165                 170                 175

Thr Asn Asp Ile Val Arg Tyr Arg Val Tyr Glu Leu Ile Ile Glu Ile
            180                 185                 190

Ser Ser Val Ser Pro Glu Ser Leu Asn Tyr Cys Thr Thr Ser Gly Leu
        195                 200                 205

Val Thr Gln Leu Leu Arg Glu Leu Thr Gly Glu Asp Val Leu Val Arg
    210                 215                 220

Ala Thr Cys Ile Glu Met Val Thr Ser Leu Ala Tyr Thr His His Gly
225                 230                 235                 240

Arg Gln Tyr Leu Ala Gln Glu Gly Val Ile Asp Gln Ile Ser Asn Ile
                245                 250                 255

Ile Val Gly Ala Asp Ser Asp Pro Phe Ser Ser Phe Tyr Leu Pro Gly
            260                 265                 270

Phe Val Lys Phe Phe Gly Asn Leu Ala Val Met Asp Ser Pro Gln Gln
        275                 280                 285

Ile Cys Glu Arg Tyr Pro Ile Phe Val Glu Lys Val Phe Glu Met Ile
    290                 295                 300

Glu Ser Gln Asp Pro Thr Met Ile Gly Val Ala Val Asp Thr Val Gly
305                 310                 315                 320

Ile Leu Gly Ser Asn Val Glu Gly Lys Gln Val Leu Gln Lys Thr Gly
                325                 330                 335

Thr Arg Phe Glu Arg Leu Leu Met Arg Ile Gly His Gln Ser Lys Asn
            340                 345                 350

Ala Pro Val Glu Leu Lys Ile Arg Cys Leu Asp Ala Ile Ser Ser Leu
        355                 360                 365

Leu Tyr Leu Pro Pro Glu Gln Gln Thr Asp Asp Leu Leu Arg Met Thr
    370                 375                 380

Glu Ser Trp Phe Ser Ser Leu Ser Arg Asp Pro Leu Glu Leu Phe Arg
385                 390                 395                 400

Gly Ile Ser Ser Gln Pro Phe Pro Glu Leu His Cys Ala Ala Leu Lys
                405                 410                 415

Val Phe Thr Ala Ile Ala Asn Gln Pro Trp Ala Gln Lys Leu Met Phe
            420                 425                 430

Asn Ser Pro Gly Phe Val Glu Tyr Val Val Asp Arg Ser Val Glu His
        435                 440                 445

Asp Lys Ala Ser Lys Asp Ala Lys Tyr Glu Leu Val Lys Ala Leu Ala
    450                 455                 460

Asn Ser Lys Thr Ile Ala Glu Ile Phe Gly Asn Pro Asn Tyr Leu Arg
465                 470                 475                 480

Leu Arg Thr Tyr Leu Ser Glu Gly Pro Tyr Tyr Val Lys Pro Val Ser
                485                 490                 495

Thr Thr Ala Val Glu Gly Ala Glu
            500

<210>  19

<211>  1128

<212>  PRT

<213>  Homo sapiens

<220>
<223> Protein: Nck-associated protein 1 (Nap 1)

<400>  19

Met Ser Arg Ser Val Leu Gln Pro Ser Gln Gln Lys Leu Ala Glu Lys
1               5                   10                  15

Leu Thr Ile Leu Asn Asp Arg Gly Val Gly Met Leu Thr Arg Leu Tyr
            20                  25                  30

Asn Ile Lys Lys Ala Cys Gly Asp Pro Lys Ala Lys Pro Ser Tyr Leu
        35                  40                  45

Ile Asp Lys Asn Leu Glu Ser Ala Val Lys Phe Ile Val Arg Lys Phe
    50                  55                  60

Pro Ala Val Glu Thr Arg Asn Asn Asn Gln Gln Leu Ala Gln Leu Gln
65                  70                  75                  80

Lys Glu Lys Ser Glu Ile Leu Lys Asn Leu Ala Leu Tyr Tyr Phe Thr
                85                  90                  95

Phe Val Asp Val Met Glu Phe Lys Asp His Val Cys Glu Leu Leu Asn
            100                 105                 110

Thr Ile Asp Val Cys Gln Val Phe Phe Asp Ile Thr Val Asn Phe Asp
        115                 120                 125

Leu Thr Lys Asn Tyr Leu Asp Leu Ile Ile Thr Tyr Thr Thr Leu Met
    130                 135                 140

Ile Leu Leu Ser Arg Ile Glu Glu Arg Lys Ala Ile Ile Gly Leu Tyr
145                 150                 155                 160

Asn Tyr Ala His Glu Met Thr His Gly Ala Ser Asp Arg Glu Tyr Pro
                165                 170                 175

Arg Leu Gly Gln Met Ile Val Asp Tyr Glu Asn Pro Leu Lys Lys Met
            180                 185                 190

Met Glu Glu Phe Val Pro His Ser Lys Ser Leu Ser Asp Ala Leu Ile
        195                 200                 205

Ser Leu Gln Met Val Tyr Pro Arg Arg Asn Leu Ser Ala Asp Gln Trp
    210                 215                 220

Arg Asn Ala Gln Leu Leu Ser Leu Ile Ser Ala Pro Ser Thr Met Leu
225                 230                 235                 240

Asn Pro Ala Gln Ser Asp Thr Met Pro Cys Glu Tyr Leu Ser Leu Asp
                245                 250                 255

Ala Met Glu Lys Trp Ile Ile Phe Gly Phe Ile Leu Cys His Gly Ile
            260                 265                 270

Leu Asn Thr Asp Ala Thr Ala Leu Asn Leu Trp Lys Leu Ala Leu Gln
        275                 280                 285

Ser Ser Ser Cys Leu Ser Leu Phe Arg Asp Glu Val Phe His Ile His
    290                 295                 300

Lys Ala Ala Glu Asp Leu Phe Val Asn Ile Arg Gly Tyr Asn Lys Arg
305                 310                 315                 320

Ile Asn Asp Ile Arg Glu Cys Lys Glu Ala Ala Val Ser His Ala Gly
                325                 330                 335

Ser Met His Arg Glu Arg Arg Lys Phe Leu Arg Ser Ala Leu Lys Glu
            340                 345                 350

Leu Ala Thr Val Leu Ser Asp Gln Pro Gly Leu Leu Gly Pro Lys Ala
        355                 360                 365

Leu Phe Val Phe Met Ala Leu Ser Phe Ala Arg Asp Glu Ile Ile Trp
    370                 375                 380

Leu Leu Arg His Ala Asp Asn Met Pro Lys Lys Ser Ala Asp Asp Phe
385                 390                 395                 400

Ile Asp Lys His Ile Ala Glu Leu Ile Phe Tyr Met Glu Glu Leu Arg
                405                 410                 415

Ala His Val Arg Lys Tyr Gly Pro Val Met Gln Arg Tyr Tyr Val Gln
            420                 425                 430

Tyr Leu Ser Gly Phe Asp Ala Val Val Leu Asn Glu Leu Val Gln Asn
        435                 440                 445

Leu Ser Val Cys Pro Glu Asp Glu Ser Ile Ile Met Ser Ser Phe Val
    450                 455                 460

Asn Thr Met Thr Ser Leu Ser Val Lys Gln Val Glu Asp Gly Glu Val
465                 470                 475                 480

Phe Asp Phe Arg Gly Met Arg Leu Asp Trp Phe Arg Leu Gln Ala Tyr
                485                 490                 495

Thr Ser Val Ser Lys Ala Ser Leu Gly Leu Ala Asp His Arg Glu Leu
            500                 505                 510

Gly Lys Met Met Asn Thr Ile Ile Phe His Thr Lys Met Val Asp Ser
        515                 520                 525

Leu Val Glu Met Leu Val Glu Thr Ser Asp Leu Ser Ile Phe Cys Phe
    530                 535                 540

Tyr Ser Arg Ala Phe Glu Lys Met Phe Gln Gln Cys Leu Glu Leu Pro
545                 550                 555                 560

Ser Gln Ser Arg Tyr Ser Ile Ala Phe Pro Leu Leu Cys Thr His Phe
                565                 570                 575

Met Ser Cys Thr His Glu Leu Cys Pro Glu Glu Arg His His Ile Gly
            580                 585                 590

Asp Arg Ser Leu Ser Leu Cys Asn Met Phe Leu Asp Glu Met Ala Lys
        595                 600                 605

Gln Ala Arg Asn Leu Ile Thr Asp Ile Cys Thr Glu Gln Cys Thr Leu
    610                 615                 620

Ser Asp Gln Leu Leu Pro Lys His Cys Ala Lys Thr Ile Ser Gln Ala
625                 630                 635                 640

Val Asn Lys Lys Ser Lys Lys Gln Thr Gly Lys Lys Gly Glu Pro Glu
                645                 650                 655

Arg Glu Lys Pro Gly Val Glu Ser Met Arg Lys Asn Arg Leu Val Val
            660                 665                 670

Thr Asn Leu Asp Lys Leu His Thr Ala Leu Ser Glu Leu Cys Phe Ser
        675                 680                 685

Ile Asn Tyr Val Pro Asn Met Val Val Trp Glu His Thr Phe Thr Pro
    690                 695                 700

Arg Glu Tyr Leu Thr Ser His Leu Glu Ile Arg Phe Thr Lys Ser Ile
705                 710                 715                 720

Val Gly Met Thr Met Tyr Asn Gln Ala Thr Gln Glu Ile Ala Lys Pro
                725                 730                 735

Ser Glu Leu Leu Thr Ser Val Arg Ala Tyr Met Thr Val Leu Gln Ser
            740                 745                 750

Ile Glu Asn Tyr Val Gln Ile Asp Ile Thr Arg Val Phe Asn Asn Val
        755                 760                 765

Leu Leu Gln Gln Thr Gln His Leu Asp Ser His Gly Glu Pro Thr Ile
    770                 775                 780

Thr Ser Leu Tyr Thr Asn Trp Tyr Leu Glu Thr Leu Leu Arg Gln Val
785                 790                 795                 800

Ser Asn Gly His Ile Ala Tyr Phe Pro Ala Met Lys Ala Phe Val Asn
                805                 810                 815

Leu Pro Thr Glu Asn Glu Leu Thr Phe Asn Ala Glu Glu Tyr Ser Asp
            820                 825                 830

Ile Ser Glu Met Arg Ser Leu Ser Glu Leu Leu Gly Pro Tyr Gly Met
        835                 840                 845

Lys Phe Leu Ser Glu Ser Leu Met Trp His Ile Ser Ser Gln Val Ala
    850                 855                 860

Glu Leu Lys Lys Leu Val Val Glu Asn Val Asp Val Leu Thr Gln Met
865                 870                 875                 880

Arg Thr Ser Phe Asp Lys Pro Asp Gln Met Ala Ala Leu Phe Lys Arg
                885                 890                 895

Leu Ser Ser Val Asp Ser Val Leu Lys Arg Met Thr Ile Ile Gly Val
            900                 905                 910

Ile Leu Ser Phe Arg Ser Leu Ala Gln Glu Ala Leu Arg Asp Val Leu
        915                 920                 925

Ser Tyr His Ile Pro Phe Leu Val Ser Ser Ile Glu Asp Phe Lys Asp
    930                 935                 940

His Ile Pro Arg Glu Thr Asp Met Lys Val Ala Met Asn Val Tyr Glu
945                 950                 955                 960

Leu Ser Ser Ala Ala Gly Leu Pro Cys Glu Ile Asp Pro Ala Leu Val
                965                 970                 975

Val Ala Leu Ser Ser Gln Lys Ser Glu Asn Ile Ser Pro Glu Glu Glu
            980                 985                 990

Tyr Lys Ile Ala Cys Leu Leu Met  Val Phe Val Ala Val  Ser Leu Pro
        995                 1000                 1005

Thr Leu  Ala Ser Asn Val Met  Ser Gln Tyr Ser Pro  Ala Ile Glu
    1010                 1015                 1020

Gly His  Cys Asn Asn Ile His  Cys Leu Ala Lys Ala  Ile Asn Gln
    1025                 1030                 1035

Ile Ala  Ala Ala Leu Phe Thr  Ile His Lys Gly Ser  Ile Glu Asp
    1040                 1045                 1050

Arg Leu  Lys Glu Phe Leu Ala  Leu Ala Ser Ser Ser  Leu Leu Lys
    1055                 1060                 1065

Ile Gly Gln Glu Thr Asp Lys Thr Thr Thr Arg Asn Arg Glu Ser
    1070                1075                1080

Val Tyr Leu Leu Leu Asp Met Ile Val Gln Glu Ser Pro Phe Leu
    1085                1090                1095

Thr Met Asp Leu Leu Glu Ser Cys Phe Pro Tyr Val Leu Leu Arg
    1100                1105                1110

Asn Ala Tyr His Ala Val Tyr Lys Gln Ser Val Thr Ser Ser Ala
    1115                1120                1125

<210> 20

<211> 4218

<212> DNA

<213> Homo sapiens

<220>
<223> cDNA: Nck-associated protein 1 (Nap 1)

<400> 20
cggcggcacc agcaccacca tgtcgcgctc agtgctgcag cccagtcagc agaagctggc 60

ggagaagctc accatcctca acgaccgggg cgtcggcatg ctcacccgcc tctacaacat 120

caagaaggca tgtggagacc ccaaggcaaa accatcctat cttatcgaca aaaacctgga 180

atctgctgtg aaattcatag tcagaaaatt ccctgctgta gaaacccgca acaacaatca 240

acagcttgca caactacaga aagaaaaatc agagattctg aaaaatctgg cattatatta 300

cttcacattt gtagatgtta tggaatttaa ggaccatgtt tgtgaattgc tgaatactat 360

tgacgtttgc caagtcttct ttgatattac tgtaaacttt gatttaacaa agaactactt 420

agatttaatt ataacctata caacactaat gatactgctg tctcgaattg aagaaaggaa 480

ggcaatcatt ggattataca actatgccca tgaaatgact catggagcaa gtgacagaga 540

atacccacgc cttggccaga tgattgtgga ttatgaaaac cctttaaaga agatgatgga 600

agaatttgta ccccatagca agtctctttc agatgcacta atttctcttc aaatggtata 660

tcctcgaagg aatctttcag ctgaccagtg gagaaatgcc cagttattga gcctcatcag 720

tgcacctagt acaatgctta atccagcaca gtccgacact atgccttgtg aatacctctc 780

tttggatgca atggaaaagt ggattatctt tggctttatt ttgtgccatg ggatcctaaa 840

tactgacgct acagcactga acctttggaa actagctctt caaagtagct cttgcctctc 900

tctctttcgg gatgaagttt tccacattca caaagctgca gaagacttat ttgtaaacat 960

acgaggctat aataaacgta ttaatgacat aagagaatgc aaggaggcag ccgtgtcaca 1020

tgctggttca atgcacagag aaagacgcaa gtttttaaga tctgcactga aggaattggc 1080

tactgtcctc tctgatcaac ctggattgct aggtcccaag gcactttttg tttttatggc 1140

attatccttt gcccgtgatg aaatcatctg gctacttcgt catgcagata acatgccaaa 1200

gaagagtgca gacgacttta tagataagca cattgctgaa ttaatatttt acatggaaga 1260
acttagagca catgtgagga aatacggacc tgtaatgcag aggtattacg tgcagtacct 1320
ttctggcttt gatgctgttg tcctcaatga actcgtgcag aatctttctg tttgccctga 1380
agatgaatca atcatcatgt cctcttttgt taacactatg acttccctaa gtgtaaaaca 1440
agttgaagat ggggaagtat ttgatttcag aggaatgaga ttagattggt ttaggttaca 1500
ggcatatact agtgtctcaa aggcttcact tggccttgca gatcacagag aacttgggaa 1560
gatgatgaat acaataattt ttcatacaaa aatggtagat tccttggtgg aaatgttggt 1620
ggaaacatca gatctctcca tattttgttt ttatagtcgt gcttttgaga agatgtttca 1680
acagtgtttg gagttaccct ctcaatcaag atactcaatt gcatttccac tactttgcac 1740
tcattttatg agttgcacgc atgaactatg tccagaagag cgacatcata ttggagatcg 1800
cagtctttcc ttatgtaata tgttcctaga tgaaatggcc aaacaagctc gaaatctcat 1860
cactgatatt tgcacagaac agtgtaccct tagtgaccag ttgctaccca agcattgtgc 1920
caaaactatc agtcaagcag tgaataagaa atcaaaaaag cagactggta agaaagggga 1980
acctgaaagg gagaaaccag gtgttgagag catgaggaaa aacaggctgg ttgtgaccaa 2040
ccttgataaa ttgcacactg cactttctga gttatgcttc tctataaatt atgtaccaaa 2100
catggtggta tgggaacata cctttacccc acgagaatat ttgacttctc atctggaaat 2160
acgctttacc aagtcaattg ttgggatgac tatgtataat caagccacac aggaaattgc 2220
aaaaccttca gaacttctaa caagtgtaag agcatacatg accgtactcc agtcaataga 2280
aaactatgtg cagattgata ttacaagagt atttaataat gtgcttcttc aacaaacaca 2340
acatttagac agtcatggag agccaaccat tacaagtcta tacacaaatt ggtatttgga 2400
aactttgtta cgacaagtca gcaatggcca tatagcatat tttcctgcaa tgaaagcgtt 2460
tgtgaactta cctacagaaa atgaattaac attcaatgca gaggaatatt ctgacatatc 2520
agaaatgagg tcattatcag aactactagg cccatatggt atgaagtttc taagtgaaag 2580
ccttatgtgg catatttcat cacaagttgc tgaacttaag aaacttgtgg tggagaatgt 2640
tgatgtgtta acacaaatga ggaccagctt tgacaaacca gaccagatgg ctgcactgtt 2700
taaaagatta tcatctgttg acagtgtctt gaagaggatg acaataattg gtgtaatttt 2760
atccttccga tcattggcac aagaagcact tagagatgtg ttatcctacc acattccttt 2820
tcttgtaagt tcaattgaag attttaagga tcacattcca agggaaactg atatgaaggt 2880
tgcaatgaat gtgtatgagt tatcatcagc tgccggatta ccttgtgaga ttgatcctgc 2940
attggtcgta gctctttctt cacaaaaatc ggaaaacatt agtccagaag aagagtataa 3000
aattgcctgc cttctcatgg tgtttgtggc agtttctttg ccaacactgg ccagtaatgt 3060
gatgtctcag tacagccctg ctatagaagg gcattgcaac aacatacatt gcttggccaa 3120
agccatcaac cagattgctg cagctttgtt tacaattcac aaaggaagca ttgaagaccg 3180

tcttaaagaa tttctggcgc ttgcatcctc cagtctactg aaaattggcc aggagacaga 3240

taaaactaca acaagaaata gagaatctgt ttatttactg ctagatatga ttgtacaaga 3300

atctccattc cttacaatgg atcttttgga atcttgtttt ccttatgtct tgctgagaaa 3360

tgcataccat gctgtctaca aacaaagtgt tacatcttct gcataaaatt acctacttaa 3420

tcaagataag cacgcatttt tgttgccttg gttttacctg tagactgtgg aactatttta 3480

ccttaagacc tgaaaaagtt ttgtggatta taaatttctt tcatacggtt gtattttctg 3540

atcattggtt tcttaatatg gttgtactac agtatacttg gttgatttag gttgcacatt 3600

cactgaattc actgagatta ttcctataat tttaaagtat catttatttg aaaaacatac 3660

attatcaaca tgtttttgat atttgataat gaaaaaaatc tttgcttgtt tatttctgaa 3720

aaagaactgt atttagtgat tattttagat agtgatatta tagcattcat ctgtgtgtaa 3780

attatttcat atagggaaga gttctgatct gtacctatgg ttcttattga aaacaacatt 3840

ggatgtgcat ttctgtgatg ttatgaatac atttctactt tattttgaaa catttgccaa 3900

actaaatact gtaacactgt ataacattta aaaatgttaa agaactgctt agtattagaa 3960

gcagatcatt tcccaaaatt ctaagagcag cagcatatgt tgttgcttgt ataaagccta 4020

gcgataattt ttagactaac ttccatggtg ccctgttggc attagcacta ccattgtacc 4080

ctgctgtata ataaacaatc ttagacattt atcaactgtt gatacaaatg ttagtcccta 4140

accacttttt atatatgttt taaatttttg aaattcaagt gtaccttcca taacataaaa 4200

taaacactag actgtatc 4218

<210> 21

<211> 166

<212> PRT

<213> Mus musculus

<220>
<223> Protein: Cofilin

<400> 21

Met Ala Ser Gly Val Thr Val Asn Asp Glu Val Ile Lys Val Phe Asn
1               5                   10                  15

Asp Met Lys Val Arg Lys Ser Ser Thr Gln Glu Glu Ile Lys Lys Arg
            20                  25                  30

Lys Lys Ala Val Leu Phe Cys Leu Ser Asp Asp Lys Arg Gln Ile Ile
        35                  40                  45

Val Glu Glu Ala Lys Gln Ile Leu Val Gly Asp Ile Gly Asp Thr Val
    50                  55                  60

Glu Asp Pro Tyr Thr Ser Phe Val Lys Leu Leu Pro Leu Asn Asp Cys
65                  70                  75                  80

Arg Tyr Ala Leu Tyr Asp Ala Thr Tyr Glu Thr Lys Glu Ser Lys Lys
                85                  90                  95

Glu Asp Leu Val Phe Ile Phe Trp Ala Pro Glu Ser Ala Pro Leu Lys
            100                 105                 110

Ser Lys Met Ile Tyr Ala Ser Ser Lys Asp Ala Ile Lys Lys Lys Phe
        115                 120                 125

Thr Gly Ile Lys His Glu Trp Gln Val Asn Gly Leu Asp Asp Ile Lys
    130                 135                 140

Asp Arg Ser Thr Leu Gly Glu Lys Leu Gly Gly Ser Val Val Val Ser
145                 150                 155                 160

Leu Glu Gly Lys Pro Leu
                165

<210>  22

<211>  2974

<212>  DNA

<213>  Mus musculus

<220>
<223> cDNA: Cofilin

<400>  22
cgcccggctg cagctcccgg cgtgccctgc actctctgct gcccgccgcc gacccctcct      60

tcttctcgtc ccagtgccac cgagccggag tccgagccac cgccgccgca gccacttcag     120

ccgcgggcac tatggcatct ggagttacag tgaatgatga agtcatcaaa gtttttaatg     180

atatgaaagt aagaaaatct tctacacagg aggagatcaa aaaaagaaag aaagcagttc     240

tcttctgttt gagcgatgac aaaagacaaa taattgtaga ggaagccaag cagatcttgg     300

tgggtgacat tggtgacact gtagaggacc cctacacatc ttttgtgaag ttgttgcctc     360

tgaatgattg ccgatatgct ttgtacgatg ccacgtacga aacaaaagag tctaagaaag     420

aagacctagt atttatattc tgggctcctg aaagtgcacc gttaaaaagc aagatgattt     480

atgctagctc taaagatgcc attaaaaaga aatttacagg tattaaacat gagtggcaag     540

taaatggctt ggacgatatt aaggaccgct cgacgctggg agagaaactg gggggcagtg     600

ttgtagtttc ccttgaagga aagccactat aaaataatag ccaagtgcca tttgatctta     660

aggggcttac acgtatctct ccagctcagt ccactggaat tgtattaggt tttgtttttt     720

ttgtttattc ccttttcact ggtcccgttc gtgaatgagt gaatataaga agcctgtcag     780

tattgccatg agactgtttc atatggttac ttttctgtat tcccaaggaa tgccttcctg     840

tcttatttta gccaaaacaa actggttcca tgccttcctt gcagtgagcg ttacaatgga     900

tgtggttgtc aatgtgaata gcttagagta ctacaaaggg taagctaact gaatgccttg     960

aaaatattat ccactggtcg gtcatatggg agacttgttt cagtattatt tatagttgca    1020

cttgattacc gttctctgag gcactggagc cttcatacac ctcacctgcc ttggcaagcc 1080
tatttttgtg acctggcagc acagatttaa cactattcgt taaaagcact tttttttaat 1140
gcgtttaatc ccttataaag aatgccaatt aagttttatt acctgtcatc aatttatcct 1200
agtatctcag tgttcattct tcttgccttc atattttttt caaagaaaca gctgtgctaa 1260
tgtctttggt ttcccgatga gtgtacacta ctgtataatt tatgtttacc atatgagtct 1320
tgaaacacta cagatatttt gaatatcagt catgatggca atttctgtat aaaagagcct 1380
taaatggaac attgttttga gatcaaactc cctaccctca caaaagtggc cacgttgcaa 1440
taaaaattgt ggcagattac agaatgttgc cttgttttcc ttggaaattt tgcaaattgt 1500
tatgtgaaat tttagggtaa cggtgattaa gctctgcact ggtatttgga attttttttc 1560
ctttaatctt tggtttaaaa acatcttaaa atcacttata tacaatcatt aaaagagtgg 1620
taattttata aatgcttatt tatgttataa aatggagatc agaaaaaaat tctttttgca 1680
ctttggccta tccagtatct tatttattct ctagataagc taggatatta atccagagtt 1740
acattactga gaattgagta gtataagtag gatgttttta ttacttggtc ataatgaaaa 1800
taatttgtaa aatgtcattc gaaggttaat gatgattgtg atgtttagga atgtttgtct 1860
cagccacagt tccttcatag cttttccaaa atgaattggg aaaaaaaatc gtatagcagt 1920
cttaaagctt agtaatggaa cttggctgtg gcccagagct ttctccttat agagaatttg 1980
atctgctccg tgtgtgctct ctgctattag ccggagctat ttatggcaaa cacatgcttt 2040
tgtatcttgt catagtcatc cacagatggc aaaactggac ttgattctac tggcatgtaa 2100
gacaggcgtg ctagtgagca gtcgtgtgtg gctctggact ctgaccccag agctctgaag 2160
aatgctctta tcagaagata ggaaatgaaa atatcctttt ttaaaatatg tggaagtaat 2220
ttgggtataa ttagtttttt tctacctttt ggaaagttgt ttttttgttg tttttttttt 2280
tttcccagat gagaacatta acatagtggt taaatgtcta ggcttccatt taaaactaca 2340
caaatgactt gggatctttt tagcactaag gaatttgatt tcagccttcc agctgttgct 2400
gtgagttgtt ccagaccttt ctgtggcttt ttggtaaggc tgcttagaag catgagaagc 2460
atgagaatgg taatgtgtgc taaacctatg tttaaccaat ctttgcacca aaggactttt 2520
tcaccaattt attttgttat tcttctaaat attaagtgat ttctaaaagg taaagggtga 2580
ccttttgttt ttatatctaa tttctcaatt tctttatatg catttttaga ataatttgag 2640
agattaaatg ctgcttgaaa ctattatact ttgagtttta gattggccaa atacattaat 2700
gtagttaaat tcatctttaa agtacacata tgtgcctaga gccaaaaaat aataatgatt 2760
taatttatga ccttatgttg agactaattt cacatcttat tttacagtca tttacagtga 2820
aacaatgttc cagctagctt taaaagctat acggtgctaa ttagtaaaat attgagggca 2880
atattttact gctagcttgc aaaattataa gtgttttaaa aataaaatac atgaaaaaaa 2940
aaaaaaaaaa aaaaaaaaaa aaaaaaaaaa aaaa 2974

<210>  23

<211>  117

<212>  PRT

<213>  Homo sapiens

<220>
<223> Protein: Ganglioside expression factor 2

<400>  23

Met Lys Trp Met Phe Lys Glu Asp His Ser Leu Glu His Arg Cys Val
1               5                   10                  15

Glu Ser Ala Lys Ile Arg Ala Lys Tyr Pro Asp Arg Val Pro Val Ile
            20                  25                  30

Val Glu Lys Val Ser Gly Ser Gln Ile Val Asp Ile Asp Lys Arg Lys
        35                  40                  45

Tyr Leu Val Pro Ser Asp Ile Thr Val Ala Gln Phe Met Trp Ile Ile
    50                  55                  60

Arg Lys Arg Ile Gln Leu Pro Ser Glu Lys Ala Ile Phe Leu Phe Val
65                  70                  75                  80

Asp Lys Thr Val Pro Gln Ser Ser Leu Thr Met Gly Gln Leu Tyr Glu
                85                  90                  95

Lys Glu Lys Asp Glu Asp Gly Phe Leu Tyr Val Ala Tyr Ser Gly Glu
            100                 105                 110

Asn Thr Phe Gly Phe
        115

<210>  24

<211>  975

<212>  DNA

<213>  Rattus norvegicus

<220>
<223> cDNA: Ganglioside expression factor 2

<400>  24
cccgcctgcc gagtagtcgt cgctgccgcc gccgcctccg ttgttgttgt ggtcgcttcg      60

ccgaagtctg cggctcaaag agccggctcc gtcgcttccc gccgccatga agtggatgtt 120

taaggaggac cactcgctgg aacacagatg cgtggaatcc gcgaagatca gagcgaaata 180

ccccgaccgg gttccggtga tcgttgagaa agtctctggc tctcagattg ttgacattga 240

caagaggaag tacttggtcc catctgacat cactgtggct cagttcatgt ggatcatcag 300

gaaaaggatc cagcttcctt ctgagaaggc catcttcttg tttgtggaca agacagtccc 360

acagtccagc ctaactatgg gacagcttta cgagaaggaa aaagatgaag atggattctt 420

gtatgtggcc tacagcggag agaacacttt tggcttctga gcccttgctg ggctaggtgc 480

acccttcctg cttgtgtatc ctgtaaataa ctggctgttc tcagttactc cgccggagcc 540

tccacacaga cctactagtg catttgtaac tggatttatt tcttaatata ttggaaggtt 600

ttgttttcct tagattagta aattatcata cagagtttta ttttcagttt tcttttgtgc 660

actgtcctca tggctatatg ctccaaggaa cctgtcctcc ggaatcacat ttaatgaaga 720

tacttccgaa atgaagggcg gtaggtgtgg tattaaagtg acaaggaggg atgacgcatt 780

gttctggatt atgttcggag tgttagacgg ctaagtatta aaagccccca aattaaatcc 840

ttagcaatca gaacacttgc ttcactagat tttgccaact gcaaatcatg ttggactgag 900

ctaatctgtt ctttctgaga ctataaggta aatgattaac aataaagcct ccatgtaaaa 960

ggcaaaaaaa aaaaa 975

<210> 25

<211> 1377

<212> PRT

<213> Homo sapiens

<220>
<223> Protein HMG1/2

<400> 25

Ile Pro Met Val Val Ser Asp Phe Asp Leu Pro Asp Gln Gln Ile Glu
1               5                   10                  15

Ile Leu Gln Ser Ser Asp Ser Gly Cys Ser Gln Ser Ser Ala Gly Asp
            20                  25                  30

Asn Leu Ser Tyr Glu Val Asp Pro Glu Thr Val Asn Ala Gln Glu Asp
        35                  40                  45

Ser Gln Met Pro Lys Glu Ser Ser Pro Asp Asp Asp Val Gln Gln Val
    50                  55                  60

Val Phe Asp Leu Ile Cys Lys Val Val Ser Gly Leu Glu Val Glu Ser
65                  70                  75                  80

Ala Ser Val Thr Ser Gln Leu Glu Ile Glu Ala Met Pro Pro Lys Cys
                85                  90                  95

Ser Asp Ile Asp Pro Asp Glu Glu Thr Ile Lys Ile Glu Asp Asp Ser
                100                 105                 110

Ile Gln Gln Ser Gln Asn Ala Leu Leu Ser Asn Glu Ser Ser Gln Phe
        115                 120                 125

Leu Ser Val Ser Ala Glu Gly Gly His Glu Cys Val Ala Asn Gly Ile
    130                 135                 140

Ser Arg Asn Ser Ser Ser Pro Cys Ile Ser Gly Thr Thr His Thr Leu
145                 150                 155                 160

His Asp Ser Ser Val Ala Ser Ile Glu Thr Lys Ser Arg Gln Arg Ser
                165                 170                 175

His Ser Ser Ile Gln Phe Ser Phe Lys Glu Lys Leu Ser Glu Lys Val
            180                 185                 190

Ser Glu Lys Glu Thr Ile Val Lys Glu Ser Gly Lys Gln Pro Gly Ala
        195                 200                 205

Lys Pro Lys Val Lys Leu Ala Arg Lys Lys Asp Asp Asp Lys Lys Lys
    210                 215                 220

Ser Ser Asn Glu Lys Leu Lys Gln Thr Ser Val Phe Phe Ser Asp Gly
225                 230                 235                 240

Leu Asp Leu Glu Asn Trp Tyr Ser Cys Gly Glu Gly Asp Ile Ser Glu
                245                 250                 255

Ile Glu Ser Asp Met Gly Ser Pro Gly Ser Arg Lys Ser Pro Asn Phe
            260                 265                 270

Asn Ile His Pro Leu Tyr Gln His Val Leu Leu Tyr Leu Gln Leu Tyr
        275                 280                 285

Asp Ser Ser Arg Thr Leu Tyr Ala Phe Ser Ala Ile Lys Ala Ile Leu
    290                 295                 300

Lys Thr Asn Pro Ile Ala Phe Val Asn Ala Ile Ser Thr Thr Ser Val
305                 310                 315                 320

Asn Asn Ala Tyr Thr Pro Gln Leu Ser Leu Leu Gln Asn Leu Leu Ala
                325                 330                 335

Arg His Arg Ile Ser Val Met Gly Lys Asp Phe Tyr Ser His Ile Pro
            340                 345                 350

Val Asp Ser Asn His Asn Phe Arg Ser Ser Met Tyr Ile Glu Ile Leu
        355                 360                 365

Ile Ser Leu Cys Leu Tyr Tyr Met Arg Ser His Tyr Pro Thr His Val
    370                 375                 380

Lys Val Thr Ala Gln Asp Leu Ile Gly Asn Arg Asn Met Gln Met Met
385                 390                 395                 400

Ser Ile Glu Ile Leu Thr Leu Leu Phe Thr Glu Leu Ala Lys Val Ile
                405                 410                 415

Glu Ser Ser Ala Lys Gly Phe Pro Ser Phe Ile Ser Asp Met Leu Ser
            420                 425                 430

Lys Cys Lys Val Gln Lys Val Ile Leu His Cys Leu Leu Ser Ser Ile
        435                 440                 445

Phe Ser Ala Gln Lys Trp His Ser Glu Lys Met Ala Gly Lys Asn Leu
    450                 455                 460

Val Ala Val Glu Glu Gly Phe Ser Glu Asp Ser Leu Ile Asn Phe Ser
465                 470                 475                 480

Glu Asp Glu Phe Asp Asn Gly Ser Thr Leu Gln Ser Gln Leu Leu Lys
                485                 490                 495

Val Leu Gln Arg Leu Ile Val Leu Glu His Arg Val Met Thr Ile Pro
            500                 505                 510

Glu Glu Asn Glu Thr Gly Phe Asp Phe Val Val Ser Asp Leu Glu His
        515                 520                 525

Ile Ser Pro His Gln Pro Met Thr Ser Leu Gln Tyr Leu His Ala Gln
    530                 535                 540

Pro Ile Thr Cys Gln Gly Met Phe Leu Cys Ala Val Ile Arg Ala Leu
545                 550                 555                 560

His Gln His Cys Ala Cys Lys Met His Pro Gln Trp Ile Gly Leu Ile
                565                 570                 575

Thr Ser Thr Leu Pro Tyr Met Gly Lys Val Leu Gln Arg Val Val Val
            580                 585                 590

Ser Val Thr Leu Gln Leu Cys Arg Asn Leu Asp Asn Leu Ile Gln Gln
        595                 600                 605

Tyr Lys Tyr Glu Thr Gly Leu Ser Asp Ser Arg Pro Leu Trp Met Ala
    610                 615                 620

Ser Ile Ile Pro Pro Asp Met Ile Leu Thr Leu Leu Glu Gly Ile Thr
625                 630                 635                 640

Ala Ile Ile His Tyr Cys Leu Leu Asp Pro Thr Thr Gln Tyr His Gln
                645                 650                 655

Leu Leu Val Ser Val Asp Gln Lys His Leu Phe Glu Ala Arg Ser Gly
            660                 665                 670

Ile Leu Ser Ile Leu His Met Ile Met Ser Ser Val Thr Leu Leu Trp
        675                 680                 685

Ser Ile Leu His Gln Ala Asp Ser Ser Glu Lys Met Thr Ile Ala Ala
    690                 695                 700

Ser Ala Ser Leu Thr Thr Ile Asn Leu Gly Ala Thr Lys Asn Leu Arg
705                 710                 715                 720

Gln Gln Ile Leu Glu Leu Leu Gly Pro Ile Ser Met Asn His Gly Val
                725                 730                 735

His Phe Met Ala Ala Ile Ala Phe Val Trp Asn Glu Arg Arg Gln Asn
            740                 745                 750

Lys Thr Thr Thr Arg Thr Lys Val Ile Pro Ala Ala Ser Glu Glu Gln
        755                 760                 765

Leu Leu Leu Val Glu Leu Val Arg Ser Ile Ser Val Met Arg Ala Glu
    770                 775                 780

Thr Val Ile Gln Thr Val Lys Glu Val Leu Lys Gln Pro Pro Ala Ile
785                 790                 795                 800

Ala Lys Asp Lys Lys His Leu Ser Leu Glu Val Cys Met Leu Gln Phe
                805                 810                 815

Phe Tyr Ala Tyr Ile Gln Arg Ile Pro Val Pro Asn Leu Val Asp Ser
            820                 825                 830

Trp Ala Ser Leu Leu Ile Leu Leu Lys Asp Ser Ile Gln Leu Ser Leu
        835                 840                 845

Pro Ala Pro Gly Gln Phe Leu Ile Leu Gly Val Leu Asn Glu Phe Ile
    850                 855                 860

Met Lys Asn Pro Ser Leu Glu Asn Lys Lys Asp Gln Arg Asp Leu Gln
865                 870                 875                 880

Asp Val Thr His Lys Ile Val Asp Ala Ile Gly Ala Ile Ala Gly Ser
                885                 890                 895

Ser Leu Glu Gln Thr Thr Trp Leu Arg Arg Asn Leu Glu Val Lys Pro
            900                 905                 910

Ser Pro Lys Ile Met Val Asp Gly Thr Asn Leu Glu Ser Asp Val Glu
        915                 920                 925

Asp Met Leu Ser Pro Ala Met Glu Thr Ala Asn Ile Thr Pro Ser Val
    930                 935                 940

Tyr Ser Val His Ala Leu Thr Leu Leu Ser Glu Val Leu Ala His Leu
945                 950                 955                 960

Leu Asp Met Val Phe Tyr Ser Asp Glu Lys Glu Arg Val Ile Pro Leu
                965                 970                 975

Leu Val Asn Ile Met His Tyr Val Val Pro Tyr Leu Arg Asn His Ser
            980                 985                 990

Ala His Asn Ala Pro Ser Tyr Arg Ala Cys Val Gln Leu Leu Ser Ser
        995                 1000                1005

Leu Ser Gly Tyr Gln Tyr Thr Arg Arg Ala Trp Lys Lys Glu Ala
    1010                1015                1020

Phe Asp Leu Phe Met Asp Pro Ser Phe Phe Gln Met Asp Ala Ser
    1025                1030                1035

Cys Val Asn His Trp Arg Ala Ile Met Asp Asn Leu Met Thr His
    1040                1045                1050

Asp Lys Thr Thr Phe Arg Asp Leu Met Thr Arg Val Ala Val Ala
    1055                1060                1065

Gln Ser Ser Ser Leu Asn Leu Phe Ala Asn Arg Asp Val Glu Leu
    1070                1075                1080

Glu Gln Arg Ala Met Leu Leu Lys Arg Leu Ala Phe Ala Ile Phe
    1085                1090                1095

Ser Ser Glu Ile Asp Gln Tyr Gln Lys Tyr Leu Pro Asp Ile Gln
    1100                1105                1110

Glu Arg Leu Val Glu Ser Leu Arg Leu Pro Gln Val Pro Thr Leu
    1115                1120                1125

His Ser Gln Val Phe Leu Phe Phe Arg Val Leu Leu Leu Arg Met
    1130                1135                1140

Ser Pro Gln His Leu Thr Ser Leu Trp Pro Thr Met Ile Thr Glu
    1145                1150                1155

Leu Val Gln Val Phe Leu Leu Met Glu Gln Glu Leu Thr Ala Asp
    1160                1165                1170

Glu Asp Ile Ser Arg Thr Ser Gly Pro Ser Val Ala Gly Leu Glu
    1175                1180                1185

Thr Thr Tyr Thr Gly Gly Asn Gly Phe Ser Thr Ser Tyr Asn Ser
    1190                1195                1200

Gln Arg Trp Leu Asn Leu Tyr Leu Ser Ala Cys Lys Phe Leu Asp
    1205                1210                1215

Leu Ala Leu Ala Leu Pro Ser Glu Asn Leu Pro Gln Phe Gln Met
    1220                1225                1230

Tyr Arg Trp Ala Phe Ile Pro Glu Ala Ser Asp Asp Ser Gly Leu
    1235                1240                1245

Glu Val Arg Arg Gln Gly Ile His Gln Arg Glu Phe Lys Pro Tyr
    1250                1255                1260

Val Val Arg Leu Ala Lys Leu Leu Arg Lys Arg Ala Lys Lys Asn
    1265                1270                1275

Pro Glu Glu Asp Asn Ser Gly Arg Thr Leu Gly Trp Glu Pro Gly
    1280                1285                1290

His Leu Leu Leu Thr Ile Cys Thr Val Arg Ser Met Glu Gln Leu
    1295                1300                1305

Leu Pro Phe Phe Asn Val Leu Ser Gln Val Phe Asn Ser Lys Val
    1310                1315                1320

Thr Ser Arg Cys Gly Gly His Ser Gly Ser Pro Ile Leu Tyr Ser
    1325                1330                1335

Asn Ala Phe Pro Asn Lys Asp Met Lys Leu Glu Asn His Lys Pro
    1340                1345                1350

Cys Ser Ser Lys Ala Arg Gln Lys Ile Glu Glu Met Val Glu Lys
    1355                1360                1365

Asp Phe Leu Glu Gly Met Ile Lys Thr
    1370                1375

<210>  26

<211>  1000

<212>  DNA

<213>  Homo sapiens

<220>
<223> cDNA HMG1/2

<400> 26
tttttttttt tttttttttg aaaaaaaaaa tgggtagtgt atattttgca ggtttaagac 60

aactcaggac aataaaaaca atggacttta catgtgtata tatatagctc tcttaggcac 120

cataatcagt atgagccaac aatatttaaa cttgattcag gccacattca gacatttgct 180

cttatataca aatatttaaa ttaaatacaa tctgaaatgt gttctgttac atacaaaaaa 240

ggaaaaacta tacaacgcag agcagtgtgt gtgttttaaa taattacatt tacatgtaag 300

ctaaatggaa ccagcaatgg tgctcaagtt tttatcatcc cttccagaaa atctttttct 360

accatctctt ctattttttg cctggctttg ctggaacatg gtttgtggtt ctccagtttc 420

atgtccttat tagggaaggc atttgagtag aggataggac tccctgagtg tcctccacat 480

cggcttgtga ctttgctgtt gaagacttga ctgagcacat tgaagaacgg caggagctgc 540

tccatactgc gcacggtgca gatggtgagc agcaagtgcc ctggctccca acccaatgtt 600

ctccctgagt tgtcttcctc tggatttttc tttgctcttt tccgaagaag ttttgctagt 660

cgtaccacgt aaggtttaaa ttctcgttga tgtataccct gccttctgac ttccaaacct 720

gaatcatctg aggcttctgg aataaaggcc catcggtaca tctgaaactg aggaaggttt 780

tcagagggca atgcgagagc caaatccaaa aatttgcaag cagagagata gaggtttaac 840

caccgctggc tgttatatga agtagagaag ccattacctc ctgtgtacgt tgtctccaga 900

ccagccacag agggccctga agtccgtgaa atatcttcat cagcagtgag ttcctgctcc 960

atcagtaaaa atacttgtac aagttctgta atcatggtag 1000

<210> 27

<211> 896

<212> PRT

<213> Mus musculus

<220>
<223> Protein: Mouse phosphoprotein (F1-20)

<400> 27
   Met Ser Gly Gln Thr Leu Thr Asp Arg Ile Ala Ala Ala Gln Tyr Ser
   1 5 10 15
   Val Thr Gly Ser Ala Val Ala Arg Ala Val Cys Lys Ala Thr Thr His
   20 25 30
   Glu Val Met Gly Pro Lys Lys Lys His Leu Asp Tyr Leu Ile Gln Ala
   35 40 45
   Thr Asn Glu Thr Asn Val Asn Ile Pro Gln Met Ala Asp Thr Leu Phe
   50 55 60
   Glu Arg Ala Thr Asn Ser Ser Trp Val Val Val Phe Lys Ala Leu Val
   65 70 75 80
   Thr Thr His His Leu Met Val His Gly Asn Glu Arg Phe Ile Gln Tyr
   85 90 95
   Leu Ala Ser Arg Asn Thr Leu Phe Asn Leu Ser Asn Phe Leu Asp Lys
   100 105 110
   Ser Gly Ser His Gly Tyr Asp Met Ser Thr Phe Ile Arg Arg Tyr Ser
   115 120 125
   Arg Tyr Leu Asn Glu Lys Ala Phe Ser Tyr Arg Gln Met Ala Phe Asp
   130 135 140
   Phe Ala Arg Val Lys Lys Gly Ala Asp Gly Val Met Arg Thr Met Val
   145 150 155 160
   Pro Glu Lys Leu Leu Lys Ser Met Pro Ile Leu Gln Gly Gln Ile Asp
   165 170 175
   Ala Leu Leu Glu Phe Asp Val His Pro Asn Glu Leu Thr Asn Gly Val
   180 185 190
   Ile Asn Ala Ala Phe Met Leu Leu Phe Lys Asp Leu Ile Lys Leu Phe
   195 200 205
   Ala Cys Tyr Asn Asp Gly Val Ile Asn Leu Leu Glu Lys Phe Phe Glu
   210 215 220
   Met Lys Lys Gly Gln Cys Lys Asp Ala Leu Glu Ile Tyr Lys Arg Phe
   225 230 235 240
   Leu Thr Arg Met Thr Arg Val Ser Glu Phe Leu Lys Val Ala Glu Gln
   245 250 255
   Val Gly Ile Asp Lys Gly Asp Ile Pro Asp Leu Thr Gln Ala Pro Ser
   260 265 270
   Ser Leu Met Glu Thr Leu Glu Gln His Leu Asn Thr Leu Glu Gly Lys
   275 280 285
   Lys Pro Gly Asn Asn Glu Gly Ser Gly Ala Pro Ser Pro Leu Ser Lys
   290 295 300
   Ser Ser Pro Ala Thr Thr Val Thr Ser Pro Asn Ser Thr Pro Ala Lys
   305 310 315 320
   Thr Ile Asp Thr Ser Pro Pro Val Asp Ile Phe Ala Thr Ala Ser Ala
   325 330 335
   Ala Ala Pro Val Ser Ser Ala Lys Pro Ser Ser Asp Leu Leu Asp Leu
   340 345 350
   Gln Pro Asp Phe Ser Gly Ala Ala Ala Gly Ala Ala Ala Pro Val Val
   355 360 365
   Pro Pro Ser Gly Gly Ala Thr Ala Trp Gly Asp Leu Leu Gly Glu Asp
       370                 375                 380
   Ser Leu Ala Ala Leu Ser Ser Val Pro Cys Glu Ala Pro Ile Ser Asp
   385                 390                 395                 400
   Pro Phe Ala Pro Glu Pro Ser Pro Pro Thr Thr Thr Thr Glu Pro Ala
                   405                 410                 415
   Ser Ala Ser Ala Ser Thr Thr Thr Ala Val Thr Ala Val Thr Thr Glu
               420                 425                 430
   Val Asp Leu Phe Gly Asp Ala Phe Ala Ala Ser Pro Gly Glu Ala Pro
           435                 440                 445
   Ala Ala Ser Glu Gly Ala Thr Ala Pro Ala Thr Pro Ala Pro Val Ala
       450                 455                 460
   Ala Ala Leu Asp Ala Cys Ser Gly Asn Asp Pro Phe Ala Pro Ser Glu
   465                 470                 475                 480
   Gly Ser Ala Glu Ala Ala Pro Glu Leu Asp Leu Phe Ala Met Lys Pro
                   485                 490                 495
   Pro Glu Thr Ser Ala Pro Val Val Thr Pro Thr Ala Ser Thr Ala Pro
               500                 505                 510
   Pro Val Pro Ala Thr Ala Pro Ser Pro Ala Pro Thr Ala Val Ala Ala
           515                 520                 525
   Thr Ala Ala Thr Thr Thr Ala Ala Ala Ala Ala Thr Thr Thr Ala Thr
       530                 535                 540
   Thr Ser Ala Ala Ala Ala Thr Thr Ala Ala Ala Pro Pro Ala Leu Asp
   545                 550                 555                 560
   Ile Phe Gly Asp Leu Phe Asp Ser Ala Pro Glu Val Ala Ala Ala Pro
                   565                 570                 575
   Lys Pro Asp Ala Ala Pro Ser Ile Asp Leu Phe Gly Thr Asp Ala Phe
               580                 585                 590
   Ser Ser Pro Pro Arg Gly Ala Ser Pro Val Pro Glu Ser Ser Leu Thr
           595                 600                 605
   Ala Asp Leu Leu Ser Val Asp Ala Phe Ala Ala Pro Ser Pro Ala Ser
       610                 615                 620
   Thr Ala Ser Pro Ala Lys Ala Glu Ser Ser Gly Val Ile Asp Leu Phe
   625                 630                 635                 640
   Gly Asp Ala Phe Gly Ser Gly Ala Ser Glu Thr Gln Pro Ala Pro Gln
                   645                 650                 655
   Ala Val Ser Ser Ser Ser Ala Ser Ala Asp Leu Leu Ala Gly Phe Gly
               660                 665                 670
   Gly Ser Phe Met Ala Pro Ser Thr Thr Pro Val Thr Pro Ala Gln Asn
           675                 680                 685
   Asn Leu Leu Gln Pro Ser Phe Glu Ala Ala Phe Gly Thr Thr Pro Ser
       690                 695                 700
   Thr Ser Ser Ser Ser Ser Phe Asp Pro Ser Gly Asp Leu Leu Met Pro
   705                 710                 715                 720
   Thr Met Ala Pro Ser Gly Gln Pro Ala Pro Val Ser Met Val Pro Pro
   725 730 735
   Ser Pro Ala Met Ala Ala Ser Lys Gly Leu Gly Ser Asp Leu Asp Ser
   740 745 750
   Ser Leu Ala Ser Leu Val Gly Asn Leu Gly Ile Ser Gly Thr Thr Ser
   755 760 765
   Lys Lys Gly Asp Leu Gln Trp Asn Ala Gly Glu Lys Lys Leu Thr Gly
   770 775 780
   Gly Ala Asn Trp Gln Pro Lys Val Thr Pro Ala Thr Trp Ser Ala Gly
   785 790 795 800
   Val Pro Pro Gln Gly Thr Val Pro Pro Thr Ser Ser Val Pro Pro Gly
   805 810 815
   Ala Gly Ala Pro Ser Val Gly Gln Pro Gly Ala Gly Phe Gly Met Pro
   820 825 830
   Pro Ser Gly Thr Gly Met Thr Met Met Ser Gln Gln Pro Val Met Phe
   835 840 845
   Ala Gln Pro Met Met Arg Pro Pro Phe Gly Ala Ala Ala Val Pro Gly
   850 855 860
   Thr Gln Leu Ser Pro Ser Pro Thr Pro Ala Thr Gln Ser Pro Lys Lys
   865 870 875 880
   Pro Pro Ala Lys Asp Pro Leu Ala Asp Leu Asn Ile Lys Asp Phe Leu
   885 890 895

<210>  28

<211>  4260

<212>  DNA

<213>  Mus musculus

<220>
<223> cDNA: Mouse phosphoprotein (F1-20)

<400>  28
gggacagggg ccgggccggg gctacaggca tcgcggcccg gggacaccag ggcggtgcgt      60

gtctgcaccc agctacctcg cgcggcgtcc gggctgcggt gctcctgggg cgggaagagg     120

aggcggtaga cgcggccggt gaagatgtcg ggccaaacgc tcacggatcg gatcgccgcc     180

gctcagtaca gcgtgactgg ctctgctgta gcaagagcag tctgcaaagc caccactcat     240

gaggtgatgg gccccaagaa gaagcacctg gactatttga tccaggctac caatgagacc     300

aatgtcaata tccctcagat ggccgacacc ctctttgagc gggcgacaaa cagtagctgg     360

gtggtggtat ttaaagcttt agtgaccaca caccatctca tggtgcatgg aaatgagaga     420

tttattcagt atttggcctc taggaatacg ctattcaatc tcagcaactt tctagataaa     480

agtggatccc acggttatga tatgtctacg ttcatacggc gttacagtag atacttgaat 540
gaaaaggctt tctcctacag acagatggca tttgactttg ccagagtgaa gaaaggggct 600
gacggtgtca tgaggacgat ggttcctgaa aagctcctga agagtatgcc aatcctgcag 660
gggcagatcg atgcactgct ggagtttgat gtgcatccaa atgaactaac caatggtgtc 720
ataaatgctg catttatgct tcttttcaaa gatcttatca aactgtttgc ttgctacaat 780
gacggcgtca ttaacttact tgaaaaattt tttgagatga agaaaggtca atgcaaagac 840
gcgctagaaa tttacaagcg atttctaact agaatgacga gggtgtccga attcctcaag 900
gtcgccgagc aagttggtat tgataaaggc gacattcccg acctcacgca ggctcccagc 960
agtcttatgg agacccttga acaacatcta aataccctag aaggaaagaa acctggaaac 1020
aatgaaggat ctggtgctcc ctctccacta agtaagtctt ctccagccac aactgttaca 1080
tctcctaatt ctacaccagc taaaactatc gacacatccc cgccagttga catatttgca 1140
acagcatccg cggctgcccc agtcagctct gctaagccat caagcgatct ccttgatctt 1200
cagcccgact tctctggagc ggctgcgggg gcagcagcac ctgtagtgcc tccttctggg 1260
ggtgcgaccg cctggggaga ccttttggga gaggattcct tggctgcact ttcctctgtt 1320
ccctgtgaag caccgatttc agacccattt gcaccagagc cttcccctcc tactacaacc 1380
actgagcctg cttcagcctc tgcctcgacc accacagctg tgacggctgt cactacggaa 1440
gtggatctct ttggagatgc ctttgcagct tctcctgggg aggcccctgc agcatccgaa 1500
ggggctaccg caccagctac cccggcccca gtggctgcag ctcttgatgc atgctcagga 1560
aatgaccctt ttgccccatc tgaaggtagc gcagaggctg cacctgagct ggacctcttt 1620
gcaatgaagc cacctgagac cagcgctcct gtagttaccc ctacagctag cacagcccct 1680
ccagttcccg caactgctcc ttctcctgct cccacggctg tggcagccac tgctgccacc 1740
accaccgccg ccgctgcagc taccaccact gccaccacct ctgctgctgc tgccaccacc 1800
gccgctgctc ctcctgctct agatatcttt ggtgatttgt ttgattctgc tcctgaagtt 1860
gctgcagcac ctaagccaga cgcggctcct agcatagacc tgtttggcac agatgctttc 1920
tcctccccgc cacgaggggc ctctccggtg cctgagagtt ctctcactgc tgacctctta 1980
tctgtggacg catttgcagc gccgtctcct gcatccactg cctctcctgc aaaggcggag 2040
tcctcgggtg tcatagacct ttttggggat gcgtttggaa gtggtgcttc tgaaacccag 2100
ccagcaccac aggctgtttc tagttcatca gcatcggcag atctactagc tggatttggg 2160
ggttctttca tggccccttc tacaacgcca gtgactccag ctcagaataa cctgctgcaa 2220
cccagtttcg aggcagcttt tggaacgacg ccttcgactt caagcagcag ctcttttgac 2280
ccatcagtgt ttgatggttt aggcgatctt ctgatgccaa ccatggcacc atccgggcag 2340
cctgcccctg tctcaatggt cccacccagt cctgcaatgg cagccagcaa aggcctcgga 2400
agtgaccttg actcgtctct ggccagttta gtaggcaatc ttgggatttc tggtaccaca 2460

tcaaaaaagg gagatctcca gtggaatgct ggggagaaaa agctgactgg tggagccaac 2520
tggcaaccga aagtcactcc agccacatgg tcagcgggtg ttcccccgca aggcactgtt 2580
ccaccaacca gctcagtccc tccgggtgcc ggggccccgt ccgttgggca acctggagca 2640
ggatttggaa tgcctccttc agggacaggc atgaccatga tgtctcagca gccagtcatg 2700
tttgcacagc ccatgatgag gccacccttt ggagctgcag ctgtgcccgg cacacagctt 2760
tctccaagcc ctacacctgc cactcagagt cccaagaaac ctccagccaa ggacccgtta 2820
gcggatctta acatcaagga tttcttgtaa acaatttaag ctgcaatttt tgtgactgaa 2880
taggaaaaaa ctaacctgag tttggaaact tcagataaga ttgatgctca gtttcaaagt 2940
gagccaccag taccaaaccc agtgtgacgc gtaacttcct ctcccaagca cacaggccag 3000
ctgtggcagt gaacattagg aatgtgtact ctttagctgt taccctgctc ttccagctcg 3060
tagcgtattc gggttctttg tgtgttgtac gaagtaaacc atgagtgaat gaatggttcc 3120
aatgccttta gtgcttttct ggacgttgcc cgtggacgga ggatgacgca gctgttctgt 3180
ggtgagccat ttggaaagat gtgtctgtgt tttcgaaagg tttcgatgta tatataactt 3240
ttggacaaac tcaagtcctc ccatgaactt tctcttctct tctgtacctc tgttacaagc 3300
gtaatgtgat actatcagct agtgagacaa acactcttaa ctatacaaaa actttttcgg 3360
tgtggagtac atttttccaa tcacagaaac ttccaacttg ttgtgagaaa tgtttatttt 3420
tgtgcactgt atatgttaag aaattttatt ttaaaaaaaa atgaagtcta atgtccataa 3480
taaaacttct ccttgatgaa gctaccttat caagaatgag aaaaaccata tgagaagtcc 3540
atatttatca ctgctatatt aagatatata ttttaattat atttgcaggg tttgcatcta 3600
aattgaccta tttattcatt tgtgatcaaa ggcaccgaaa agtggagctt gtttctattg 3660
tgtctgtgac aatgcagtta gagatgtgct gtttctgtga ctacgaacgt gccccaagag 3720
acatctgtaa cttaaagaga actgcaaata ttttttattt cagtgtgggt ttacgtacat 3780
ctgttcagtt agtatttctt tgtgtgttct atagagtagt gtttctccat ccttcaattg 3840
agctcaaagt gacttctatt gtacctttgt gataggattg aaaccaattc agcgaattgt 3900
atcttttaat gtacataatg tattctttga ttttcagttt gttgtcatac tgtctgtgcc 3960
gatggcttgg ctgaagattt tgatgcgtac acaaggtcac tgttgatcag tgttgtttag 4020
tggcttggca gctctttgta aaagcatatt gggttggaaa ggcatttgcc tatttttcaa 4080
attatttaat agatgtatgg taccctttaa agtggttgta tctgaattta ctgtggggat 4140
aacatacact gtaatggggg aaaattacct aaaaccaatt tcaaaatggc ttttctttgt 4200
attttggttt aaaaacccag tgcatgtctg ccctctgaga tgcaataaac accttgaacc 4260

<210> 29
<211> 196

<212>  PRT

<213>  Rattus norvegicus


<220>
<223> Protein: ribosomal protein L 19

<400>  29

<210>  30

<211>  701

<212>  DNA

<213>  Rattus norvegicus


<220>
<223> cDNA: ribosomal protein L 19

<400>  30
ctttcctttc gctgctgcgt ctgcagccat gagtatgctt aggctacaga agaggcttgc      60

ctctagcgtc ctccgctgtg gtaaaaagaa ggtgtggttg gaccccaatg aaaccaacga     120

aatcgccaat gccaactctc gtcaacagat caggaagctg atcaaagatg gcctgatcat 180

ccggaagcct gtgactgtcc attcccgggc tcgatgccgg aagaacacct tggcccgacg 240

gaagggcagg catatgggca tagggaagag gaagggtact gccaacgctc ggatgcccga 300

gaaggtgacc tggatgcgaa ggatgaggat cctgcgccgg cttctcagga gataccggga 360

atctaagaag attgaccgtc atatgtatca cagcctgtac ctgaaggtca aggggaatgt 420

gttcaaaaac aagcggattc tcatggagca catccacaaa ctgaaggcag acaaggcccg 480

caagaagcta ctggctgacc aggctgaggc tcgcaggtct aagaccaagg aagcacgaaa 540

gcgccgggag gagcgcctcc aagccaagaa ggaggagatc atcaagactc tgtccaagga 600

ggaagagacc aagaaatgaa gcgtccctcg tgtctgtaca tagtggctag gctatggccc 660

acatggatca gtcattaaaa taaaacaagc cttcgtcctt g 701

<210> 31

<211> 245

<212> PRT

<213> Rattus norvegicus

<220>
<223> Protein: 14-3-3 zeta-isoform

<400> 31

Met Asp Lys Asn Glu Leu Val Gln Lys Ala Lys Leu Ala Glu Gln Ala
1               5                   10                  15

Glu Arg Tyr Asp Asp Met Ala Ala Cys Met Lys Ser Val Thr Glu Gln
            20                  25                  30

Gly Ala Glu Leu Ser Asn Glu Glu Arg Asn Leu Leu Ser Val Ala Tyr
        35                  40                  45

Lys Asn Val Val Gly Ala Arg Arg Ser Ser Trp Arg Val Val Ser Ser
    50                  55                  60

Ile Glu Gln Lys Thr Glu Gly Ala Glu Lys Lys Gln Gln Met Ala Arg
65                  70                  75                  80

Glu Tyr Arg Glu Lys Ile Glu Met Glu Leu Arg Asp Ile Cys Asn Asp
                85                  90                  95

Val Leu Ser Leu Leu Glu Lys Phe Leu Ile Pro Asn Ala Ser Gln Pro
            100                 105                 110

Glu Ser Lys Val Phe Tyr Leu Lys Met Lys Gly Asp Tyr Tyr Arg Tyr
        115                 120                 125

Leu Ala Glu Val Ala Ala Gly Asp Asp Lys Lys Gly Ile Val Asp Gln
    130                 135                 140

Ser Gln Gln Ala Tyr Gln Glu Ala Phe Glu Ile Ser Lys Lys Glu Met
145                 150                 155                 160

Gln Pro Thr His Pro Ile Arg Leu Gly Leu Ala Leu Asn Phe Ser Val

                165                 170                 175

Phe Tyr Tyr Glu Ile Leu Asn Ser Pro Glu Lys Ala Cys Ser Leu Ala
            180                 185                 190

Lys Thr Ala Phe Asp Glu Ala Ile Ala Glu Leu Asp Thr Leu Ser Glu
        195                 200                 205

Glu Ser Tyr Lys Asp Ser Thr Leu Ile Met Gln Leu Leu Arg Asp Asn
    210                 215                 220

Leu Thr Leu Trp Thr Ser Asp Thr Gln Gly Asp Glu Ala Glu Ala Gly
225                 230                 235                 240

Glu Gly Gly Glu Asn
                245

<210>  32

<211>  1687

<212>  DNA

<213>  Rattus norvegicus

<220>
<223> cDNA: 14-3-3 zeta-isoform

<400>  32
cccagagact gccgagccgg gtccgtgtgc cgccacccac tccggacaca gaatatccag      60

ttatggataa aaatgagctg gtgcagaagg ccaagctggc cgagcaggca gagcgatacg     120

atgacatggc agcctgcatg aagtctgtca ctgagcaagg agccgagctg tctaacgagg     180

agaggaacct tctctctgtt gcttataaaa acgttgtagg agcccgtagg tcatcttgga     240

gggtcgtctc gagtattgag cagaagacgg aaggtgctga gaaaaagcag cagatggctc     300

gagaatacag agagaagatc gagatggagc tgagggacat ctgcaacgac gtactgtctc     360

ttttggaaaa gttcttgatc cccaatgctt cgcagccaga aagcaaagtc ttctatttga     420

aaatgaaggg tgactactac cgctacttgg ctgaggttgc tgctggtgat gacaagaaag     480

gaattgtgga ccagtcacag caagcatacc aagaagcatt tgaaatcagc aaaaaggaga     540

tgcagccgac acaccccatc agactgggtc tggccctcaa cttctctgtg ttctactatg     600

agatcctgaa ctccccagag aaagcctgct ctcttgcaaa aacagctttt gatgaagcca     660

ttgctgaact tgatacatta agtgaagagt cgtacaaaga cagcacgcta ataatgcagt     720

tactgagaga caacttgaca ttgtggacat cggataccca aggagacgaa gcagaagcgg     780

gagaaggagg ggaaaattaa ccggccttcc aacctttgtc tgcctcattc taaaatttac     840

acagtagacc atttgtcatc catgctgtcc cacagatagt ttttttgttt acgatttatg     900

acaggtttat gttacttcta tttgaatttc tatatttccc atgtggtttt atgttttaat     960

attaggggag tagagccagt taactttagg gagttactcg ttttcatctt gaggtggcca    1020

atatgggatg tggaattttt acatgagtta cacatgtttg gcatagttag tacttttggt    1080

ccattgtggc ttcagaaggg ccagtgttca aactgcttcc atgtctaagc aaagaaaact 1140

gcctacatat tggtgtgtgc tggcggggaa taaacaggat aatgggtcca gtcatgagtg 1200

tagtctgtgt gggtactgta aggcttggag cacttgtgag gctgggacac gaacaccctg 1260

tggatgcacg ctaagaccgt gtgtctgcgt gcacaccctt gaccacagct ccagaagttg 1320

tctgtagaca aagttgtgac ccaatttact ctgataaggg cagaaacggt tcacattcca 1380

ttatttgtaa agttacctgc tgtttgcttt cattattttt gctacacatt ttatttgtat 1440

ttaaatgttt taggcaatct aagaacaaat gtaaaagtaa agatgcagta caaacgagtt 1500

gcttggtgtt cccggctcca tgcggatcaa gcacagcggt aaacaaaatc ccatgtattt 1560

aacttttttt ttttaagttt tttgttttgt tttgtttttg cttttgtgat tttttttctc 1620

tttttgatac ttgcctaaca tgcatgtgct gtaaaaatag ttaacaggga aataacttga 1680

gatgacg 1687

<210> 33

<211> 246

<212> PRT

<213> Rattus norvegicus

<220>
<223> Protein: 14-3-3-protein eta subtype

<400> 33

Met Gly Asp Arg Glu Gln Leu Leu Gln Arg Ala Arg Leu Ala Glu Gln
1               5                   10                  15

Ala Glu Arg Tyr Asp Asp Met Ala Ser Ala Met Lys Ala Val Thr Glu
            20                  25                  30

Leu Asn Glu Pro Leu Ser Asn Glu Asp Arg Asn Leu Leu Ser Val Ala
        35                  40                  45

Tyr Lys Asn Val Val Gly Ala Arg Arg Ser Ser Trp Arg Val Ile Ser
    50                  55                  60

Ser Ile Glu Gln Lys Thr Met Ala Asp Gly Asn Glu Lys Lys Leu Glu
65                  70                  75                  80

Lys Val Lys Ala Tyr Arg Glu Lys Ile Glu Lys Glu Leu Glu Thr Val
                85                  90                  95

Cys Asn Asp Val Leu Ala Leu Leu Asp Lys Phe Leu Ile Lys Asn Cys
            100                 105                 110

Asn Asp Phe Gln Tyr Glu Ser Lys Val Phe Tyr Leu Lys Met Lys Gly
        115                 120                 125

Asp Tyr Tyr Arg Tyr Leu Ala Glu Val Ala Ser Gly Glu Lys Lys Asn
    130                 135                 140

Ser Val Val Glu Ala Ser Glu Ala Ala Tyr Lys Glu Ala Phe Glu Ile

145                 150                 155                 160

Ser Lys Glu His Met Gln Pro Thr His Pro Ile Arg Leu Gly Leu Ala
                165                 170                 175

Leu Asn Phe Ser Val Phe Tyr Tyr Glu Ile Gln Asn Ala Pro Glu Gln
            180                 185                 190

Ala Cys Leu Leu Ala Lys Gln Ala Phe Asp Asp Ala Ile Ala Glu Leu
        195                 200                 205

Asp Thr Leu Asn Glu Asp Ser Tyr Lys Asp Ser Thr Leu Ile Met Gln
    210                 215                 220

Leu Leu Arg Asp Asn Leu Thr Leu Trp Thr Ser Asp Gln Gln Asp Glu
225                 230                 235                 240

Glu Ala Gly Glu Gly Asn
                245

<210>  34

<211>  1689

<212>  DNA

<213>  Rattus norvegicus


<220>
<223> cDNA: 14-3-3-protein eta subtype

<400>  34
tgcagccagc tagcgagaag gcgcgagcgg cggcgcagcc agcagcctcc cgccagccgg      60

cgagccagtg cgcgtgcgcg gcggcggcct cggcggcgac cgggaagcgg acgggcgggc     120

gaggcgagcg aggcaggcgg tgcgggcgtg cgaggcgagg ccgatcgcga gcgacatggg     180

ggaccgagag cagctgctgc agcgggcgcg actggcggag caggcggagc gctacgacga     240

catggcctcc gccatgaagg cggtgacaga gctgaatgaa cctctatcta atgaagatag     300

aaatctcctc tctgtggcct acaagaatgt agttggtgcc aggcgatctt cttggagggt     360

tattagtagc attgagcaga aaaccatggc agatgggaat gagaagaagc tggagaaagt     420

caaagcctat cgggagaaga ttgagaagga gctggagaca gtttgcaatg atgtcttggc     480

tctgctcgac aagttcctta tcaagaactg caatgatttt cagtacgaga gcaaggtgtt     540

ctacctgaaa atgaagggcg attactaccg ctacctggca gaggtggctt ctggggagaa     600

gaaaaacagt gtggttgaag cttctgaggc agcgtataag gaagccttcg aaatcagcaa     660

agagcacatg cagccaacac accccatccg gcttggcctg gccctcaatt tttctgtgtt     720

ctactatgag atccagaatg caccagagca ggcctgcctc ttagccaaac aagccttcga     780

tgatgctata gctgagctgg acacattaaa cgaggattcc tataaggact ccactctcat     840

catgcagttg ctgcgagaca acctcaccct ctggacgagc gaccagcagg atgaagaagc     900

cggagaaggc aactgaagac ccatcaggtc cctggccctt cctttaccca ccacccccat     960

tatcactgat tcttccttgc cacaatcact atatctagtg ctaaacctat ctgtattggc 1020

agcacagcta ttcagatctg ccctcctgtc ccttggaagc agtttcagat aaaccttcat 1080

gggcatttgc tggactgatg gttgctttga gccacagagc gctccctttt tgaattgtgc 1140

agagaagtgt gttctgaacg aggcatttta ttatgtctgt tgatctgtag caaatccatg 1200

tgatggtaat tgagtgtaga aaggagaatt agccaacaca ggctatggct gctatttaaa 1260

acaagctgat agtgtgttgt taagcagtac atctcgtgca tgcaaaaatg aatttgaccc 1320

tctcacccct tctttcagct aatggaaact gacacacgac aacttgttcc ttcaccatca 1380

gctttataaa ctgtttctcg tgagctttca ggcccctgct gtgcctcttt aaattatgat 1440

gtgcgcacac cttcttttca atgcaatgca tcagaggttt ttgatatgtg taactttttt 1500

ttttggttgt gattaagaat catggattta ttttttgtaa ctctttggct attgttcttg 1560

tgtaccctga cagcatcatg tgtgtcaacc tgtgtcaatc atgatgggtg gttatgaaat 1620

gccagattgc taaaataaat gttttggact taaaaagagt aaataaatgc tgctttgggg 1680

atattaaaa 1689

## Description

### FIELD OF THE INVENTION

The present invention relates to nucleic acids, their expression products and pathways involved in pain, and their use in screening for molecules that can alleviate pain. The invention further relates to methods for the assay and diagnosis of pain in patients.

### BACKGROUND TO THE INVENTION

Pain is currently classified into four general types. Post-operative acute pain can be successfully treated with existing pain medications of e.g. the opioid and non-steroidal anti-inflammatory (NSAID) types, and is usually short-term and self-limiting. A second type of pain, present e.g. in cancer and arthritis, is also responsive to medication initially with a NSAID and in its later stages with opioids. Neuropathic pain arises from damage to the central or peripheral nerve systems, and is more effectively treated with antidepressants or anticonvulsants. A fourth type of pain called central sensitization results from changes in the central nervous system as a result of chronic pain, these changes often being irreversible and difficult to treat. Nerve pain from shingles or diabetes falls into this and the neuropathic category. Changes occur where pain is at first poorly controlled and gradually progress to the point where a person is sensitive to stimuli which would not normally cause pain, for example a light touch. People with pain of this kind often describe a widening of the pain area to include areas which had originally not been injured or which were thought not to be involved in pain. This classification is, however based on clinical symptoms rather than on the underlying pain mechanisms.

Opiates such as morphine belong to a traditional class of pain-relieving compounds that are now recognized as binding to opiate receptors. Naturally occurring polypeptides have also been found to have opiate-like effects on the central nervous system, and these include β-endorphin, met-enkephalin and leu-enkephalin.

Salicin was isolated at the beginning of the 19th century, and from that discovery a number of NSAIDs such as aspirin, paracetamol, ibuprofen, flurbiprofen and naproxen were developed. NSAIDs are by far the most widely used pain-relieving compounds, but can exhibit side effects, in particular irritation of the GI tract that can lead to the formation of ulcers, gastrointestinal bleeding and anemia.

Interest in the neurobiology of pain is developing: see a colloquium sponsored by the US National Academy of Sciences in December 1998 concerning the neurobiology of pain and reviewed in *The Scientist* **13[1]**, 12, 1999. Many pain mechanisms were discussed including the role of the capsaicin receptors in pain, (M.J. Caterina *et al., Nature*, **389**, 816-824, 1997). Large dosages of capsaicin were reported to disable that receptor, (W.R. Robbins *et al., Anesthesia and Analgesia*, **86**, 579-583, 1998). Additionally, a tetrodotoxin-resistant sodium channel found in small diameter pain-sensing neurons (PN3) was discussed (A.N. Akopian *et al., Nature*, **379**, 257-262, 1986) and L. Sangameswaran *et al., Journal of Biological Chemistry*, **271**, 953-956, 1996). Its involvement in transmission and sensitization to pain signals has been reported, (S.D. Novakovic *et al., Journal of Neuroscience*, **18**, 2174-2187, 1998). A further tetrodotoxin-resistant sodium channel has been reported (S. Tate *et al., Nature Neuroscience,* **1,** 653-655 1998).

Second messenger systems have also been shown to be important since knockout-mice lacking protein kinase C (PKC) γ were reported to respond to acute pain e.g. from a hot surface, but not to respond to neuropathic pain when their spinal nerves are injured (Malmberg *et al., Science*, **278**, 279-283 (1997).

Present methods for identifying novel compounds that relieve pain of one or more of the types indicated above suffer from the defect that they are dependent either on the relatively limited number of receptors known to be involved in pain or on the empirical identification of new receptors which is an uncertain process. In relation to known receptors, for example the opioid receptor, research directed to improved compounds offers the possibility of screening compounds that have a better therapeutic ratio and fewer side effects. This does not lead naturally to compounds for different pain receptors that have new modes of action and new and qualitatively different benefits. Even when newly identified additional receptors are taken into account, known receptors revolve around tens of gene products. However, there are between 30,000 and 40,000 genes in the genome of an animal and more of them are concerned with nervous system function than with peripheral function. We therefore concluded that a large number of receptors and pathways are important to the transduction of pain, but up to now have remained unknown.

### SUMMARY OF THE INVENTION

It is an object of the invention to provide sequences of genetic material for which no role in pain has previously been disclosed, and which are useful, for example, in:
- identifying metabolic pathways for the transduction of pain
- identifying from said metabolic pathways compounds having utility in the diagnosis or treatment of pain
- producing proteins and polypeptides with a role in the transduction of pain;
- producing genetically modified non-human animals that are useful in the screening of compounds having utitlity in the treatment or diagnosis of pain.
- Identifying ligand molecules for receptors involved in said metabolic pathways and having utility in the treatment of pain.

It is yet a further object of the invention to provide research tools, for example non-human animals and microorganisms, that can be used in screening compounds for pharmacological activity, especially pain-reducing activity.

The present invention is based on sequences that are up-regulated in two models of chronic pain, namely streptozocin-induced diabetes and chronic constrictive injury (CCI) to a nerve leading to the spine, for example the sciatic nerve.

In one aspect, the invention relates to the use in the screening of compounds that are effective in the treatment of pain, or in the diagnosis of pain, of:
(a) an isolated gene sequence that is up-regulated in the spinal cord of a mamal in response to first and second models of pain, for example in response to streptozocin-induced diabetes and in response to a chronic constrictive injury to a nerve leading into the spine;
(b) an isolated gene sequence having at least 80% sequence identity with any of the the nucleic acid sequences of the accompanying Table 1 in the specification, preferably 85% sequence identity, more preferably 90%, increasingly preferably 95%, most preferably 99%.
(c) an isolated nucleic acid comprising a sequence that is hybridizable to any of the gene sequences according to (a) or (b) under stringent hybridisation conditions;
(d) a recombinant vector comprising any one of the gene sequences according to (a) to (c);
(e) a host cell containing the vector according to (d);
(f) a non-human animal, for use in the screening of compounds that are effective in the treatment of pain, or in the diagnosis of pain, having in its genome an introduced gene sequence or a removed or down-regulated nucleotide sequence, said sequence becoming up-regulated in the spinal cord of a mammal in response to first and second models of pain, particularly neuropathic or sensitisation pain, for example in response to streptozocin-induced diabetes and in response to a chronic constrictive injury to a nerve leading into the spine;
(g) an isolated polypeptide containing an amino acid sequence at least 90% identical to an amino acid sequence encoded by a nucleotide sequence according to any one of (a) to (d), or a variant thereof with sequential amino acid deletions from the C terminus and/or the N-terminus; or
(h) an isolated antibody that binds to the isolated polypeptide according to (g).

The invention further provides a compound that is useful in the treatment or diagnosis of pain and that modulates the action of an expression product of a gene sequence that becomes up-regulated in the spinal cord of a mammal in response to first and second models of pain, for example being up-regulated both in response to streptozocin induced diabetes and in response to chronic constrictive injury to a nerve leading into the spine.

The invention also relates to the use of naturally occurring compounds such as peptide ligands of the expression products of the above gene sequences and their associated signal transduction pathways for the treatment of pain.

### DESCRIPTION OF PREFERRED EMBODIMENTS

### DEFINITIONS

Within the context of the present invention:
- "Comprising" means consisting of or including. Thus nucleic acid comprising a defined sequence includes nucleic acid that may contain a full-length gene or full-length cDNA. The gene may include any of the naturally occurring regulatory sequence(s), such as a transcription and translation start site, a promoter, a TATA box in the case of eukaryotes, and transcriptional and translational stop sites. Further, a nucleic acid sequence comprising a cDNA or gene may include any appropriate regulatory sequences for the efficient expression thereof *in vitro.*
- "Isolated" requires that the material be removed from its original environment (e.g. the natural environment if it is naturally occurring). For example, a naturally occurring polynucleotide or a peptide present in a living animal is not isolated, but the same polynucleotide or peptide, separated from some or all of the coexisting materials in the natural system, is isolated. Such polynucleotide can be part of a vector and/or such polynucleotide or peptide can be part of a composition, and still be isolated in that the vector or composition is not a part of its natural environment.
- "Mechanistically distinct" in relation to pain models implies that the pain is induced by mechanisms that differ in kind rather than being variants of a similar pain model. Thus diabetic pain and chronic constrictive pain models are mechanistically distinct whereas spinal nerve ligation models and sciatic nerve ligation models are not.
- "Purified" does not require absolute purity; instead it is intended as a relative definition. Purification of starting materials or natural materials from their native environment to at least one order of magnitude, preferably two or three orders, and more preferably four or five orders of magnitude is expressly contemplated.
- "Nucleic acid sequence" or "gene sequence" means a sequence of nucleotides or any variant or homologue thereof, or truncated or extended sequence thereof, and is preferably indicated by a Genebank accession number. Also within the scope of the present invention are up-regulated nucleic acid sequences which encode expression products which are components of signaling pathways. This invention also includes any variant or homologue or truncated or extended sequence of the up-regulated nucleotide sequence. Also within the scope of the present invention, the term "nucleic acid(s) product", or "expression product" or "gene product" or a combination of these terms refers without being biased, to any, protein(s), polypeptide(s), peptide(s) or fragment(s) encoded by the up-regulated nucleotide sequence.
- "Operably linked" refers to a linkage of polynucleotide elements in a functional relationship. For instance, a promoter or an enhancer is operably linked to a coding sequence if it regulates the transcription of the coding sequence. In particular, two DNA molecules (such as a polynucleotide containing a promoter region and a polynucleotide encoding a desired polypeptide) are said to be "operably linked" if the nature of the linkage between the two polynucleotides does not (1) result in the introduction of a frame-shift mutation and (2) interfere with the ability of the polynucleotide containing the promoter to direct the transcription of the coding polynucleotide.
   "Gene product" refers to polypeptide - which is interchangeable with the term protein - which is encoded by a nucleotide sequence and includes single-chain polypeptide molecules as well as multiple-polypeptide complexes where individual constituent polypeptides are linked by covalent or non-covalent means. Polypeptides of the present invention may be produced by synthetic means (e.g. as described by Geysen *et al.*, 1996) or by recombinant means.
   The terms "variant", "homologue", "fragment", "analogue" or "derivative" in relation to the amino acid sequence for the polypeptide of the present invention includes any substitution of, variation of, modification of, replacement of, deletion of or addition of one (or more) amino acid from or to the sequence providing the resultant polypeptide has the native gene product activity. In particular, the term "homologue" covers homology with respect to structure and/or function. With respect to sequence homology, there is at least 90%, more preferably at least 95% homology to an amino acid sequence encoded by the relevant nucleotide sequence shown in Table 1, preferably there is at least 98% homology.
   Typically, for the variant, homologue or fragment of the present invention, the types of amino acid substitutions that could be made should maintain the hydrophobicity/hydrophilicity of the amino acid sequence. Amino acid substitutions may include the use of non-naturally occurring amino acid analogues.
   In addition, or in the alternative, the protein itself could be produced using chemical methods to synthesize a polypeptide, in whole or in part. For example, peptides can be synthesized by solid phase techniques, cleaved from the resin, and purified by preparative high performance liquid chromatography (e.g. Creighton (1983) Proteins Structures and Molecular Principles, WH Freeman and Co., New York, NY, USA). The composition of the synthetic peptides may be confirmed by amino acid analysis or sequencing (e.g. the Edman degradation procedure).
   Direct peptide synthesis can be performed using various solid-phase techniques (Roberge JY *et al* Science Vol 269 1995 202-204) and automated synthesis may be achieved, for example, using the ABI 431 A Peptide Synthesizer (Perkin Elmer) in accordance with the instructions provided by the manufacturer. Additionally, the amino acid sequence of a gene product, or any part thereof, may be altered during direct synthesis and/or combined using chemical methods with a sequence from other subunits, or any part thereof, to produce a variant polypeptide.
   In another embodiment of the invention, a gene product natural, modified or recombinant amino acid sequence may be ligated to a heterologous sequence to encode a fusion protein. For example, for screening of libraries for compounds and peptide agonists and antagonists of gene product activity, it may be useful to encode a chimeric gene product expressing a heterologous epitope that is recognised by a commercially available antibody. A fusion protein may also be engineered to contain a cleavage site located between a gene product sequence and the heterologous protein sequence, so that the gene product may be cleaved and purified away from the heterologous moiety.
   The gene product may also be expressed as a recombinant protein with one or more additional polypeptide domains added to facilitate protein purification. Such purification facilitating domains include, but are not limited to, metal chelating peptides such as histidine-tryptophan modules that allow purification on immobilised metals (Porath J, Protein Expr Purif Vol 3 1992 p263-281), protein A domains that allow purification on immobilised immunoglobulin, and the domain utilised in the FLAGS extension/affinity purification system (Immunex Corp, Seattle, WA, USA). The inclusion of a cleavable linker sequence such as Factor XA or enterokinase (Invitrogen, San Diego, CA, USA) between the purification domain and the gene product is useful to facilitate purification.
- "Pain" includes chronic pain and in particular diabetic pain.
- "Stringent hybridization conditions" is a recognized term in the art and for a given nucleic acid sequence refers to those conditions which permit hybridization of that sequence to its complementary sequence and closely homologous nucleic acid sequences. Conditions of high stringency may be illustrated in relation to filter-bound DNA as for example 2X SSC, 65°C (where SSC = 0.15M sodium chloride, 0.015M sodium citrate, pH 7.2); or as 0.5M NaHPO₄, 7% sodium dodecyl sulfate (SDS), 1mM EDTA, at 65°C, and washing in 0.1xSCC/0.1% SDS at 68°C (Ausubel F.M. *et al.,* eds, 1989, *Current Protocols in Molecular Biology,* Vol. I, Green Publishing Associates, Inc., and John Wiley & Sons Inc., New York, at p. 2. 10.3). Hybridization conditions can be rendered highly stringent by raising the temperature and/or by the addition of increasing amounts of formamide, to destabilize the hybrid duplex of non-homologous nucleic acid sequence relative to homologous and closely homologous nucleic acid sequences. Thus, particular hybridisation conditions can be readily manipulated, and will generally be chosen depending on the desired results.
- "Variants or homologues" include (a) sequence variations of naturally existing gene(s) resulting from polymorphism(s), mutation(s), or other alteration(s) as compared to the above identified sequences, and which do not deprive the encoded protein of function (b) recombinant DNA molecules, such as cDNA molecules encoding genes indicated by the relevant Genebank accession numbers and (c) any sequence that hybridizes with the above nucleic acids under stringent conditions and encodes a functional protein or fragment thereof.

### IDENTIFIED SEQUENCES

The inventors have identified nucleotide sequences that give rise to expression products listed in Table 1, that become differentially expressed in the spinal cord in response to two distinct chronic pain stimuli, for example neuropathic pain stimuli, and that are believed to be involved in the transduction of pain. In Table 1, * denotes more preferred nucleotide sequences and ** denotes most preferred nucleotide sequences. These nucleotide sequences have not previously been implicated in the transduction of pain.

The validity of the present experimental procedure was confirmed by the fact that nucleotide sequences were obtained as a result of the investigation whose function in the transduction of pain has been previously confirmed and established. These nucleic acid sequences are not part of this invention. Any of the nucleic acid sequences and expression products can be used to develop screening technologies for the identification of novel molecules for the prevention or treatment of pain. These screening technologies could also be used to ascribe new pain therapeutic indications to molecules that have not previously been identified as being useful for the prevention or treatment of pain. Furthermore, the said nucleic acid sequences can be used as diagnostic tools and for the development of diagnostic tools.

### PRODUCTION OF POLYPEPTIDES AND NUCLEIC ACIDS

### Vectors

Recombinant expression vectors comprising a nucleic acid can be employed to express any of the nucleic acid sequences of the invention. The expression products derived from such vector constructs can be used to develop screening technologies for the identification of molecules that can be used to prevent or treat pain, and in the development of diagnostic tools for the identification and characterization of pain. The expression vectors may also be used for constructing transgenic non-human animals.

Gene expression requires that appropriate signals be provided in the vectors, said signals including various regulatory elements such as enhancers/promoters from viral and/or mammalian sources that drive expression of the genes or nucleotide sequences of interest in host cells. The regulatory sequences of the expression vectors used in the invention are operably linked to the nucleic acid sequence encoding the pain-associated protein of interest or a peptide fragment thereof.

Generally, recombinant expression vectors include origins of replication, selectable markers, and a promoter derived from a highly expressed gene to direct transcription of a downstream nucleotide sequence. The nucleotide sequence is assembled in an appropriate frame with the translation, initiation and termination sequences, and if applicable a leader sequence to direct the expression product to the periplasmic space, the extra-cellular medium or cell membrane.

In a specific embodiment wherein the vector is adapted for expressing desired sequences in mammalian host cells, the preferred vector will comprise an origin of replication from the desired host, a suitable promoter and an enhancer, and also any necessary ribosome binding sites, polyadenylation site, transcriptional termination sequences, and optionally 5'-flanking non-transcribed sequences. DNA sequences derived from the SV40 or CMV viral genomes, for example SV40 or CMV origin, early promoters, enhancers, and polyadenylation sites may be used to provide the required non-transcribed genetic elements.

A recombinant expression vector used in the invention advantageously also comprises an untranscribed polynucleotide region located at the 3'end of the coding sequence open reading frame (ORF), this 3'-untranslated region (UTR) polynucleotide being useful for stabilizing the corresponding mRNA or for increasing the expression rate of the vector insert if this 3'-UTR harbours regulation signal elements such as enhancer sequences.

Suitable promoter regions used in the expression vectors are chosen taking into account the host cell in which the nucleic acid sequence is to be expressed. A suitable promoter may be heterologous with respect to the nucleic acid sequence for which it controls the expression, or alternatively can be endogenous to the native polynucleotide containing the coding sequence to be expressed. Additionally, the promoter is generally heterologous with respect to the recombinant vector sequences within which the construct promoter/coding sequence has been inserted. Preferred promoters are the LacI, LacZ, T3 or T7 bacteriophage RNA polymerase promoters, the lambda P_{R}, P_{L} and Trp promoters (EP-0 036 776), the polyhedrin promoter, or the p10 protein promoter from *baculovirus* (kit Novagen; Smith *et al.*, (1983); O'Reilly *et al.* (1992).

Preferred selectable marker genes contained in the expression recombinant vectors used in the invention for selection of transformed host cells are preferably dehydrofolate reductase or neomycin resistance for eukaryotic cell culture, TRP1 for S. *cerevisiae* or tetracycline, rifampicin or ampicillin resistance in *E. coli,* or Levamsaccharase for *Mycobacteria,* this latter marker being a negative selection marker.

Preferred bacterial vectors are listed hereafter as illustrative but not limitative examples: pQE70, pQE60, pQE-9 (Quiagen), pD10, phagescript, psiX174, p.Bluescript SK, pNH8A, pNH16A, pNH18A, pNH46A (Stratagene); pKK223-3, pKK233-3, pDR540, pRIT5 (Pharmacia); pWLNEO, pSV2CAT, pOG44, pXT1, pSG (Stratagene); pSVK3, pBPV, pMSG, pSVL (Pharmacia); pQE-30 (QIA express).

Preferred bacteriophage recombinant vectors of the invention are P1 bacteriophage vectors such as described by Sternberg N.L. (1992;1994).

A suitable vector for the expression of any of the pain associated polypeptides used in the invention or fragments thereof, is a baculovirus vector that can be propagated in insect cells and in insect cell-lines. A specific suitable host vector system is the pVL 1392/1393 *baculovirus* transfer vector (Pharmingen) that is used to transfect the SF9 cell line (ATCC N°CRL 1711) that is derived from *Spodoptera frugiperda.*

The recombinant expression vectors of the invention may also be derived from an adenovirus. Suitable adenoviruses are described by Feldman and Steig (1996) or Ohno *et al.* (1994). Another preferred recombinant adenovirus is the human adenovirus type two or five (Ad 2 or Ad 5) or an adenovirus of animal origin (Patent Application WO 94/26914).

Particularly preferred retrovirus for the preparation or construction of retroviral *in vitro* or *in vivo* gene delivery vehicles include retroviruses selected from the group consisting of Mink-Cell Focus Inducing Virus, Murine Sarcoma Virus, and Ross Sarcoma Virus. Other preferred retroviral vectors are those described in Roth *et al.* (1996), in PCT Application WO 93/25234, in PCT Application WO 94/06920, and also in Roux *et al.* (1989), Julan *et al.* (1992) and Nada *et al.* (1991).

Yet, another viral vector system that is contemplated is the Adeno Associated Viruses (AAV) such as those described by Flotte *et al.* (1992), Samulski *et al.* (1989) and McLaughlin *et al.* (1996).

### Host cells expressing pain associated polypeptides

Host cells that endogenously express pain associated polypeptides or have been transformed or transfected with one of the nucleic acid sequences described herein, or with one of the recombinant vectors described above, particularly a recombinant expression vector, can be used in the present invention. Also included are host cells that are transformed (prokaryotic cells) or are transfected (eukaryotic cells) with a recombinant vector such as one of those described above.

Preferred host cells used as recipients for the expression vectors used in the invention are the following:
(a) prokaryotic host cells: *Escherichia coli,* strains. (i.e. DH5-α, strain) *Bacillus subtilis, Salmonella typhimurium* and strains from species like *Pseudomonas, Streptomyces* and *Staphylococcus* for the expression of up and down-regulated nucleic acid sequences modulated by pain, characterized by having at least 80% sequence identity with any of the nucleic acid sequences of Table 1. Plasmid propagation in these host cells can provide plasmids for transfecting other cells.
(b) eukaryotic host cells: HeLa cells (ATCC N°CCL2; N°CCL2.1; N°CCL2.2), Cv 1 cells (ATCC N°CCL70), COS cells (ATCC N°CRL 1650; N°CRL 1651), Sf-9 cells (ATCC N°CRL 1711), C127 cells (ATCC N°CRL-1804), 3T3 cells (ATCC N°CRL-6361), CHO cells (ATCC N°CCL-61), human kidney 293 cells (ATCC N° 45504; N°CRL-1573), BHK (ECACC N°84100 501; N°84111301), PC12 (ATCC N° CRL-1721), NT2, SHSY5Y (ATCC N° CRL-2266), NG108 (ECACC N°88112302) and F11, SK-N-SH (ATCC N° CRL-HTB-11), SK-N-BE(2) (ATCC N° CRL-2271), IMR-32 (ATCC N° CCL-127). A preferred system to which the nucleic acids of the invention can be expressed are neuronal cell lines such as PC12, NT2, SHSY5Y, NG108 and F11, SK-N-SH, SK-N-BE(2) , IMR-32 cell lines, COS cells, 3T3 cells, HeLa cells, 292 cells and CHO cells. The above cell lines could be used for the expression of any of the nucleic acid sequences of Table 1.

When a nucleic acid sequence of the Table 1 is expressed using a neuronal cell line, the sequence can be expressed under an endogenous promoter or native neuronal promoter or an exogenous promoter. Suitable exogenous promoters include SV40 and CMV and eukaryotic promoters such as the tetracycline promoter. The preferred promoter when pain associated molecules are endogenously expressed is an endogenous promoter. A preferred promoter in a recombinant cell line is the CMV promoter.

In a specific embodiment of the host cells described above, these host cells have also been transfected or transformed with a polynucleotide or a recombinant vector for the expression of a natural ligand of any of the nucleic acid sequences of Table 1 or a modulator of these expression products.

### PROTEINS, POLYPEPTIDES AND FRAGMENTS

The expression products of the nucleic acid sequences of Table 1 below or fragment(s) thereof can be prepared using recombinant technology, from cell lines or by chemical synthesis. Recombinant methods, chemical methods or chemical synthetic methods can be used to modify a gene in order to introduce into the gene product, or a fragment of the gene product, features such as recognition tags, cleavage sites or other modifications. For efficient polypeptide production, the endogenous expression system or recombinant expression system should allow the expression products to be expressed in a manner that will allow the production of a functional protein or fragment thereof which can be purified. Preferred cell lines are those that allow high levels of expression of polypeptide or fragments thereof. Such cell lines include cell lines which naturally express any of the nucleic acid sequences of Table 1 or common mammalian cell lines such as CHO cells or COS cells, etc, or more specific neuronal cell lines such as PC12. However, other cell types that are commonly used for recombinant protein production such as insect cells, amphibian cells such as oocytes, yeast and prokaryotic cell lines such as *E. coli* can also be used.

The expression products of Table 1 or fragments thereof can be utilized in screens to identify potential therapeutic ligands, either as a purified protein, as a protein chimera such as those produced by phage display, as a cell membrane (lipid or detergent) preparation, or in intact cells.

The invention also relates generally to the use of proteins, peptides and peptide fragments for the development of screening technologies for the identification of molecules for the prevention or treatment of pain, and the development of diagnostic tools for the identification and characterization of pain. These peptides include expression products of the nucleic acid sequences of Table 1 and purified or isolated polypeptides or fragments thereof having at least 90%, preferably 95%, more preferably 98% and most preferably 99% sequence identity with the any of the expression products of nucleic acid sequences of Table 1. Expressed peptides and fragments of any of these nucleic acid sequences can be used to develop screening technologies for the identification of novel molecules for the prevention or treatment of pain. These screening technologies could also be used to ascribe new pain therapeutic indications to molecules, which have not previously been ascribed for the prevention or treatment of pain. Furthermore the said expressed peptides and fragments can be used as diagnostic tools and for the development of diagnostic tools.

### SCREENING METHODS

As discussed above, we have identified nucleic acid sequences whose expression is regulated by pain, particularly chronic pain and more particularly diabetic pain. The expression products of these nucleic acids can be used for screening ligand molecules for their ability to prevent or treat pain, and particularly, but not exclusively, chronic pain. The main types of screens that can be used are described below. The test compound can be a peptide, protein or chemical entity, either alone or in combination(s), or in a mixture with any substance. The test compound may even represent a library of compounds.

The expression products of any of the nucleic acid sequences of Table 1 or fragments thereof can be utilized in a ligand binding screen format, a functional screen format or in vivo format. Examples of screening formats are provided.

### A) Ligand binding screen

In ligand binding screening a test compound is contacted with an expression product of one of the sequences of Table 1, and the ability of said test compound to interact with said expression product is determined, e.g. the ability of the test compound(s) to bind to the expression product is determined. The expression product can be a part of an intact cell, lipidic preparation or a purified polypeptide(s), optionally attached to a support, such as beads, a column or a plate etc.

Binding of the test compound is preferably performed in the presence of a ligand to allow an assessment of the binding activity of each test compound. The ligand may be contacted with the expression product either before, simultaneously or after the test compound. The ligand should be detectable and/or quantifiable. To achieve this, the ligand can be labeled in a number of ways, for example with a chromophore, radioactive, fluorescent, phosphorescent, enzymatic or antibody label. Methods of labeling are known to those in the art. If the ligand is not directly detectable it should be amenable to detection and quantification by secondary detection, which may employ the above technologies. Alternatively the expression product or fragment thereof can be detectable or quantifiable. This can be achieved in a similar manner to that described above.

Binding of the test compound modifies the interaction of the ligand with its binding site and changes the affinity or binding of the ligand for/to its binding site. The difference between the observed amount of ligand bound relative to the theoretical maximum amount of ligand bound (or to the ligand bound in the absence of a test compound under the same conditions) is a reflection of the binding ability (and optionally the amount and/or affinity) of a test compound to bind the expression product.

Alternatively, the amount of test compound bound to the expression product can be determined by a combination of chromatography and spectroscopy. This can also be achieved with technologies such as Biacore (Amersham Pharmacia). The amount of test compound bound to the expression product can also be determined by direct measurement of the change in mass upon compound or ligand binding to the expression product. Alternatively, the expression product, compound or ligand can be fluorescently labelled and the association of expression product with the test compound can be followed by changes in Fluorescence Energy Transfer (FRET).

The invention therefore includes a method of screening for pain alleviating compounds, comprising:
a) contacting a test compound or test compounds in the presence of a ligand with an expression product of any of the nucleic acid sequences of Table 1 or with a cell expressing at least one copy of the expression product or with a lipidic matrix containing at least one copy of the expression product; determining the binding of the test compound to the expression product, and
b) selecting test compounds on the basis of their binding abilities.

In the above method, the ligand may be added prior to, simultaneously with or after contact of the test compound with the expression product. Non limiting examples and methodology can be gained from the teachings of the *Molecular Probes handbook* and references therein (Molecular Probes, Inc., 4849 Pitchford Ave, Eugene, USA), *Methods in neurotransmitter receptor analysis* (Yamamura HI., Enna, SJ., and Kuhar, MJ., Raven Press New York, the *Glaxo Pocket Guide to Pharmacology*, Dr. Michael Sheehan, Glaxo Group Research Ltd, Ware, Herts SG12 0DP), *Bylund DB and Murrin LC* (2000, Life Sciences, 67 (24) 2897-911), *Owicki JC* (*2000, J. biomol Screen (5) 297-306), Alberts et al (1994, Molecular Biology of the Cell, 3*^{*rd*} *Edn, Garland Publication Inc), Butler JE, (2000 Methods 22(1):4-23, Sanberg SA* (2000, Curr Opin Biotechnol 11(1) 47-53), and *Christopoulos A* (1999, biochem Pharmacol 58(5) 735-48).

### Functional screening

### (a) Kinase assays

The expression products of any of the nucleic acid sequences of Table 1 which encode a kinase are amenable to screening using kinase assay technology.

Kinases have the ability to add phosphate molecules to specific residues in ligands such as binding peptides in the presence of a substrate such as adenosine triphosphate (ATP). Formation of a complex between the kinase, the ligand and substrate results in the transfer of a phosphate group from the substrate to the ligand. Compounds that modulate the activity of the kinase can be determined with a kinase functional screen. Functional screening for modulators of kinase activity therefore involves contacting one or more test compounds with an expression product of one of the nucleic acid sequences of Table 1 which encodes a kinase, and determining the ability of said test compound to modulate the transfer of a phosphate group from the substrate to the ligand.

The expression product can be part of an intact cell or of a lipidic preparation or it can be a purified polypeptide(s), optionally attached to a support, for example beads, a column, or a plate. Binding is preferably performed in the presence of ligand and substrate to allow an assessment of the binding activity of each test compound.

The ligand should contain a specific kinase recognition sequence and it should not be phosphorylated at its phosphoryation site. The ligand and/or substrate may be contacted with the kinase either before, simultaneously or after the test compound. Optionally the substrate may be labelled with a kinase transferable labelled phosphate. The assay is monitored by the phosphorylation state of the substrate and/or the ligand. The ligand should be such that its phosphorylation state can be determined. An alternative method to do this is to label the ligand with a phosphorylation-state-sensitive molecule. To achieve this, the ligand can be labelled in a number of ways, for example with a chromophore, radioactive, fluorescent, phosphorescent, enzymatic or antibody label. If the ligand is not directly detectable it should be amenable to detection and quantification by secondary detection, which may employ the above technologies. Such technologies are known to those in the art.

Binding of the test compound to the kinase modifies its ability to transfer a phosphate group from the substrate to the ligand. The difference between the observed amount of phosphate transfer relative to the theoretical maximum amount of phosphate transfer is a reflection of the modulatory effect of the test compound. Alternatively, the degree of phosphate transfer can be determined by a combination of chromatography and spectroscopy. The extent of phosphorylation of the ligand peptide or dephosphorylation of the substrate can also be determined by direct measurement. This can be achieved with technologies such as Biacore (Amersham Pharmacia).

The invention also provides a method for screening compounds for the ability to relieve pain, which comprises:
(a) contacting one or more test compounds in the presence of ligand and substrate with an expression product of any of the nucleotide sequences of Table 1 which is a kinase or with a cell containing at least 1 copy of the expression product or with a lipidic matrix containing at least 1 copy of an expression product;
(b) determining the amount of phosphate transfer from the substrate to the ligand; and
(c) selecting test compounds on the basis of their capacity to modulate phosphate transfer.

Optionally ligand, substrate and/or other essential molecules may be added prior to contacting the test compound with expression product of step (a) or after step (a). Non limiting examples and methodology can be gained from the teachings of the *Molecular* *Probes handbook* and references therein (Molecular Probes, Inc., 4849 Pitchford Ave, Eugene, USA), *Methods in Molecular Biology 2000;* 99: 191-201, *Oncogene* 2000 20; 19(49): 5690-701, and *FASAB Journal*, (10, 6, P55, P1458, 1996, Pocius D Amrein K *et al*).

### b) Phosphatase assays

The expression products of any of the nucleic acid sequences of Table 1 which encode a phosphatase are amenable to screening using phosphatase assay technology.

Phosphatase enzymes have the ability to remove phosphate molecules from specific residues in ligands such as peptides. This reaction takes place in the presence of a substrate such as Adenosine Diphosphate (ADP). The complexing of the phosphatase polypeptide, ligand and substrate results in the transfer of a phosphate group from the ligand to substrate. Compounds that modulate the activity of the Phosphatase can be determined with a Phosphatase functional screen. This screen detects the reverse of the Kinase functional screen outlined above.

The invention also provides a method for screening compounds for the ability to relieve pain, which comprises:
(a) contacting one or more test compounds in the presence of ligand and substrate with an expression product of any of the nucleotide sequences of Table 1 which is a phosphatase or with a cell containing at least 1 copy of the expression product or with a lipidic matrix containing at least 1 copy of an expression product;
(b) determining the amount of phosphate transfer from the ligand to the substrate; and
(c) selecting test compounds on the basis of their capacity to modulate phosphate transfer.

Optionally ligand, substrate and/or other essential molecules may be added prior to contacting the test compound with expression product of step (a) or after step (a). Nonlimiting examples and methodology can be gained from the teachings of the *Molecular Probes handbook* and references therein (Molecular Probes, Inc., 4849 Pitchford Ave, Eugene, USA), and *FASAB Journal*, (10, 6, P55, P1458, 1996, Pocius D Amrein K *et al*).

### c) Phosphodiesterase assays

An expression product of any of the nucleic acid sequences of Table 1 which encodes a phosphodiesterase is amenable to screening using phosphodiesterase assay technology.

Phosphodiesterases have the ability to cleave cyclic nucleotides cAMP (cyclic adenosine monophosphate) and/or cGMP (cyclic guanosine monophosphate) (substrate) at their 3'phosphatase bond to form 5'AMP and 5'GMP. Functional screening for modulators of phosphodiesterase polypeptide comprises contacting one or more test compounds with an expression product as set out above which is a phosphodiesterase and determining the ability of said test compound(s) to modulate the cleavage of cyclic nucleotides cAMP and/or cGMP at their 3'phosphatase bond to form 5'AMP and 5'GMP. The expression product can be part of an intact cell or lipidic preparation or a purified polypeptide(s), optionally attached to a support, for example beads, a column, or a plate. Binding is preferably performed in the presence of cAMP or cGMP to allow an assessment of the binding activity of each test compound. The cAMP or cGMP and other essential molecules may be contacted with the phosphodiesterase peptide either before, simultaneously or after the test compound(s).

A characteristic of the cAMP or cGMP is that it can readily be radio labeled (Thompson *et al, Advances in cyclic nucleotide research,* **10,** 69-92 (1974)). The conversion of cAMP or cGMP to 5'AMP or 5'GMP can be detected with the use of chromatography and separation technologies. Such technology is known to those in the art. Binding of the test compound to the phosphodiesterase polypeptide modifies its ability to convert cAMP or cGMP to 5'AMP or 5'GMP. The difference between the observed amount of conversion relative to the theoretical maximum amount of conversion is a reflection of the modulatory effect of the test compound(s).

A non-limiting general understanding of how to assay for the activity of Phosphodiesterases can be gained from *Horton, JK. And Baxendale, PM* (Methods in molecular Biology, 1995, 41, p 91-105, Eds. Kendall, DA. and Hill, SJ, Humana Press, Towota, NJ) and *Molecular Probes handbook* and references therein (Molecular Probes, Inc., 4849 Pitchford Ave, Eugene, USA).

The invention also provides methods of screening for pain alleviating compounds, comprising;
a) contacting one or more test compounds in the presence of cAMP and/or cGMP with an expression product of any of the nucleotide sequences of Table 1 which is a phosphodiesterase or with a cell expressing at least 1 copy of an expression product or with a lipidic matrix containing at least 1 copy of an expression product;
b) determining the amount of cAMP and/or cGMP converted to 5'AMP and/or 5'GMP, and
**c)** selecting test compounds on the basis of their ability to modulate said conversion.
**d) Ion Channel Protein assays**

An expression product of any of the nucleic acid sequences of Table 1 which encodes an ion channel protein, and in particular any of the nucleic acid sequences listed in Table 1, is amenable to screening using ion channel protein assay technology.

Ion channels are membrane associated proteins. They are divided into three main groups:
(1) ligand gated ion channels;
(2) voltage gated ion channels; and
(3) mechano gated ion channels.

Ion channels allow the passage of ions through cellular membranes upon stimulation by ligand, change in membrane potential or physical changes in environment such as temperature and pH. Compounds that modulate the activity of ion channels can be determined with an ion channel functional screen.

Functional screening for modulators of ion channels involves contacting a test compound with an expression product as aforesaid which is an ion channel protein or a fragment thereof and determining the ability of said test compound to modulate the activity of said expression product or fragment thereof. The expression product can be a part of an intact cell, membrane preparation or lipidic preparation, optionally attached to a support, for example beads, a column, or a plate. The ligand may be contacted with the ion channel peptide before, simultaneously with or after the test compound. Optionally other molecules essential for the function of the ion channel may be present. Ion channel opening is detectable with the addition of Ion channel sensitive dye, such dyes are known to those in the art. Examples are provided in *Molecular Probes handbook* and references therein (Molecular Probes, Inc., 4849 Pitchford Ave, Eugene, USA) and *Glaxo Pocket Guide to Pharmacology* (Dr Michael Sheenal Pharmacology Division Staff, Glaxo Group Research Ltd., Ware, Herts SG12 0DP). Binding of the test compound to the ion channel protein modifies its ability to allow ion molecules across a membrane. The difference between the observed amount of movement of ions across a membrane relative to the theoretical maximum amount of ions that can move across the membrane is a reflection of the modulatory effect of the test compound.

The invention therefore also relates to a method of screening compounds for their ability to alleviate pain, which method comprises:
(a) contacting at least one test compound in the presence of voltage potential sensing dye with a cell containing at least 1 copy of an expression product of any of the nucleotide sequences of Table 1 which is an ion channel protein or with a lipidic matrix containing at least one copy of the expression product;

(a) applying a ligand or stimulus;
(b) determining the opening of the ion channel, and
(c) selecting a test compound on the basis of its ability to modulate movement of ions across the membrane.

### e) Receptor assays

An expression product of any of the nucleic acid sequences of Table 1 which encodes a receptor is amenable to screening using receptor assay technology.

Receptors are membrane associated proteins that initiate intracellular signalling upon ligand binding. Therefore, the identification of molecules for the prevention and treatment of pain can be achieved with the use of a ligand-binding assay, as outlined above. Such an assay would utilize an endogenous or non-endogenous ligand as a component of the ligand-binding assay. The binding of this ligand to the receptor in the presence of one or more test compounds would be measured as described above. Such is the nature of receptors that the assay is usually, but not exclusively performed with a receptor as an intact cell or membranous preparation.

The invention therefore includes a method of screening for pain alleviating compounds, comprising:
(a) contacting a test compound or test compounds in the presence of a ligand with cells expressing at least one copy of the expression product of any of the nucleotide sequences of Table 1 which is a receptor or with a lipidic matrix containing at least one copy of the expression product;
(b) determining the binding of the test compound to the expression product, and
(c) selecting test compounds on the basis of their binding abilities.

### Transporter protein assays

An expression product of any of the nucleic acid sequences of Table 1 which encodes a transporter protein which is amenable to screening using transporter protein assay technology. Non limiting examples of technologies and methodologies are given by *Carroll FI, et al* (1995, Medical Research Review, Sep15 (5) p419-444), *Veldhuis JD and Johnson Ml* (1994, Neurosci. Biobehav Rep., winter 18(4) 605-12), *Hediger MA and Nussberger S* (1995, Expt Nephrol, July-Aug 3(4) p211-218, *Endou H and Kanai Y,* (1999, Nippon Yakurigaku Zasshi, Oct. 114 Suppl 1:1p-16p), *Olivier B et al* (2000, Prog. Drug Res., 54, 59-119), *Braun A et al* (2000, Eur J Pharm Sci, Oct 11, Supply 2 S51-60) and *Molecular Probes handbook and references therein* (Molecular Probes, Inc., 4849 Pitchford Ave, Eugene, USA).

The main function of transporter proteins is to facilitate the movement of molecules across a cellular membrane. Compounds that modulate the activity of transporter proteins can be determined with a transporter protein functional screen. Functional screening for modulators of transporter proteins comprises contacting at least one test compound with an expression product as aforesaid which is a transporter protein and determining the ability of said test compound to modulate the activity of said transporter protein. The expression product can be part of an intact cell, or lipidic preparation, optionally attached to a support, for example beads, a column or a plate. Binding is preferably performed in the presence of the molecule to be transported, which should only able to pass through a cell membrane or lipidic matrix with the aid of the transporter protein. The molecule to be transported should be able to be followed when it moves into a cell or through a lipidic matrix. Preferably the molecule to be transported is labelled to aid in characterization, e.g. with a chromophore, radioactive, fluorescent, phosphorescent, enzymatic or antibody label. If the molecule to be transported is not directly detectable it should be amenable to detection and quantification by secondary detection, which may employ the above technologies. The molecule to be transported may be contacted with the transporter protein before, simultaneously with or after the test compound. If the binding of the test compound to the transporter protein modifies its ability to transport molecules through a membranous or lipidic matrix, then the difference between the observed amount of transported molecule in a cell/or through a lipidic matrix relative to the theoretical maximum amount is a reflection of the modulatory effect of the test compound.

The invention further provides a method for screening compounds for their ability to relieve pain, comprising
a) contacting at least one test compound in the presence of transporter molecules with a cell containing at least one copy of an expression product of any of the nucleotide sequences of Table 1 which is a transporter protein or with a lipidic matrix containing at least one copy of the expression product;
c) measuring the movement of transported molecules into or from the cell, or across the lipidic matrix; and
d) selecting test compounds on the basis of their ability to modulate the movement of transported molecules.

### DNA-binding protein assays

An expression product of any of the nucleic acid sequences of Table 1 that encodes a DNA-binding protein is amenable to screening using DNA-binding protein assay technology.

DNA binding proteins are proteins that are able to complex with DNA. The complexing of the DNA binding protein with the DNA in some instances requires a specific nucleic acid sequence. Screens can be developed in a similar manner to ligand binding screens as previously indicated and will utilise DNA as the ligand. DNA-binding protein assays can be carried out using similar principles described in ligand binding assays as described above. Non limiting examples of methodology and technology can be found in the teachings of *Haukanes BI and Kvam* C (Biotechnology, 1993 Jan 11 60-63), *Alberts B et al* (Molecular Biology of the Cell, 1994, 3^{rd} Edn., Garland Publications Inc, *Kirigiti P and Machida CA* (2000 Methods Mol Biol, 126, 431-51) and *Molecular Probes handbook* and references therein (Molecular Probes, Inc., 4849 Pitchford Ave., Eugene, USA).

The invention therefore includes a method of screening for pain alleviating compounds, comprising:
(a) contacting a test compound or test compounds in the presence of a plurality of nucleic acid sequences with an expression product of any of the nucleic acid sequences of Table 1 which is a DNA binding protein or with a cell expressing at least one copy of the expression product or with a lipidic matrix containing at least one copy of the expression product;
(b) determining the binding of the test compound to the expression product, and
(c) selecting test compounds on the basis of their binding abilities.

In the above method, the plurality of nucleic acid sequence may be added prior to, simultaneously with or after contact of the test compound with the expression product.

### Oxidoreductases

Oxidoreductases are enzymes that catalyse the transfer of hydrogen or oxygen atoms or electrons. These enzymes can by sub-grouped into twenty categories according to their specific mode of action. These groups are oxidoreductases acting on the CH-OH group of donors (E.C. No1.1), oxidoreductases acting on the aldehyde or oxo group of donors (E.C. No 1.2), oxidoreductases acting on the CH-CH group of the donor (E.C. No 1.3), oxidoreductases acting on the CH-NH2 group of donors (E.C. 1.4), oxidoreductases acting on the CH-NH group of donors (E.C. 1.5), oxidoreductases acting on NADH or NADPH (E.C. No 1.6), oxidoreductases acting on other nitrogen compounds as donors (E.C. No 1.7), oxidoreductases acting on a sulphur group of donors (E.C. No 1.8), oxidoreductases acting on a haem group of donors (E.C. No1.9), oxidoreductases acting on diphenols and related substances as donors (E.C. 1.10), oxidoreductases acting on hydrogen peroxide as acceptor (E.C. No 1.11), oxidoreductases acting on hydrogen as donor (E.C. No 1.12), oxidoreductases acting on single donors with incorporation of molecular oxygen (E.C. No 1.13), oxidoreductases acting on paired donors with incorporation of molecular oxygen (E.C. No 1.14), oxidoreductases acting on superoxide radicals as acceptors (E.C. 1.15), oxidoreductases oxidizing metal ions (E.C. No 1.16), oxidoreductases acting on -CH2 groups (E.C. No 1.17), oxidoreductases acting on reduced ferredoxin as donor (E.C. No 1.18), oxidoreductases acting on reduced flavodoxin as donor (E.C. No 1.19) and other oxidoreductases (E.C. No 1.97) (Analytical Biochemistry 3^{rd} Edn, David J. Holme and Hazel Peck, Longman Press). The enzyme commission number (E.C.) of the International Union of Biochemistry relates to the type of reaction catalysed by the enzyme. Further teachings on how to develop assays and screens for oxidoreductases can be obtained from *Methods in Enzymology* (Academic Press) with special reference to volume 249.

### Hydrolases

Hydrolases are enzymes that catalyse hydrolytic reactions and are sub-grouped into eleven classes according to the type of reaction they carry out. Hydrolases acting on ester bonds (E.C. No 3.1), hydrolases acting on glycosyl compounds (E.C. No 3.2), hydrolases acting on ether bonds (E.C. No 3.3), hydrolases acting on peptide bonds (E.C. No 3.4), hydrolases acting on carbon-nitrogen bonds, other than peptide bonds (E.C. No 3.5), hydrolases acting on acid anhydrides (E.C. No 3.6), hydrolases acting on carbon-carbon bonds (E.C. No 3.7), hydrolases acting on halide bonds (E.C. No 3.8), hydrolases acting on phosphorous-nitrogen bonds (E.C. No 3.9), hydrolases acting on sulphur-nitrogen bonds (E.C. No 3.10) and hydrolases acting on carbon-phosphorous bonds (Analytical Biochemistry 3^{rd} Edn, David J. Holme and Hazel Peck, Longman Press). The enzyme commission number (E.C.) of the International Union of Biochemistry relates to the type of reaction catalysed by the enzyme. Further teachings on how to develop assays and screens for hydrolases can be obtained from Methods in Enzymology (Academic Press) with special reference to volume 249.

### C) In vivo functional screen

Any of the nucleotide sequences of Table 1 or homologues thereof may be inserted by means of an appropriate vector into the genome of a lower vertebrate or of an invertebrate animal or may be inactivated or down regulated in the genome of said animal. The resulting genetically modified animal may be used for screening compounds for effectiveness in the regulation of pain. The invertebrate may, for example, be a nematode e.g. *Caenorhabditis elegans*, which is a favourable organism for the study of response to noxious stimuli. Its genome sequence has been determined, see *Science*, **282**, 2012 (1998), it can be bred and handled with the speed of a micro-organism (it is a self-fertilizing hermaphrodite) and can therefore be used in a high throughput screening format (WO 00/63424, WO 00/63425, WO 00/63426 and WO 00/63427), and it offers a full set of organ systems, including a simple nervous system and contains many similarly functioning genes and signaling pathways to mammals. A thermal avoidance model based on a reflexive withdrawal reaction to an acute heat stimulus has been described by Wittenburg *et al, Proc. Natl. Acad. Sci.USA*, **96**, 10477-10482 (1999), and allows the screening of compounds for the treatment of pain with the modulation of pain sensation as an endpoint.

The genome of *C. elegans* can be manipulated using homologous recombination technology which allows direct replacement of nucleic acids encoding *C. elegans* with their identified mammalian counterpart. Replacement of these nucleic acids with those nucleic acids outlined above would allow for the direct screening of test compound(s) with their expression products. Any of the pain-related genes described above may be ligated into a plasmid and introduced into oocytes of the worm by microinjection to produce germline transformants. Successful plasmid injection into *C. elegans* and expression of inserted sequences has been reported by Devgen B.V., Ghent, Belgium. It is also possible to produce by routine methods worms in which the target sequences are down-regulated or not expressed (knock-out worms). Further non limiting examples of methodology and technology can be found in the teachings of *Hazendonk et al* (1997, Nat genet. 17(1) 119-21), *Alberts et al*, (1994, Molecular Biology of the Cell 3^{rd} Ed. Garland Publishing Inc, *Caenorhabditis elegans* is anatomically and genetically simple), *Broverman S et al*, (1993, PNAS USA 15; 90(10) 4359-63) and Mello et al (1991, 10(12) 3959-70).

A further method for screening compounds for ability to modify response to pain, e.g. relieve pain, comprises:
(a) contacting one or more test compounds with at least one *C elegans* containing at least one copy of a sequence as set out above;
(b) subjecting the *C. elegans* to a nociceptive stimulus;
(c) observing the response of the *C. elegans* to said stimulus; and
(d) selecting test compounds on the basis of their ability to modify the response of *C. elegans* to said stimulus.

### DIAGNOSTIC TOOLS AND KITS

### Affinity peptides, ligands and substrates

Pain associated polypeptides and fragments thereof can be detected at the tissue and cellular levels with the use of affinity peptides, ligands and substrates, which will enable a skilled person to define more precisely a patient's ailment and help in the prescription of a medicament. Such affinity peptides are characterized in that firstly they are able to bind specifically to a pain associated polypeptide, and secondly that they are capable of being detected. Such peptides can take the form of a peptide or polypeptide for example an antibody domain or fragment, or a peptide/polypeptide ligand or substrate, or a polypeptide complex such as an antibody.

The preparation of such peptides and polypeptides are known to those in the art. Antibodies, these may be polyclonal or monoclonal, and include antibodies derived from immunized animals or from hybridomas, or derivatives thereof such as humanized antibodies, Fab or F(ab')2 antibody fragments or any other antibody fragment retaining the antigen binding specificity.

Antibodies directed against pain-associated gene product molecules may be produced according to conventional techniques, including the immunization of a suitable mammal with the peptides or polypeptides or fragment thereof. Polyclonal antibodies can be obtained directly from the serum of immunized animals. Monoclonal antibodies are usually produced from hybridomas, resulting from a fusion between splenocytes of immunized animals and an immortalized cell line (such as a myeloma). Fragments of said antibodies can be produced by protease cleavage, according to known techniques. Single chain antibodies can be prepared according to the techniques described in US 4,946,778. Detection of these affinity peptides could be achieved by labeling. Technologies, which allow detection of peptides, such as enzymatic labeling, fluorescence labeling or radiolabeling are well known to those in the art. Optionally these affinity peptides, ligands and substrates could themselves be detected with the use of a molecule that has specific affinity to the peptides, ligands and substrates and is itself labeled.
The invention further provides a kit comprising;
(a) affinity peptide and/or ligand and/or substrate for an expression product of a gene sequence that is up-regulated in the spinal cord of a mammal in response to first and second models of neuropathic or central sensitization pain ; and
(b) a defined quantity of an expression product of a gene sequence that is up-regulated in the spinal cord of a mammal both in response to first and second models of neuropathic or central sensitization pain, for simultaneous, separate or sequential use in detecting and/or quantifying an expression product of a gene sequence that is up-regulated in the spinal cord of a mammal in response to first and second models of neuropathic or central sensitization pain.

### Complimentary nucleic acids

Pain associated nucleic acid sequences can be characterized at the tissue and cellular levels with the use of complimentary nucleic acid sequences. Detection of the level of expression of pain associated nucleic acid sequences can help in the prognosis of a pain condition and the prescription of a medicament. These complimentary nucleic acids are characterized in that they can hybridize to a pain associated nucleic acid sequence and their presence can be detected through various techniques. Such techniques are known to those in the art and may include detection by polymerase chain reaction or detection by labeling of complimentary nucleic acid sequences by enzymatic labeling, affinity labeling fluorescent labeling or radiolabeling. Complimentary strand nucleic acid sequences of this invention are 10 to 50 bases long, more preferably 15 to 50 bases long and most preferably 15 to 30 bases long, and hybridize to the coding sequence of the nucleic acid sequences.
A further aspect of this invention is a kit that comprises:
(a) nucleic acid sequences capable of hybridization to a nucleic acid sequence that is up-regulated in the spinal cord of a mammal in response to first and second models of neuropathic or central sensitization pain; and
(b) a defined quantity of one or more nucleic acid sequences capable of hybridization to a nucleic acid sequence that is up-regulated in the spinal cord of a mammal in response to first and second models of neuropathic or central sensitization pain, for simultaneous, separate or sequential use in detecting and/or quantifying a gene sequence that is up-regulated in the spinal cord of a mammal in response to first and second models of neuropathic or central sensitization pain.

### IDENTIFICATION AND VALIDATION

Subtractive hybridization enables the identification of nucleic acid sequences whose expression profiles are modified by a stimulus. Upon stimulation of a system (in the case of this invention a nociceptive stimulus on an animal model) all observed changes in the level of nucleic acid sequence expression are due to the reaction of the system to the stimulus. Characterization of these changes in expression by way of identification of nucleic acid sequence and level of expression is both identification and validation.

The inventors have developed a four step process which allows for the simultaneous identification and validation of nucleic acid sequences whose expression are regulated by a pain stimulus, preferably a chronic pain stimulus, and more preferably a diabetic pain stimulus. This process may comprise the following steps:
(a) induction of a nociceptive stimulus in test animals;
(b) extraction of nucleic acids from specific neuronal tissue of test and control animals;
(c) selective amplification of differentially expressed nucleic acid sequence; and
(d) identification and characterization of differentially expressed gene products that are modulated by a nociceptive stimulus.

The above process is described in more detail below.

### (a) Induction of nociceptive stimulus

The effect of the selected nociceptive stimulus on the test animal needs to be confirmable. The test subjects are therefore a species that has a "developed" nervous system, preferably similar to that of humans, most preferably rats or mice. Advantageously, the nociceptive stimulus is analogous to known pain paradigms in humans. One such paradigm of pain is the pain associated with diabetes, which can be induced in rodents with the use of streptozotocin (STZ). The present application requires the sequences to be up-regulated in two pain models, and mechanical damage can provide an appropriate second model.

Streptozotocin (STZ) induces hyperglycemia and Type 1 diabetes mellitus in rats. In particular, STZ contains a glucose analogue that allows it to be taken up by the glucose transporter 2 present on the surface of pancreatic β cells, the site of insulin synthesis. Once inside the cell, STZ causes a reduction in the level of nicotinamide adenine dinucleotide (NAD⁺). The decrease in NAD⁺ levels eventually leads to necrosis of the pancreatic (β cell, causing a reduction in insulin levels and then diabetes, leading to neuropathy (diabetic) and neuropathic pain (R. B. Weiss, *Cancer Treat. Rep.,* **66**, 427-438 1982, Guy *et al, Diabetologica*, **28**, 131-137 1985; Ziegler *et al, Pain*, **34**, 1-10 1988; Archer *et al, J. Neural. Neurosurgeon. Psychiatry*, **46**, 491-499 1983). The diabetic rat model has been shown to be a reliable model of hyperalgesia. We have used an STZ-induced diabetic rat model to create a state of hyperlagesia that can be compared with control animals (Courteix *et al, Pain*, **53**, 81-88 1993).

Three models of neuropathic pain in rats,which involve nerve injury, may be used, see Ralston, DD (1998) Present models of neuropathic pain. *Pain Rev.* **5:** 83-100. The injuries are caused by (1) loosely tying four chromic gut sutures around the sciatic nerve (CCI model developed by Bennett,GJ and Y-K Xie, A peripheral mononeuropathy in rat that produces disorders of pain sensation like those seen in man, *Pain* **33**: 87-107, 1988), (2) tightly ligating one third to one half of the fibers in the sciatic nerve (model developed by Seltzer, Z, R Dubner, Y Shire, A novel behavioral model of neuropathic pain disorders produced in rats by partial sciatic nerve injury, *Pain* **43**: 205-218, 1990), and (3) tightly ligating the dorsal spinal nerve of a rat at the L5 or L5 and L6 levels (L5 model developed by Kim, SH and JM Chung, An experimental model for peripheral neuropathy produced by segmental spinal nerve ligation in the rat, *Pain* **50**:355-363, 1992).

### (b) Extraction of nucleic acids from neuronal tissue of test and control animals

The inventors have determined that RNA extraction of whole spinal cord nervous tissue would provide a way of identifying nucleic acid sequences whose expression in spinal tissue is modulated by streptozotocin induced diabetes or by a mechanical nerve damage model for neuropathic pain e.g. CCI. Test (subjected to the nociceptive stimulus) and control animals were sacrificed, and the tissue to be studied e.g. neural tissue separated. Techniques for so doing vary widely from animal to animal and will be familiar to skilled persons.

A cDNA library can be prepared from total RNA extracted from neural tissue of the test and control animals. Where possible, however, it is preferred to isolate the mRNA from the total RNA of the test and control animals, by affinity chromatography on oligo (dT)-cellulose, and then reverse transcribe the mRNA from the test and control animals to give test and control cDNA. Converting mRNA from the test and control animals to corresponding cDNA may be carried out by any suitable reverse transcription method, e.g. a method as described by Gubler & Hoffman, *Gene*, 25, 263-269 (1983). If desired a proprietary kit may be used e.g. the CapFinder PCR cDNA Library Construction Kit (Life Technologies) which is based on long-distance PCR and permits the construction of cDNA libraries from nanograms of total RNA.

### (c) Selective amplification of differentially expressed nucleic acids

The reverse transcribed cDNA of the test and control animals is subjected to subtractive hybridisation and amplification so that differentially expressed sequences become selectively amplified and commonly expressed sequences become suppressed, so as to over-produce DNA associated with said nociceptic stimulus. A wide range of subtractive hybridisation methods can be used, but the preferred method is so-called suppression subtractive hybridisation, see US-A-5565340 and Diatchenko *et al, Proc. Nat. Acad. Sci. USA*, 93, 6025-6030 (1996), the disclosures of which are herein incorporated by reference. Kits for carrying out this method are available from CLONTECH Laboratories, Inc.

### (d) Cloning and Sequencing the differentially expressed cDNA

The differentially expressed cDNA is ligated into a cloning vector, after which cells of *E. coli* are transformed with the vector and cultured. Positive clones are selected and lysed to release plasmids containing the cDNA insert. The plasmids are primed using forward and reverse primers to either side of the cloning site and the cDNA insert is sequenced. Vector and adaptor sequences are then removed from the output data from the sequencer, leaving only the nucleotide sequence of the differentially expressed gene. The sequence is then checked against data held in a database for homology to known nucleotide sequences including expressed sequence tags (ESTs) and coding sequences for proteins.

### (e) Validation of the above method

The importance of the sequences that we have identified in pain is confirmed by the fact that genes that represent nucleic acid sequences which have previously been implicated in pain, including Calmodulin (pRCM1, Genebank X13933), Enkephalin (Genebank Y07503) and Neurotensin receptor type 2 (Genebank X97121) have also been identified using this method.

The inventors have identified nucleic acid sequences of the MAP kinase pathway, a previously non pain-associated biological pathway. The inventors have subsequently shown that intra-spinal injection of a MEK inhibitor (MEK is part of the MAP kinase pathway) produces a powerful inhibition of pain (Patent application No US 60/144292). Subsequently, it has been shown that the MAP kinase is also implicated in acute inflammatory pain (Woolf *et al*, Nature Neuroscience 1999).

The invention will now be further described in the following Example.

### EXAMPLE

### Induction of diabetes

Diabetes was induced in adult (150-200g) male Sprague-Dawley rats (n=6) as described by Courteix *et al (supra).* Animals were injected intraperitoneally with streptozotocin (STZ)(50 mg/kg) dissolved in distilled water. Control or sham animals (age-matched animals, n=6) were injected with distilled water only.

### Chronic Constrictive Injury (CCI)

Rats were anaesthetized with i.p. sodium phenobarbital, after which the common left sciatic nerve was exposed at the level of the middle of the thigh by blunt dissection through the biceps femoris and proximal to the sciatic trifurcation. Four ligatures (4.0 braided silk) were tied loosely around it with about 1mm spacing. The muscle was closed in layers and two wound clips were applied to close the skin incision. The wound was then covered with topical antibiotics.

### Nociceptive testing

Static allodynia (a form of hyperlagesia) was measured using a method described by Chaplan *let al*, "Quantitative assessment of tactile allodinya in the rat paw", *J. Neurosci. Methods*, **53**, 55-63 (1994). A series of von Frey filaments of different buckling weight (i.e. the load required for the filament to bend) were applied to the plantar surface of the right hand paw. The starting filament had a buckling weight of 20g. Lifting of the paw was taken to be a positive result, in which case a filament with the next lowest buckling weight was used for the next measurement. The test was continued until a filament was found for which there was an absence of response for longer than 5 seconds whereas a re-test with the next heaviest filament gave a positive response. Animals were considered hyperalgesic if their thresholds were found to be <4g of those of comparable untreated rats, see Calcutt & Chaplan, "Spinal pharmacology of tactile allodynia in diabetic rats", *British J. Pharmacol*, **122**,1478-1482 (1997).

### Tissue Extraction

STZ-treated, CCI-treated and control animals were anaesthetized with 4% halothane and perfused with ice-cold 0.9% saline containing 1% citric acid (pH adjusted to 7.4 with NaOH). The animals were decapitated and the lumbar spinal cord exposed. A 2-centimetre length of spinal cord ending at L6 (lumbar-6 forward) was removed from the spinal column. Attached dorsal root ganglia and contaminating spinal connective tissues were removed. The spinal cord tissue was snap frozen in dry ice and isopentane. In the experiments that follow, procedures on the streptozocin-treated and control groups of animals are disclosed. It will be understood that for identification of CCI-treated animals the same experiments are performed, but using tissues from the CCI-treated animals and from control animals.

### Total RNA Extraction

Total RNA was extracted from the pooled male rat tissues of the streptozocin-treated and control groups using the TRIZOL Reagent Kit (Life Technologies). Briefly, tissue samples were homogenised fully using a Polytron homogenizer in 1ml of TRIZOL reagent per 50-100mg of tissue. Homogenized samples were then incubated at room temperature for 5 minutes and phase separated using 0.2ml chloroform per 1ml TRIZOL reagent followed by centrifugation at 3,000g. The aqueous phase was transferred to a fresh tube and the RNA precipitated with an equal volume of isopropyl alcohol and followed by centrifugation at 10,000g. The RNA pellet was washed in 75% ethanol and re-centrifuged. The pellet was then air dried and re-suspended in water.

### mRNA Extraction

In contrast to ribosomal RNA and transfer RNA, the vast majority of mRNAs of mammalian cells carry tracts of poly(A⁺) at their 3' termini. mRNAs can therefore be separated from the bulk of cellular RNA by affinity chromatography on oligo (dT)-cellulose. mRNA was extracted from Total RNA using the MESSAGEMAKER Kit (Life Technologies) in which mRNA (previously heated to 65°C in order to disrupt secondary structures and so expose the poly (A⁺) tails) was bound to oligo(dT) cellulose under high salt concentrations (0.5M NaCl) in a filter syringe. Unbound RNA was then washed away and the poly(A⁺) mRNA eluted in distilled water. A tenth of the volume of 7.5 M Ammonium Acetate, 50µg of glycogen/ml mRNA sample and 2 volumes of absolute alcohol were then added to the samples which are then placed at -20°C overnight. Following precipitation, the mRNA was spun down at 12,000g for 30 minutes at 4°C. RNAase free water was used to re-suspend the pellets, which were then and stored at - 80°C.

### PCR SELECT

The technique used was based on that of the CLONTECH PCR Select Subtraction Kit. The following protocol was performed using STZ-treated lumbar spinal cord Poly A ⁺ RNA as the 'Tester' and Sham lumbar spinal cord poly A⁺ RNA as the 'Driver' (Forward Subtraction). A second subtraction experiment using the Sham lumbar spinal cord mRNA as the 'Tester' and STZ treated lumbar spinal cord mRNA as the 'Driver' (Reverse Subtraction) was performed in parallel using the same reagents and protocol. As a control for both experiments, the subtraction was also carried out using human skeletal muscle mRNA that had been provided by the kit manufacturer.

### First-Strand cDNA Synthesis

2 µg of PolyA⁺ RNA and 1 µl of cDNA synthesis primer (10 µM) were combined in a 0.5ml Eppendorf tube and sterile H₂O was added where necessary to achieve a final volume of 5 µl. The contents were mixed gently and incubated in a thermal cycler at 70°C for 2 min. The tubes were then cooled on ice for two minutes, after which 2 µl of 5X First-strand buffer, 1 µl of dNTP mix (10 mM each), sterile H₂O and 1 µl of AMV reverse transcriptase (20 units/µl) was also added. The tubes were then placed at 42°C for 1.5 hr in an air incubator. First-strand cDNA synthesis was terminated by placing the tubes on ice. (the human skeletal muscle cDNA produced by this step was used as the 'control driver' in later steps).

### Second-Strand cDNA Synthesis

48.4 µl of Sterile H₂O, 16.0 µl of 5X Second-strand buffer, 1.6 µl of dNTP mix (10 mM) and 4.0 µl of 20X second-strand enzyme cocktail were added to each of the first-strand synthesis reaction tubes. The contents were then mixed and incubated at 16°C in a thermal cycler for 2 hr. 6 units (2 µl) of T4 DNA polymerase was then introduced and the tubes were incubated for a further 30 min at 16°C. In order to terminate second-strand synthesis, 4 µl of 20X EDTA/glycogen mix was added. A phenol:chloroform extraction was then carried out using the following protocol:-
100 µl of phenol:chloroform:isoamyl alcohol (25:24:1) was added to the tubes which were then vortexed thoroughly and centrifuged at 14,000 rpm for 10 min at room temperature. The top aqueous layer was removed and placed in a fresh tube. 100 µl of chloroform:isoamyl alcohol (24:1) was then added to the aqueous layer and the tubes were again vortexed and centrifuged at 14,000 rpm for 10 min. 40 µl of 4 M NH₄OAc and 300 µl of 95% ethanol were then added and the tubes centrifuged at 14,000 rpm for 20 min. The supernatant was removed carefully, then 500 µl of 80% ethanol was added to the pellet. The tubes were centrifuged at 14,000 rpm for 10 min and the supernatant was removed so that the pellet could be air-dried. The precipitate was then dissolved in 50 µl of H₂O. 6 µl was transferred to a fresh microcentrifuge tube. The remainder of the sample was stored at -20°C until needed.

### Rsa I Digestion

All products of the above procedures were subjected to a restriction digest, using the restriction endonuclease *Rsa I,* in order to generate ds cDNA fragments that are short and thus are optimal for subtraction hybridisation due to the standard kinetics of the hybridisation. Also, as *Rsa* I makes a double stranded cut in the middle of a recognition sequence, 'blunt ends' of a known nucleotide sequence are produced allowing ligation of adaptors onto these ends in a later step. The following reagents were added to the 6 µl product of the second hybridisation (see above): 43.5 µl of ds cDNA, 5.0 µl 10X *Rsa* I restriction buffer and 1.5 µl of *Rsa* I (10 units/ µl). The reaction mixture was incubated at 37°C for 1.5 hr. 2.5 µl of 20X EDTA/glycogen mix was used to terminate the reaction. A phenol:chloroform extraction was then performed as above (second-strand cDNA synthesis section). The pellet produced was then dissolved in 5.5 µl of H₂O and stored at - 20°C until needed. The preparation of the experimental 'Driver' cDNAs and the control skeletal muscle cDNA was thus completed.

### Adaptor Ligation

The adaptors were not ligated to the driver cDNA.

1 µl of each *Rsa* I-digested experimental cDNA (from the *Rsa* I Digestion above) was diluted with 5 µl of sterile water. Preparation of the control skeletal muscle tester cDNA was then undertaken by briefly centrifuging the tube containing control DNA (*Hae* III-digest of φX174 DNA [3 ng/ µl]) and diluting 2 µl of the DNA with 38 µl of sterile water (to 150 ng/ml). 1 µl of control skeletal muscle cDNA (from the *Rsa* I Digestion) was then mixed with 5 µl of the diluted φX174/ *Hae* III DNA (150 ng/ml) in order to produce the control skeletal muscle tester cDNA.

### Preparation of the adaptor-ligated tester cDNA

A ligation master mix was prepared by combining 3 µl of sterile water, 2 µl of 5X ligation buffer and 1 µl T4 DNA ligase (400 units/µl) per reaction. 2 µl of adaptor 1 (10 µM) was then added to 2 µl of the diluted tester cDNA. To this, 6 µl of the ligation master mix was also added. The tube was therefore labeled Tester 1-1. In a separate tube, 2 µl of the adaptor 2R (10 µM) was mixed with 2 µl of the diluted tester cDNA and 6 µl of the master mix. This tube was named Tester 1-2.

2 µl of Tester 1-1 and 2 µl of Tester 1-2 were then placed into fresh tubes. These would later be used as the unsubtracted tester control. The remainder of the contents of Tester 1-1 and Tester 1-2 tubes were then centrifuged briefly and incubated at 16°C overnight. The ligation reaction was stopped by adding 1 µl of EDTA/glycogen mix and the samples were heated at 72°C for 5 min in order to inactivate the ligase. In doing so, preparation of the experimental and control skeletal muscle adaptor-ligated tester cDNAs was complete.

1 µl from each unsubtracted tester control was then removed and diluted into 1 ml of water. These samples were set aside as they were to be used later for PCR (see below). All of the samples were stored at -20°C

### Analysis of Ligation efficiency

1 µl of each ligated cDNA was diluted into 200 µl of water and the following reagents were then combined in four separate tubes:

| **Component** | **Tube:** | **1** | **2** | **3** | **4** |
|---|---|---|---|---|---|
| Tester 1-1 (ligated to Adaptor 1) | | 1 | 1 | - | - |
| Tester 1-2 (ligated to Adaptor 2R) | | - | - | 1 | 1 |
| G3PDH 3' primer (10 µM) | | 1 | 1 | 1 | 1 |
| G3PDH 5' primer (10 µM) | | - | 1 | - | 1 |
| PCR primer 1 (10 µM) | | 1 | - | 1 | - |
| Total volume µl | | 3 | 3 | 3 | 3 |

A master mix for all of the reaction tubes plus one additional tube was made up by adding 18.5 µl of sterile H₂O, 2.5 µl of 10X PCR reaction buffer, 0.5 µl of dNTP mix (10 mM), and 0.5 µl of 50X Advantage cDNA Polymerase Mix, per reaction, into a fresh tube. 22 µl of this master mix was then aliquotted into each of the 4 reaction tubes prepared above. The contents of the tubes were overlaid with 50 µl of mineral oil. The reaction mix was incubated in a thermal cycler at 75°C for 5 min in order to extend the adaptors. The following protocol was then carried out immediately in a thermal cycler (Perkin-Elmer GeneAmp PCR Systems 2400): 94°C for 30 sec (1 cycle), 94°C 10 sec, 65°C 30 sec and then 68°C 2.5 min (25 cycles)

### First Hybridisation

1.5 µl of the Adaptor 1-ligated Tester 1-1 was combined with 1.5 µl of the *Rsa I*-digested driver cDNA, prepared earlier and 1 µl of 4X Hybridisation buffer. This process was then repeated combining the Adaptor 2R-ligated Tester 1-2 with the *Rsa* I-digested driver cDNA and 4X hybridisation buffer. The samples were incubated in a thermal cycler at 98°C for 1.5 min followed by incubation at 68°C for 8 hr.

### Second Hybridisation

1 µl of Driver cDNA (i.e. the *Rsa* I-digested cDNA (see above)), 1 µl 4X Hybridisation buffer and 2 µl Sterile H₂O were all combined in a fresh tube. 1 µl of this mix was then removed and placed in a new tube, overlaid with 1 drop of mineral oil and incubated at 98°C for 1.5 min in order to denature the driver. The following procedure was used to simultaneously mix the driver with hybridisation samples 1 and 2 (prepared in the first hybridisation), thus ensuring that the two hybridisation samples were mixed together only in the presence of freshly denatured driver: A micropipettor was set at 15 µl. The pipette tip was then touched onto the mineral oil/sample interface of the tube containing hybridisation sample 2. The entire sample was drawn partway into the tip before it was removed from the tube in order to draw a small amount of air into the tip. The pipette tip was then touched onto the interface of the tube containing the freshly denatured driver (i.e. the tip contained both samples separated by a small pocket of air) before the entire mixture was transferred to the tube containing hybridisation sample 1. The reaction was then incubated at 68°C overnight. 200 µl of dilution buffer was added to the tube, which was then heated in a thermal cycler at 68°C for 7 min. The product of this second hybridisation was stored at -20°C.

### PCR Amplification

Seven PCR reactions were set up: (1) The forward-subtracted experimental cDNA, (2) the unsubtracted tester control (see preparation of the adaptor ligated tester cDNA), (3) the reverse-subtracted experimental cDNA, (4) the unsubtracted tester control for the reverse subtraction, (5) the subtracted control skeletal muscle cDNA, (6) the unsubtracted tester control for the control subtraction, and (7) the PCR control subtracted cDNA (provided in the kit). The PCR control subtracted cDNA was required to provide a positive PCR control as it contained a successfully subtracted mixture of *Hae* III-digested φX174 DNA.

The PCR templates were prepared by aliquotting 1 µl of each diluted cDNA (i.e., each subtracted sample from the second hybridisation and the corresponding diluted unsubtracted tester control produced by the adaptor ligation, see above) into an appropriately labeled tube. 1 µl of the PCR control subtracted cDNA was placed into a fresh tube. A master mix for all of the primary PCR tubes, plus one additional reaction, was then prepared by combining 19.5 µl of sterile water, 2.5 µl of 10X PCR reaction buffer, 0.5 µl of dNTP Mix (10 mM), 1.0 µl of PCR primer 1 (10 µM) and 0.5 µl of 50X Advantage cDNA Polymerase Mix. 24 µl of Master Mix was then aliquotted into each of the 7 reaction tubes prepared above and the mixture was overlaid with 50 µl of mineral oil, before being incubated in a thermal cycler at 75°C for 5 min in order to extend the adaptors. Thermal cycling was then immediately started using the following protocol: 94°C 25 sec (1 cycle), 94°C 10 sec, 66°C 30 sec and 72°C 1.5 min (32 cycles).

3 µl of each primary PCR mixture was then diluted in 27 µl of H₂O, 1 µl of each of these dilutions was then placed into a fresh tube.

A master mix for the secondary PCRs, (plus an additional reaction) was set up by combining 18.5 µl of sterile water, 2.5 µl of 10X PCR reaction buffer, 1.0 µl of Nested PCR primer 1 (10 µM), 1.0 µl of Nested PCR primer 2R (10 µM), 0.5 µl of dNTP Mix (10 mM) and 0.5 µl of 50X Advantage cDNA Polymerase Mix per reaction. 24 µl of this Master Mix was then added into each reaction tube containing the 1 µl diluted primary PCR mixture. The following PCR protocol was then carried out: 94°C 10 sec, 68°C 30 sec and 72°C 1.5 min (12 cycles). The reaction products were then stored at -20°C.

### Ligation into a Vector/ Transformation & PCR

The products of the PCR amplification (enriched for differentially expressed cDNAs) were ligated into the pCR2.1-TOPO vector using a T/A cloning kit (Invitrogen), transformed into TOPO One Shot competent cells according to the manufacturers protocol and grown up on LB (Luria-Bertani) Agar plates overnight at 37°C. 1,000 colonies were then individually picked (using fresh sterile tips) and dipped into 5 µl of sterile water which had been aliquotted previously into 96 well PCR plates. The water/colonies were heated in a thermal cycler at 100°C for 10 minutes in order to burst the cells, thus releasing the plasmids containing a differentially expressed cDNA insert. The 5 µl of water/plasmid was then used as a template in a PCR reaction (see below) using M13 Forward and Reverse primers (10 ng/µl), complementary to the M13 site present on either side of the cloning site on the vector. 5 µl of the PCR product was then run on a 2% agarose gel and stained by ethidium bromide. PCR products of an amplified insert were identified and 5 µl of the remainder of the PCR product (i.e. from the 15 µl that had not been run on the gel) was diluted 1/10 with water. 5 µl of the diluted PCR product was then used as a template in a sequencing reaction.

### Sequencing

A sequencing reaction containing M13 primer (3.2 pmol/µl), 'BigDye' reaction mix (i.e. AmpliTaq® DNA polymerase, MgCl_{2,} buffer and fluorescent dNTPs [each of the four deoxynucleoside triphosphates is linked to a specific fluorescent donor dye which in turn is attached to a specific acceptor dye]) and cDNA template (diluted PCR product) was set up. The reaction was carried out on a thermal cycler for 25 cycles of 10 seconds at 96°C, 20 seconds at 50°C and 4 minutes at 60°C. Each reaction product was then purified through a hydrated Centri-Sep column, and lyophilised. The pellets were resuspended in Template Supression Reagent and sequenced on an ABI Prism 310 Genetic Analyser. The analyser uses an ion laser to excite the specific donor dye that transfers its energy to the acceptor dye, which emits a specific energy spectrum that can be read by the sequencer.

The potentially upregulated genes of the streptozocin-induced diabetes experiment and of the CCI experiment were sequenced at Parke-Davis, Cambridge and at the applicant's core sequencing facility in Ann Arbor, MI, USA.

### Bioinformatics

The sequencing results were analysed using the computer program CHROMAS in which the vector and adaptor sequences were clipped off, leaving only the nucleotide sequence of the differentially expressed gene. Each sequence was then checked for homology to known genes, Expressed Sequence Tags (ESTs) and Proteins using various Basic Local Alignment Search Tool (BLAST) searches against the Genbank sequence database at the National Centre for Biotechnology Information, Bethesda, Maryland, USA (NCBI).

Lists were derived called STZup and STZdown and CCIup and CCIdown that contain the nucleic acid sequences from the forward and back subtracted libraries respectively. In each list there are given accession numbers and descriptions for the known rat genes identified, and where available corresponding mouse or human genes. Sequences that are considered to be of interest and that are up-regulated both in a streptozocin-induced diabetes model and in a chronic constrictive injury pain model are listed in Table 1 below.

References have been given where available for the sequences that have been found, and sequence listings have been given in the form in which they currently appear in publicly searchable databases e.g. the NCBI databaase (National Center for Biotechnology Information, National Library of Medicine, National Institutes of Health, Bethesda, Maryland, MD 20894, USA, www.ncbi.nlm.nih.gov). These sequence listings are given for the purposes of identification only. The invention includes the use of subsequently revised versions of the above sequences (which may incorporate small differences to the version set out herein) and homologous sequences or similar proteins in other species as determined by a high percentage identity (e.g. above 50%, preferably above 90%), length of alignment and functional equivalence.

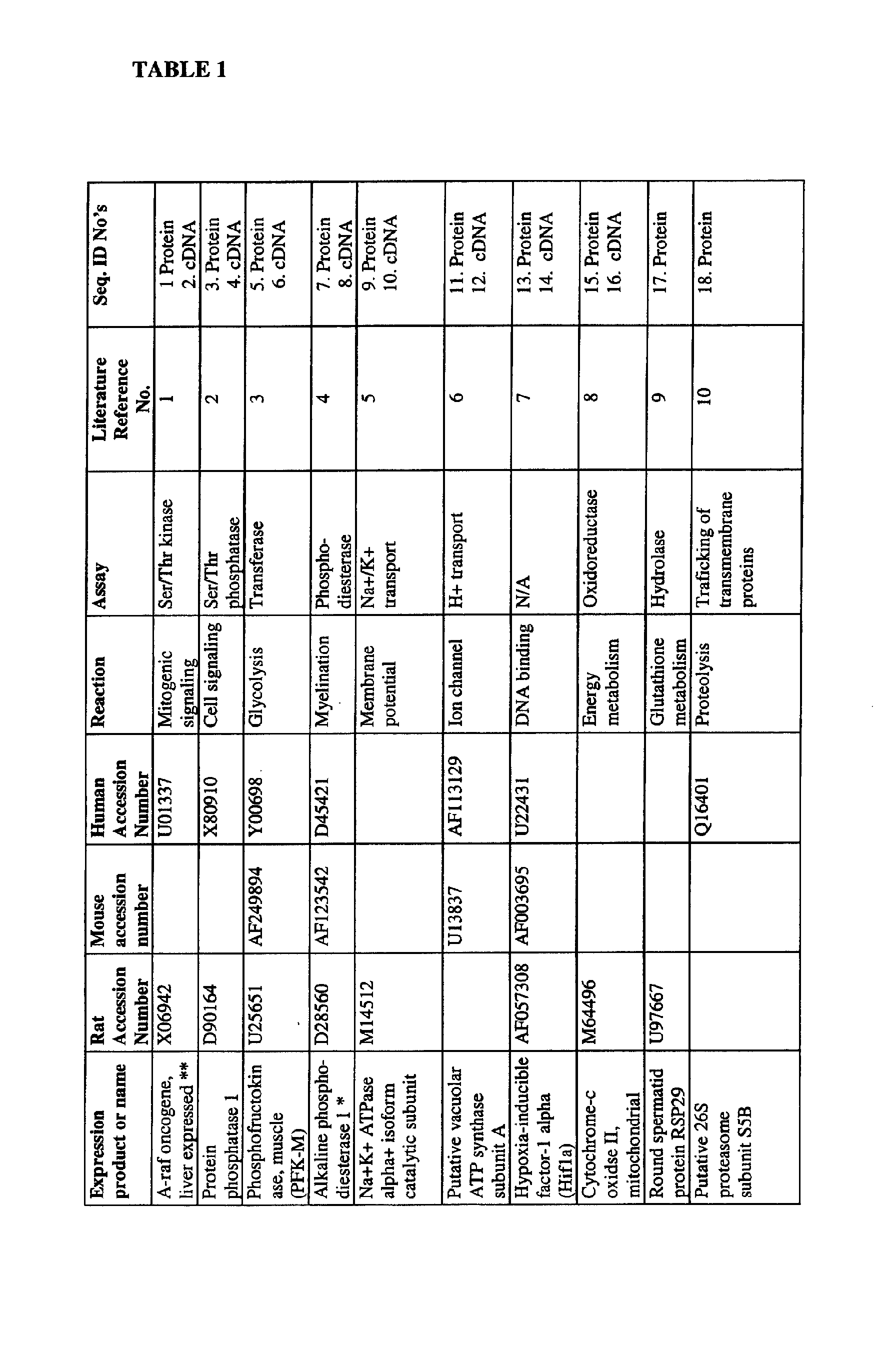

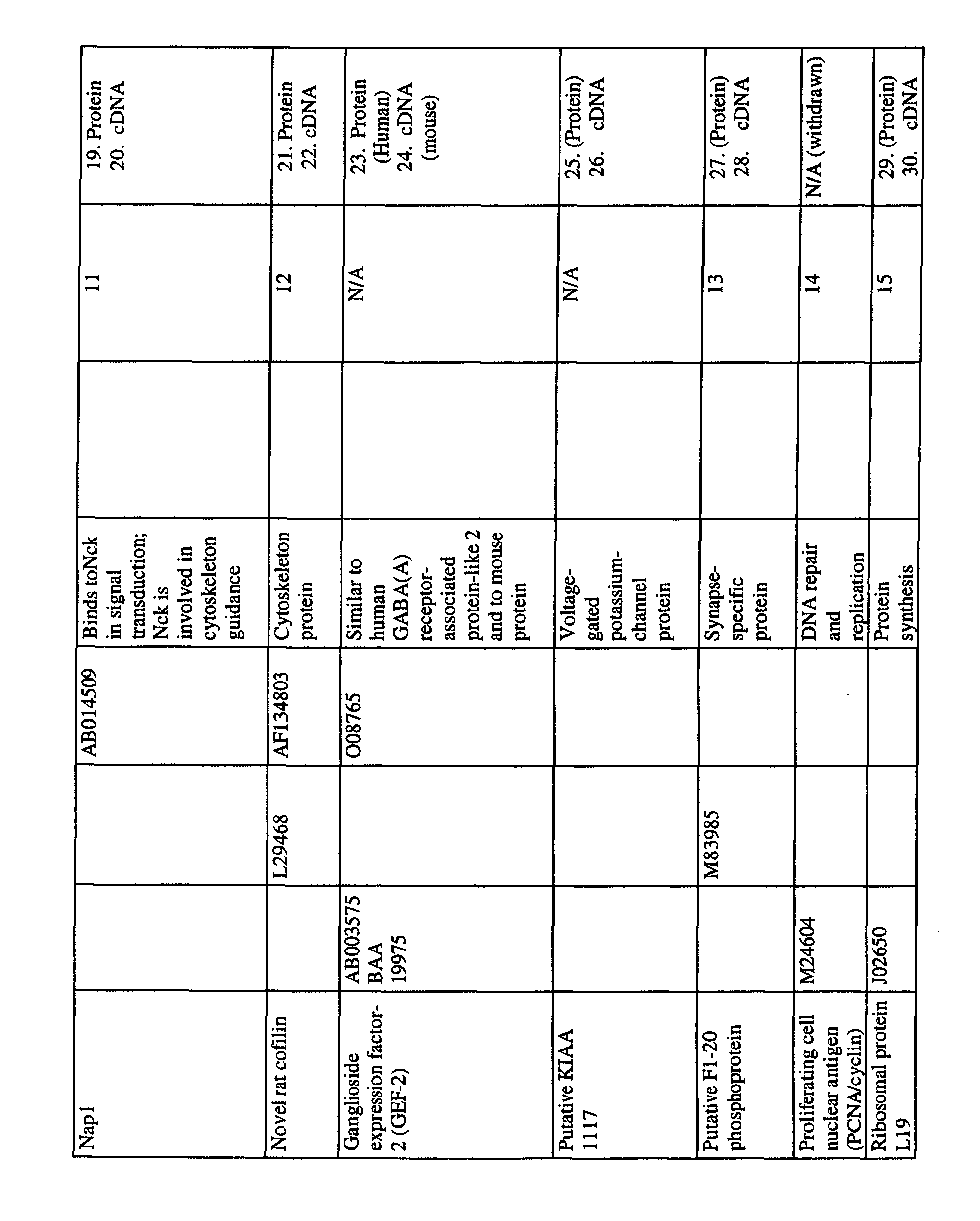

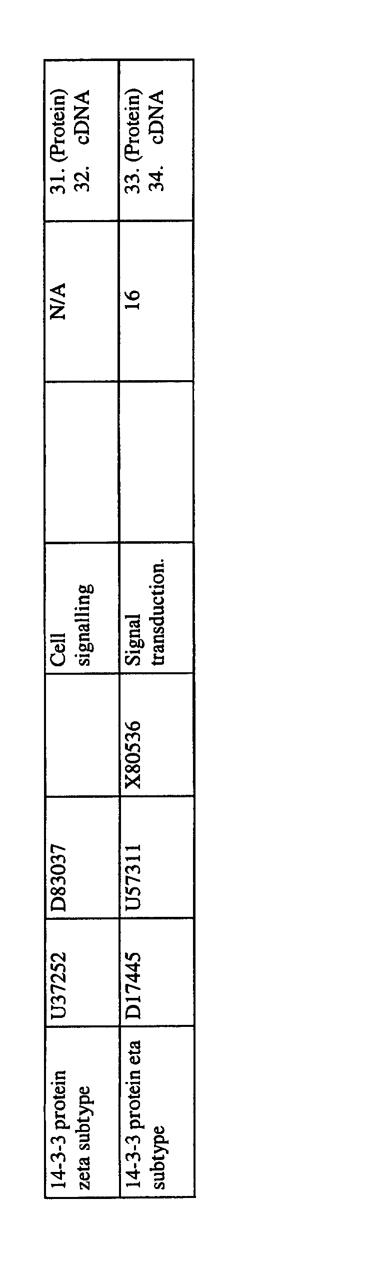

**References in the Table 1**
1. Ishikawa,F., Takaku,F., Nagao,M. and Sugimura,T., The complete primary structure of the rat A-raf cDNA coding region: conservation of the putative regulatory regions present in rat c-raf, *Oncogene Res.,* **1 (3),** 243-253 (1987).
2. Sasaki,K., Shima,H., Kitagawa,Y., Irino,S., Sugimura,T. and Nagao,M., Identification of members of the protein phosphatase 1 gene family in the rat and enhanced expression of protein phosphatase 1 alpha gene in rat hepatocellular carcinomas, *Jpn. J. Cancer Res.* **81 (12),** 1272-1280 (1990), Erratum: Jpn J Cancer Res 1991 Jul; **82(7)**, 873.
3. Ma,Z., Ramanadham,S., Kempe,K., Hu,Z., Ladenson,J. and Turk,J., Characterization of expression of phosphofructokinase isoforms in isolated rat pancreatic islets and purified beta cells and cloning and expression of the rat phosphofructokinase-A isoform, *Biochim. Biophys. Acta*, **1308 (2),** 151-163 (1996).
4 . Narita,M., Goji,J., Nakamura,H. and Sano,K., Molecular cloning, expression, and localization of a brain-specific phosphodiesterase I/nucleotide pyrophosphatase (PD-I alpha) from rat brain, *J. Biol. Chem.*, **269 (45)**, 28235-28242 (1994)
5. Shull,G.E., Greeb,J. and Lingrel,J.B., Molecular cloning of three distinct forms of the Na+,K+-ATPase alpha-subunit from rat brain, *Biochemistry,* **25,** 8125-8132 (1986).
6. Laitala-Leinonen,T., Howell,M.L., Dean,G.E. and Vaananen,H.K., Resorption-cycle-dependent polarization of mRNAs for different subunits of V-ATPase in bone-resorbing osteoclasts, *Mol. Biol. Cell* **7 (1),** 129-142 (1996).
7. Zou,A.P., Yang,Z.Z., Li,P.L. and Cowley AW,J.R., Oxygen-dependent expression of hypoxia-inducible factor-1alpha in renal medullary cells of rats, *Physiol. Genomics (Online)*, **6 (3)**, 159-168 (2001).
8. Cao,J.L., Revzin,A. and Ferguson-Miller,S., Conversion of a mitochondrial gene for mammalian cytochrome c oxidase subunit II into its universal codon equivalent and expression in vivo and in vitro, *Biochemistry*, **30 (10),** 2642-2650 (1991)
9. Ji,X., Moore,H.D., Russell,R.G. and Watts,D.J., cDNA cloning and characterization of a rat spermatogenesis-associated protein RSP29, *Biochem. Biophys. Res. Commun.,* **241 (3),** 714-719 (1997).
10. Deveraux,Q., Jensen,C. and Rechsteiner,M., Molecular cloning and expression of a 26 S protease subunit enriched in dileucine repeats, J. Biol. Chem. 270 (40), 23726-23729 (1995); Nomura,N., Nagase,T., Miyajima,N.; Sazuka,T., Tanaka,A., Sato,S., Seki,N., Kawarabayasi,Y., Ishikawa,K. and Tabata,S., Prediction of the coding sequences of unidentified human genes. II., *DNA Res*. **1 (5)**, 223-229 (1994).
11. Suzuki,T., Nishiyama,K., Yamamoto,A., Inazawa,J., Iwaki,T., Yamada,T., Kanazawa,I. and Sakaki,Y., Molecular cloning of a novel apoptosis-related gene, human nap1 (NCKAP1), and its possible relation to alzheimer disease, *Genomics,* **63 (2),** 246-254 (2000).
12. Ono,S., Minami,N., Abe,H. and Obinata,T., Characterization of a novel cofilin isoform which is predominantly expressed in mammalian skeletal muscle, *J. Biol. Chem.,* **269,** 15280-15286 (1994).
13. Lafer,E., Zhou,S., Sousa,R. and Tannery,N.H., Characterization of a novel synapse-specific protein. II. CDNA cloning and sequence analysis of the F1-20 protein, *J. Neurosci.*, **12**, 2144-2155 (1992)
14. Hsueh-We1 Chang *et al.*, UV Inducubility of Rat Proliferating Cell Nuclear Antigen Gene Promoter, *J. Cellular Biochem.*, **73**, 423-432 (1999) and references cited therein.
15. Chan,Y.-L., Lin,A., McNally,J., Peleg,D., Meyuhas,O. and Wool,I.G., The primary structure of rat ribosomal protein L19: A determination from the sequence of nucleotides in a cDNA and from the sequence of amino acids in the protein, *J. Biol. Chem.,* **262**, 1111-1115 (1987)
16. Watanabe, M., Isobe, T., Okuyama, T., Ichimura, T., Kuwano, R., Takahashi, Y. and Kondo, H., Molecular cloning of cDNA to rat 14-3-3 eta chain polypeptide and the neuronal expression of the mRNA in the central nervous system, *Brain Res. Mol. Brain Res*., **10 (2)**, 151-158 (1991); Watanabe,M., Isobe,T., Ichimura,T., Kuwano,R., Takahashi,Y., Kondo,H. and Inoue,Y., Molecular cloning of rat cDNAs for the zeta and theta subtypes of 14-3-3 protein and differential distributions of their mRNAs in the brain, *Brain Res. Mol. Brain Res.*, **25 (1-2)**, 113-121 (1994).

## Claims

1. Use of:
(a) an isolated gene sequence that is up-regulated in the spinal cord of a mammal in response to mechanistically distinct first and second models of neuropathic or central sensitization pain;
(b) an isolated gene sequence comprising a nucleic acid sequence of Table 1;
(c) an isolated gene sequence having at least 80% sequence identity with a nucleic acid sequence of Table 1;
(d) an isolated nucleic acid sequence that is hybridizable to any of the gene sequences according to (a), (b) or (c) under stringent hybridisation conditions;
(e) a recombinant vector comprising a gene sequence or nucleic acid sequence according to any one of (a) to (d);
(f) a host cell comprising the vector according to (e);
(g) a non-human animal having in its genome an introduced gene sequence or nucleic acid sequence or a removed or down-regulated gene sequence or nucleic acid sequence according to any one of (a) to (d);
(h) an isolated polypeptide comprising an amino acid sequence at least 90% identical to an amino acid sequence encoded by a nucleotide sequence according to any one of (a) to (d), or a polypeptide variant thereof with sequential amino acid deletions from the C terminus and/or the N-terminus;
(i) an isolated polypeptide encoded by a nucleotide sequence according to any one of (a) to (d); or
(j) an isolated antibody that binds specifically to a polypeptide according to (h) or (i);
in the screening of compounds for the treatment of pain, or for the diagnosis of pain.

2. Use according to claim 1, wherein the isolated gene sequence is up-regulated both in response to streptozocin-induced diabetes and in response to surgical injury of a nerve leading to the spine.

3. Use according to claim 1 or 2 wherein the isolated gene sequence encodes a kinase.

4. Use according to claim 3, wherein the isolated gene sequence encodes an expression product or fragment thereof of phosphofructokinase, muscle (PFK-M) (U25651, AF249894, Y00698).

5. Use according to claim 1, 2 or 3, wherein the isolated gene sequence encodes an expression product or fragment thereof of A-raf oncogene, liver expressed (X06942, U01337).

6. Use according to claim 1 or 2 wherein the isolated gene sequence encodes a phosphatase.

7. Use according to claim 1, 2 or 6, wherein the isolated gene sequence encodes an expression product or fragment thereof of protein phosphatase I (D90164, X80910).

8. Use according to claim 1 or 2 wherein the isolated gene sequence encodes a phosphodiesterase.

9. Use according to claim 1, 2 or 8, wherein the isolated gene sequence encodes an expression product or fragment thereof of alkaline phosphodiesterase I ((D28560, AF123542, D45421).

10. Use according to claim 1 or 2 wherein the isolated gene sequence encodes an ion channel protein.

11. Use according to claim 1, 2 or 10, wherein the ion channel protein is putative vacuolar ATP synthase subunit A (U13837, AF113129) or Na⁺K⁺ ATPase alpha+ isoform catalytic subunit (M14512).

12. Use according to claim 1 or 2 wherein the isolated gene sequence encodes a DNA binding protein.

13. Use according to claim 1, 2 or 12, wherein the isolated gene sequence encodes an expression product or fragment thereof of hypoxia-inducible factor-1 alpha (Hif1a) (AF057308, AF003695, U22431).

14. Use according to claim 1 or 2, wherein the isolated gene sequence encodes an oxidoreductase or hydrolase.

15. Use according to claim 1, 2 or 14, wherein the isolated gene sequence encodes an expression product or fragment thereof of cytochrome-c oxidase II, mitochondrial (M64496) or round spermatid protein RSP29 (U97667).

16. A non-human animal having in its genome an introduced gene sequence or a removed or down-regulated gene sequence, said sequence being up-regulated in the spinal cord of a mammal in response to first and second models of neuropathic or central sensitization pain.

17. A non-human animal according to claim 16, wherein said gene sequence becomes up regulated both in response to streptozocin induced diabetes and in response to chronic constriction injury.

18. A non-human animal according to claim 17, wherein the introduced gene sequence is according to any of claims 1 to 15.

19. A non-human animal according to any one of claims 16 to 18 which is *C. elegans.*

20. A kit comprising;
(a) affinity peptide and/or ligand and/or substrate for an expression product of a gene sequence that is up-regulated in the spinal cord of a mammal in response to a mechanistically distinct first and second models of neuropathic or central sensitization pain ; and
(b) a defined quantity of an expression product of a gene sequence that is up-regulated in the spinal cord of a mammal both in response to first and second models of neuropathic or central sensitization pain, for simultaneous, separate or sequential use in detecting and/or quantifying up-regulation of a gene sequence in the spinal cord of a mammal in response to first and second models of neuropathic or central sensitization pain.

21. A kit according to claim 20, wherein the gene sequence is defined in any one of claims 1 to 15.

22. A kit comprising:
(a) nucleic acid sequences capable of hybridization to a nucleic acid sequence that is up-regulated in the spinal cord of a mammal in response to first and second models of neuropathic or central sensitization pain; and
(b) a defined quantity of one or more nucleic acid sequences capable of hybridization to a nucleic acid sequence that is up-regulated in the spinal cord of a mammal in response to first and second models of neuropathic or central sensitization pain, for simultaneous, separate or sequential use in detecting and/or quantifying up-regulation of a gene sequence in the spinal cord of a mammal in response to first and second models of neuropathic or central sensitization pain.

23. The kit of claim 22, wherein the gene sequence is according to any of claims 1 to 15.

24. A compound that modulates the action of an expression product of a gene sequence that is up-regulated in the spinal cord of a mammal in response to first and second models of neuropathic or central sensitization pain.

25. A compound according to claim 24 wherein the gene sequence is listed in Table 1.

26. A compound according to claim 24 or 25 wherein the nucleotide sequence is according to any one of claims 1 to 15.

27. A compound according to any one of claims 24 to 26 for use as a medicament.

28. A compound according to any one of claims 24 to 27 for the treatment or diagnosis of pain.

29. A pharmaceutical composition comprising a compound according to any one of claims 24 to 28 and a pharmaceutically acceptable carrier or diluent.

30. Use of a compound according to any one of claims 24 to 28 in the manufacture of a medicament for the treatment or diagnosis of pain.

31. Use of a compound according to any one of claims 24 to 28 in the manufacture of a medicament for the treatment or diagnosis of chronic pain.

32. A method of treatment of pain, which comprises administering to a patient an effective amount of a compound according to any one of claims 24 to 28.
